(19) Europäisches Patentamt
European Patent Office
Office européen des brevets

(11) EP 4 428 132 A1

(12) **EUROPEAN PATENT APPLICATION**

(43) Date of publication:
**11.09.2024 Bulletin 2024/37**

(21) Application number: **23160797.9**

(22) Date of filing: **08.03.2023**

(51) International Patent Classification (IPC):
***C07D 413/04*** (2006.01) ***C07D 413/14*** (2006.01)
***C07D 417/04*** (2006.01) ***A61K 31/553*** (2006.01)
***A61K 31/554*** (2006.01) ***A61P 17/00*** (2006.01)

(52) Cooperative Patent Classification (CPC):
**C07D 413/04; C07D 413/14; C07D 417/04**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Johann-Wolfgang-Goethe-Universität Frankfurt am Main**
**60323 Frankfurt am Main (DE)**

(72) Inventors:
- **Schmidtko, Achim**
  **60323 Frankfurt am Main (DE)**
- **Proschak, Eugen**
  **61381 Friedrichsdorf (DE)**
- **Balzulat, Annika**
  **60385 Frankfurt am Main (DE)**
- **Zhu, Wenxin**
  **63225 Langen (DE)**
- **Flauaus, Cathrin**
  **60598 Frankfurt am Main (DE)**
- **Steinhilber, Dieter**
  **61389 Schmitten (DE)**
- **Lu, Ruirui**
  **60437 Frankfurt am Main (DE)**
- **Hernandez Olmos, Victor**
  **65931 Frankfurt am Main (DE)**
- **Heering, Jan**
  **60433 Frankfurt am Main (DE)**

(74) Representative: **Kuttenkeuler, David**
**SKM-IP PartGmbB**
**Oberanger 45**
**80331 München (DE)**

Remarks:
A request for correction of the claims has been filed pursuant to Rule 139 EPC. A decision on the request will be taken during the proceedings before the Examining Division (Guidelines for Examination in the EPO, A-V, 3.).

# (54) SLACK-ACTIVATING COMPOUNDS AND THEIR MEDICAL USE

(57) The present invention pertains to novel compounds and their synthesis which are structurally derived from 2-chloro-11-(4-methylpiperazin-1-yl)dibenzo[*b*,*f*][1,4]oxazepine. The compounds of the invention were shown in a comparative study to bind and activate potassium channels, in particular Slack. Based on their activating activity, the compounds of the invention and pharmaceutical compositions containing them can be used in various therapeutic applications for the treatment or prevention of pathologies.

EP 4 428 132 A1

## Description

FIELD OF THE INVENTION

The present invention pertains to novel compounds and their synthesis which are structurally derived from 2-chloro-11-(4-methylpiperazin-1-yl)dibenzo[b,f][1,4]oxazepine. The compounds of the invention were shown in a comparative study to bind and activate potassium channels, in particular Slack. Based on their activating activity, the compounds of the invention and pharmaceutical compositions containing them can be used in various therapeutic applications for the treatment or prevention of pathologies.

DESCRIPTION

Itch (also known as pruritus) is a debilitating symptom that accompanies various skin disorders, systemic diseases such as chronic kidney or cholestatic liver disease, psychiatric diseases, and neuropathies, or is idiopathic with unknown reason. It is defined as an unpleasant sensation that evokes a desire to scratch and affects about one third of dermatology patients and nearly 15% of the general population[1,2,] Like pain, acute itch serves as an important protective mechanism to detect potentially harmful stimuli. However, chronic itch (i.e., persisting for more than 6 weeks in humans) does not serve a useful function but instead imposes suffering and may compromise quality of life to a degree often comparable to that for chronic pain.

Itch can be categorized into histamine-dependent (histaminergic) and histamine-independent (non-histaminergic) itch. As most types of chronic itch are histamine-independent and therefore resistant to antihistamines, there is an urgent need to develop novel treatment strategies.

Most known mechanisms of pruriception (itch sensation) begin with the activation of itch-sensitive sensory neurons. Several key receptors, including members of the Mas-related G-protein-coupled receptor (Mrgpr) families, were found to be important for detecting non-histaminergic chemical itch signals[3]. Recent single-cell RNA-sequencing (scRNA-seq) studies have revealed that itch-related genes are highly expressed in distinct subpopulations of sensory neurons. For example, in a pioneering scRNA-seq study by Usoskin and colleagues 11 principal types of sensory neurons have been identified, of which 3 non-peptidergic populations (termed NP1, NP2 and NP3) were proposed to be itch-sensitive[4]. After activation, these sensory neurons signal to the dorsal horn of the spinal cord, where the ongoing information is further processed and transmitted to itch-signaling pathways ascending to the brains.

The excitability of sensory neurons is driven by various types of ion channels, among which $K^+$ channels are the most populous and diverse class that are governed by more than 75 genes in humans and feature tissue-dependent expression patterns[6] Notably, the $K^+$ channel Slack (also termed $K_{Na}1.1$; gene *Kcnt1*), which is activated by intracellular $Na^+$ and inhibited by bivalent cations[7,8], is highly enriched in the itch-sensitive NP1, NP2 and NP3 subsets of sensory neurons in mice[4,9] (**Fig. 1A**), suggesting that Slack has a role in itch. In fact, a significant functional role of Slack in pruriception is reflected by the increased scratching behavior of Slack knockout mice after exposure to pruritogens such as chloroquine and histamine[10]. These findings in combination with the observation that Slack expression in non-human primate sensory neurons[11] (**Fig. 1C**) and in human sensory neurons[12] (**Fig. 1B**) is enriched in itch-associated cell populations support the hypothesis that activators of Slack channels hold therapeutic potential for treatment of itch.

A previous study with a library screen of pharmacologically active compounds reported that the first-generation antipsychotic drug Loxapine activates Slack[13]. In preliminary experiments, the inventors observed that systemic administration of a low dose of Loxapine significantly alleviated chloroquine-induced scratching behavior of wildtype mice but did not affect the scratching behavior in Slack knockout mice (data not shown), indicating that the Loxapine-induced antipruritic effect depends on Slack activation.

Despite the need for drugs to treat histamine-independent itch, the clinical use of Loxapine is unfortunately limited by the typical adverse events of first-generation antipsychotic drugs that are related to blocking activities at dopamine receptors and other receptors[14,15].

Starting from the structure of Loxapine, inventors developed novel structural Loxapine derivatives that have an activating effect on Slack. Notably, the Loxapine derivatives of the present invention feature a different and more favorable pharmacological profile than Loxapine. Further, the compounds of the invention, which act as Slack activators, have comparable or improved binding properties to Slack, a reduced BBB permeability, which translates to less unwanted side effects, and improved off target activity in comparison to the parent molecule Loxapine.

BRIEF DESCRIPTION OF THE INVENTION

Generally, and by way of brief description, the main aspects of the present invention can be described as follows:

**In a first aspect,** the invention pertains to a compound, wherein the compound has the formula I:

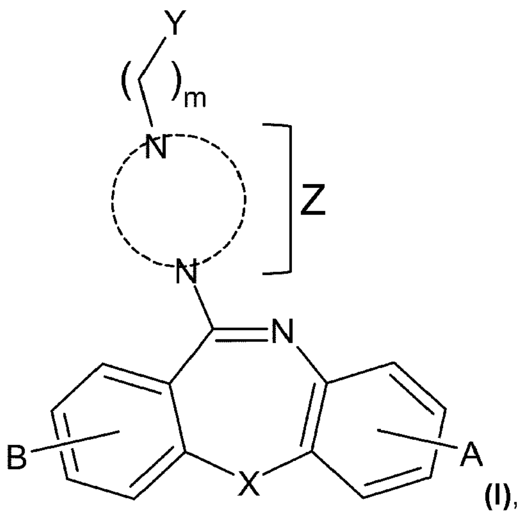

(I),

or salt, complex, diastereomer, enantiomer and/or tautomer of a compound with the formula (I), wherein

**A** is one or two substituents independently selected from -H, -F, -Cl, -Br, -I, $-CF_3$, $-OCF_3$, $-CF_2H$, and $-OCF_2H$;

**B** is one or two substituents independently selected from -F, -Cl, -Br, -I, $-CF_3$, $-OCF_3$, $-CF_2H$, and $-OCF_2H$;

**X** is selected from -S- and -O-;

**m** is 0 to 3;

**Y** is selected from

-H;

a nitrile group,

a 5- to 6-membered unsaturated heterocycle comprising 3 hetero atoms that are selected from O and N, preferably wherein 2 or 3 heteroatoms are nitrogen, wherein the heterocycle is mono or di-substituted with =S or =O, preferably with =O; or

a linear $-C_{1-3}$alkyl, wherein a, preferably one, $-(CH_2)-$ of the linear $-C_{1-3}$alkyl is optionally monosubstituted with -OH, and wherein the terminal $-CH_3$ of the linear $-C_{1-3}$alkyl is monosubstituted with $-OR^1$ or $-SR^1$, preferably $-OR^1$, wherein $R^1$ is -H, $-(CH_2)-COOH$ or $-(CH_2)-(CH_2)-OH$; or

$-(CH_2)_i-CX^1R^2$, wherein

$X^1$ is $=NR^3$ or =O, wherein $R^3$ is -H or -OH.
i is 0 or 1, and
$R^2$ is $-OR^4$ or $-NR^5R^6$, wherein $R^4$ is -H or $-C_{1-2}$alkyl; wherein $R^5$ and $R^6$ are independently selected from -H or -methyl;

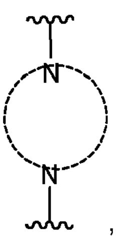

,

herein denoted as "Z", is an aliphatic group comprising at least one, preferably 1 or 2, heterocycle(s) which comprise together at least two nitrogen atoms, wherein the first of the two nitrogen atoms connects Z to

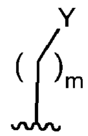

of formula I and the second of the two nitrogen atom connects Z to

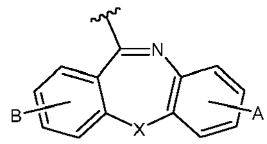

of formula I, preferably to

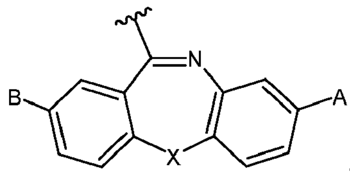

,

and wherein within group Z:

- the two nitrogen atoms are each a heteroatom in one of two connected 4-membered heterocycles (each heterocycle having no more than one heteroatom), wherein the two connected 4-membered heterocycles form a spirocycle, preferably wherein the 4-membered heterocycle with the first nitrogen atom is connected via a shared carbon atom to the 4-membered heterocycle with the second nitrogen atom, thus forming a spirocycle with two heteroatoms that are the two nitrogen atoms; or
- the first of the two nitrogen atoms is connected to a 4-membered heterocycle with one heteroatom, wherein the second of the two nitrogen atoms is the heteroatom in the 4-membered heterocycle that the first of the two nitrogen atoms is connected to; wherein the first nitrogen atom is further substituted with methyl; or
- the two nitrogen atoms are heteroatoms in a 6- to 7-membered heterocycle with two heteroatoms, wherein the heterocycle is optionally substituted, preferably monosubstituted, with methyl.

**In a second aspect,** the invention pertains to a pharmaceutical composition comprising the compound of any one of the aspects herein, or a salt, solvate, or ester thereof, and a pharmaceutically acceptable carrier or excipient.

**In a third aspect,** the invention pertains to a compound or composition for use in the treatment of a condition in a subject, the compound or composition comprising an effective amount of the compound of the first aspect of the invention, or a salt, solvate, or ester thereof, wherein the condition is treatable by activating a potassium channel in a cell associated with the pathology of the condition, the treatment comprising administering the compound or composition to the subject in need thereof.

**In a fourth aspect,** the invention pertains to a method of synthesizing a compound of the first aspect of the invention.

DETAILED DESCRIPTION OF THE INVENTION

In the following, the elements of the invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered

disclosed by the description of the present application unless the context indicates otherwise.

**In a first aspect,** the invention pertains to a compound, wherein the compound has the formula I:

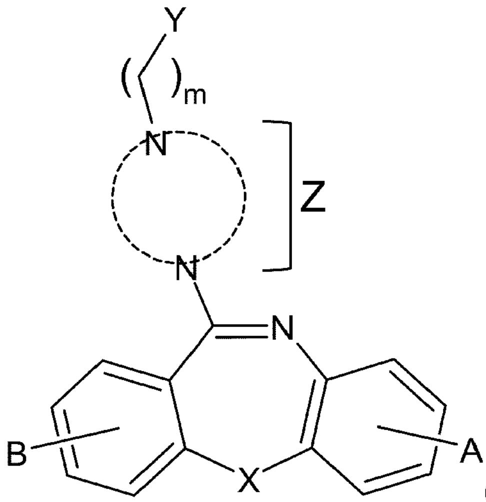

**(I)**,

or salt, complex, diastereomer, enantiomer and/or tautomer of a compound with the formula (I), wherein

**A** is one or two substituents independently selected from -H, -F, -Cl, -Br, -I, $-CF_3$, $-OCF_3$, $-CF_2H$, and $-OCF_2H$;

**B** is one or two substituents independently selected from -F, -Cl, -Br, -I, $-CF_3$, $-OCF_3$, $-CF_2H$, and $-OCF_2H$;

**X** is selected from -S- and -O-;

**m** is 0 to 3;

**Y** is selected from

-H;

a nitrile group;

a 5- to 6-membered unsaturated heterocycle comprising 3 hetero atoms that are selected from O and N, preferably wherein 2 or 3 heteroatoms are nitrogen, wherein the heterocycle is mono or di-substituted with =S or =O, preferably with =O; or

a linear $-C_{1-3}$alkyl, wherein a, preferably one, $-(CH_2)-$ of the linear $-C_{1-3}$alkyl is optionally monosubstituted with -OH, and wherein the terminal $-CH_3$ of the linear $-C_{1-3}$alkyl is monosubstituted with $-OR^1$ or $-SR^1$, preferably $-OR^1$, wherein $R^1$ is H, $-(CH_2)-COOH$ or - $(CH_2)-(CH_2)-OH$; or

$-(CH_2)_i-CX^1R^2$, wherein

$X^1$ is $=NR^3$ or =O, wherein $R^3$ is -H or -OH.
i is 0 or 1, and
$R^2$ is $-OR^4$ or $-NR^5R^6$, wherein $R^4$ is -H or $-C_{1-2}$alkyl; wherein $R^5$ and $R^6$ are independently selected from -H or -methyl.

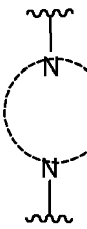

,

herein denoted as "Z", is an aliphatic group comprising at least one, preferably 1 or 2, heterocycle(s) which comprise together at least two nitrogen atoms, wherein the first of the two nitrogen atoms connects Z to

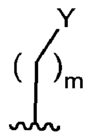

of formula I and the second of the two nitrogen atom connects Z to

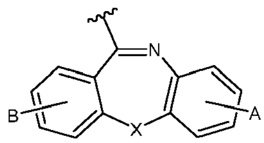

of formula I, preferably to

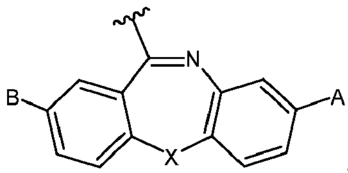

,

and wherein within group Z:

- the two nitrogen atoms are each a heteroatom in one of two connected 4-membered heterocycles (each heterocycle having no more than one heteroatom), wherein the two connected 4-membered heterocycles form a spirocycle, preferably wherein the 4-membered heterocycle with the first nitrogen atom is connected via a shared carbon atom to the 4-membered heterocycle with the second nitrogen atom, thus forming a spirocycle with two heteroatoms that are the two nitrogen atoms; or
- the first of the two nitrogen atoms is connected to a 4-membered heterocycle with one heteroatom, wherein the second of the two nitrogen atoms is the heteroatom in the 4-membered heterocycle that the first of the two nitrogen atoms is connected to; wherein the first nitrogen atom is further substituted with methyl; or
- the two nitrogen atoms are heteroatoms in a 6- to 7-membered heterocycle with two heteroatoms, wherein the heterocycle is optionally substituted, preferably monosubstituted, with methyl.

In preferred embodiments of the invention, the compound is not

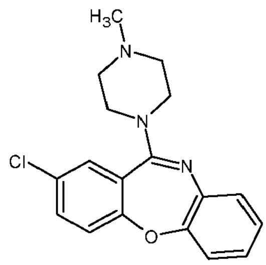

.

In preferred embodiments of the invention, the compound is a salt, complex, diastereomer, enantiomer and/or tautomer of a compound with the formula (I).

In the context of the invention, when a first chemical group is "substituted" with a second chemical group, this indicates that the second chemical group is bound to the first chemical group, preferably by replacing a hydrogen atom of the first chemical group at the position where the respective bond formed between the first and the second chemical group. In this context, the term "substituted" does not indicate the nature of the chemical reaction. Similarly, if a first chemical group is a "substituent" of a second chemical group, this indicates that the chemical groups are attached to each other.

The term "carbocyclic", "carbocycle" or "carbocyclyl" includes, unless otherwise indicated, in general a 3- to 9-membered, preferably a 4- to 8-membered, a 3- to 6-membered or a 5- to 7-membered, more preferably a 5- or 6-membered monocyclic ring comprising 3 to 9, preferably 4 to 8, 3 to 6 or 5 to 7, more preferably 5 or 6 ring carbon atoms. In preferred embodiments, a carbocycle according to the invention comprises 4, 5, 6, or 7 ring carbon atoms. The carbocycle may be saturated, partially or fully unsaturated, or aromatic, wherein saturated means that only single bonds are present, and partially or fully unsaturated means that one or more double bonds may be present in suitable positions, while the Huckel rule for aromaticity is not fulfilled, whereas aromatic means that the Huckel (4n + 2) rule is fulfilled. The term "carbocycle", "carbocyclic" or "carbocyclyl" may therefore cover inter alia cycloalkyl, cycloalkenyl, as well as phenyl. Preferably, the term "carbocyclic", "carbocyclyl" or "carbocycle" covers cycloalkyl and cycloalkenyl groups, for example cyclopropane, cyclobutane, cyclopentane and cyclohexane rings. In preferred embodiments, the "carbocycle", "carbocyclic" or "carbocyclyl" is a non-aromatic "carbocycle", "carbocyclic" or "carbocyclyl".

The term "cycloalkyl" as used herein, unless otherwise indicated, denotes in each case a monocyclic cycloaliphatic group having usually from 3 to 9 or from 4 to 7 ring carbon atoms and no ring heteroatoms. Examples of such a cycloalkyl include cyclopropyl, cyclobutyl, cyclopentyl, cyclohexyl, cycloheptyl, cyclooctyl, cyclononyl and cyclodecyl or cyclopropyl, cyclobutyl, cyclopentyl and cyclohexyl.

The term "aryl" or "aromatic carbocycle" preferably includes 6-membered aromatic carbocyclic rings based on carbon atoms as ring members. A preferred example is phenyl.

The term "heterocyclic", "heterocycle" or "heterocyclyl" includes, unless otherwise indicated, in general a 3- to 9-membered, preferably a 4- to 7-membered, preferably in each case a 5- to 7-membered, 5- to 6-membered, 6- to 7-membered, or a 3- to 5- membered, in particular a 4- 5-, 6- or 7-membered monocyclic ring. The heterocycle typically comprises one or more, e.g. 1, 2, 3, or 4, preferably 1, 2, or 3, preferably 1 or 2 heteroatoms, selected from N, O, and S as ring members, where S-atoms as ring members may be present as S, SO or $SO_2$, where N-atoms as ring members may be present as N or NH, where O as ring members may be present as O. Preferably, the heteroatom is a N-atom. The remaining ring members are carbon atoms.

In the context of the invention, when referring to hetero atoms in a heterocycle, the heteroatom "N" preferably refers to a ring member present in the form of N, or NH, the heteroatom "O" preferably refers to a ring member present in the form of O, the heteroatom S preferably refers to a ring member present in the form of S, SO or $SO_2$.

The heterocyclyl may be saturated, partially or fully unsaturated, or aromatic, wherein saturated means that only single bonds are present, and partially or fully unsaturated means that one or more double bonds may be present in suitable positions, while the Huckel rule for aromaticity is not fulfilled, whereas aromatic means that the Huckel (4n + 2) rule is fulfilled.

The term "heterocycloalkyl" as used herein, unless otherwise indicated, denotes a heterocyclyl as defined above, wherein the monocyclic ring is a monocyclic cycloaliphatic group having usually from 3 to 9 or from 4 to 7 ring atoms, in each case 5 to 7 ring atoms, 5 to 6 ring atoms, 6 to 7 ring atoms, 3 to 5 ring atoms, in particular a 4- 5-, 6- or 7 ring atoms, comprising as ring members one or more, e.g. 1, 2, 3 or 4, preferably 1, 2, or 3, preferably 1 or 2 heteroatoms, selected from N, O and S as ring members, where S-atoms as ring members may be present as S, SO or $SO_2$, where N-atoms as ring members may be present as N or NH, where O as ring members may be present as O. The remaining ring members are carbon atoms. Preferably, the term "heterocycloalkyl" includes, unless otherwise indicated, a 3- to 9-membered, preferably a 4-to 7-membered, preferably in each case a 5- to 7-membered, 5- to 6-membered, 6- to 7-membered, or a 3- to 5- membered, in particular a 4- 5-, 6- or 7-membered monocyclic ring.

The term "hetaryl" or "heteroaryl" or "aromatic heterocycle" or "aromatic heterocyclic ring", unless otherwise indicated, includes monocyclic 5- or 6-membered aromatic heterocycles comprising as ring members 1, 2, 3 or 4 heteroatoms. In preferred embodiments, the heterocyclyl is a non-aromatic heterocyclyl.

The term "spirocycle" as used herein, if not indicated otherwise, refers to a group with at least two, preferably two, monocyclic rings, wherein two of the at least two monocyclic rings are connected by a shared atom which is a member of both connected monocyclic rings.

The term "carbospirocycle" as used herein, if not indicated otherwise, refers to a group with at least two, preferably two, carbocycles, wherein two of the at least two carbocycles are connected by a shared atom which is a member of both connected carbocycles.

The term "heterospirocycle" as used herein, if not indicated otherwise, refers to a group with at least two, preferably two, monocyclic rings, wherein the monocyclic rings are connected by a shared atom which is a member of both connected monocyclic rings, and wherein at least one of the monocyclic rings is a heterocycle.

A spirocycle in the context of the invention preferably contains at least one, preferably 1 or 2, heteroatoms, wherein the heteroatoms are preferably N. Preferably, a spirocycle in the context of the invention comprises two monocyclic rings with 4 ring atoms each.

In the context of the invention, the term "alkyl" refers to a saturated straight or branched hydrocarbon. For example, alkyl may in certain embodiments refer to a methyl, ethyl, propyl, isopropyl (also called 2-propyl or 1-methylethyl), butyl, isobutyl, tert-butyl, n-pentyl, iso-pentyl, sec-pentyl, neo-pentyl, 1,2-dimethyl-propyl, iso-amyl, n-hexyl, isohexyl, sec-hexyl, n-heptyl, iso-heptyl, n-octyl, 2-ethyl-hexyl, n-nonyl, n-decyl, n-undecyl, n-dodecyl.

In preferred embodiments, m is 0 to 2, more preferably 0 or 1, most preferably m is 1. In preferred embodiments, m is 0.

In preferred embodiments, Y is not -H. In preferred embodiments, Y is selected from

a nitrile group,

a 5- to 6-membered unsaturated heterocycle comprising 3 hetero atoms that are selected from O and N, preferably wherein 2 or 3 heteroatoms are nitrogen, wherein the heterocycle is mono or di-substituted with =S or =O, preferably with =O;

a linear $-C_{1-3}$alkyl, wherein a, preferably one, $-(CH_2)-$ of the linear $-C_{1-3}$alkyl is optionally monosubstituted with -OH, and wherein the terminal $-CH_3$ of the linear $-C_{1-3}$alkyl is monosubstituted with $-OR^1$ or $-SR^1$, preferably $-OR^1$, wherein $R^1$ is H, $-(CH_2)-COOH$ or $-(CH_2)-(CH_2)-OH$; or

$-(CH_2)_i-CX^1R^2$, wherein

$X^1$ is $=NR^3$ or =O, wherein $R^3$ is -H or -OH.

i is 0 or 1, and $R^2$ is $-OR^4$ or $-NR^5R^6$, wherein $R^4$ is -H or $-C_{1-2}$alkyl; wherein $R^5$ and $R^6$ are independently selected from -H or -methyl.

In preferred embodiments, Y is not -H. In preferred embodiments, Y is not a nitrile group. In preferred embodiments, Y is selected from

a 5- to 6-membered unsaturated heterocycle comprising 3 hetero atoms that are selected from O and N, preferably wherein 2 or 3 heteroatoms are nitrogen, wherein the heterocycle is mono or di-substituted with =S or =O, preferably with =O;

a linear $-C_{1-3}$alkyl, wherein a, preferably one, $-(CH_2)-$ of the linear $-C_{1-3}$alkyl is optionally monosubstituted with -OH, and wherein the terminal $-CH_3$ of the linear $-C_{1-3}$alkyl is monosubstituted with $-OR^1$ or $-SR^1$, preferably $-OR^1$, wherein $R^1$ is H, $-(CH_2)-COOH$ or $-(CH_2)-(CH_2)-OH$; or

$-(CH_2)_i-CX^1R^2$, wherein

$X^1$ is $=NR^3$ or =O, wherein $R^3$ is -H or -OH.

i is 0 or 1, and

$R^2$ is $-OR^4$ or $-NR^5R^6$, wherein $R^4$ is -H or $-C_{1-2}$alkyl; wherein $R^5$ and $R^6$ are independently selected from -H or

-methyl.

In preferred embodiments, Y is not -H. In preferred embodiments, Y is selected from

a 5- to 6-membered unsaturated heterocycle comprising 3 hetero atoms that are selected from O and N, preferably wherein 2 or 3 heteroatoms are nitrogen, wherein the heterocycle is mono or di-substituted with =S or =O, preferably with =O; or

a linear $-C_{1-3}$alkyl, wherein a, preferably one, $-(CH_2)-$ of the linear $-C_{1-3}$alkyl is optionally monosubstituted with -OH, and wherein the terminal $-CH_3$ of the linear $-C_{1-3}$alkyl is monosubstituted with $-OR^1$ or $-SR^1$, preferably $-OR^1$, wherein $R^1$ is H, $-(CH_2)-COOH$ or $-(CH_2)-(CH_2)-OH$.

In preferred embodiments, Y is -H.

In preferred embodiments, Y is a nitrile group.

In preferred embodiments, Y is a 5- to 6-membered unsaturated heterocycle comprising 3 hetero atoms that are selected from O and N, preferably wherein 2 or 3 heteroatoms are nitrogen, wherein the heterocycle is mono or di-substituted with =S or =O, preferably with =O.

In preferred embodiments, Y is selected from a linear $-C_{1-3}$alkyl, wherein a $-(CH_2)-$ of the linear $-C_{1-3}$alkyl is optionally monosubstituted with -OH, and wherein the terminal $-CH_3$ of the linear $-C_{1-3}$alkyl is monosubstituted with $-OR^1$ or-$SR^1$, preferably $-OR^1$, wherein $R^1$ is -H, $-(CH_2)-COOH$ or $-(CH_2)-(CH_2)-OH$. More preferably, Y is selected from a linear $-C_{1-2}$alkyl, wherein a $-(CH_2)-$ of the linear $-C_{1-2}$alkyl is optionally monosubstituted with -OH, and wherein the terminal $-CH_3$ of the linear $-C_{1-2}$alkyl is monosubstituted with $-OR^1$ or $-SR^1$, preferably $-OR^1$, wherein $R^1$ is H, $-(CH_2)-COOH$ or $-(CH_2)-(CH_2)-OH$. Most preferably, Y is a methyl group that is monosubstituted with $-OR^1$ or-$SR^1$, preferably-$OR^1$, wherein $R^1$ is H, $-(CH_2)-COOH$ or-$(CH_2)-(CH_2)-OH$.

In preferred embodiments, Y is selected from a linear $-C_{1-3}$alkyl, wherein a $-(CH_2)-$ of the linear $-C_{1-3}$alkyl is optionally monosubstituted with -OH, and wherein the terminal $-CH_3$ of the linear $-C_{1-3}$alkyl is monosubstituted with $-OR^1$ or $-SR^1$, preferably $-OR^1$, wherein $R^1$ is -H, or $-(CH_2)-COOH$. More preferably, Y is selected from a linear $-C_{1-2}$alkyl, wherein a $-(CH_2)-$ of the linear $-C_{1-2}$alkyl is optionally monosubstituted with -OH, and wherein the terminal $-CH_3$ of the linear $-C_{1-2}$alkyl is monosubstituted with $-OR^1$ or $-SR^1$, preferably $-OR^1$, wherein $R^1$ is H, or $-(CH_2)-COOH$. Most preferably, Y is a methyl group that is monosubstituted with $-OR^1$ or $-SR^1$, preferably $-OR^1$, wherein $R^1$ is H, or $-(CH_2)-COOH$.

In preferred embodiments, Y is selected from a linear $-C_{1-3}$alkyl, wherein a $-(CH_2)-$ of the linear $-C_{1-3}$alkyl is optionally monosubstituted with -OH, and wherein the terminal $-CH_3$ of the linear $-C_{1-3}$alkyl is monosubstituted with $-OR^1$ or $-SR^1$, preferably $-OR^1$, wherein $R^1$ is or $-(CH_2)-COOH$. More preferably, Y is selected from a linear $-C_{1-2}$alkyl, wherein a $-(CH_2)-$ of the linear $-C_{1-2}$alkyl is optionally monosubstituted with -OH, and wherein the terminal $-CH_3$ of the linear $-C_{1-2}$alkyl is monosubstituted with $-OR^1$ or $-SR^1$, preferably $-OR^1$, wherein $R^1$ is-$(CH_2)-COOH$. Most preferably, Y is a methyl group that is monosubstituted with $-OR^1$ or $-SR^1$, preferably $-OR^1$, wherein $R^1$ is $-(CH_2)-COOH$.

In preferred embodiments, Y is $-(CH_2)_i-CX^1R^2$, wherein

$X^1$ is $=NR^3$ or =O, wherein $R^3$ is -H or -OH; i is 0 or 1, and

$R^2$ is $-OR^4$ or $-NR^5R^6$; wherein $R^4$ is -H or $-C_{1-2}$alkyl; wherein $R^5$ and $R^6$ are independently selected from -H or -methyl.

In preferred embodiments, i is 0.

In preferred embodiments, $R^3$ is -OH.

In preferred embodiments, $R^5$ and $R^6$ are both -H.

In preferred embodiments, i is 0, $X^1$ is $=NR^3$, $R^3$ is -OH, $R^2$ is $-NR^5R^6$ and $R^5$ and $R^6$ are both -H.

In preferred embodiments, i is 0 or 1, $X^1$ is =O, $R^2$ is $-NR^5R^6$ and $R^5$ and $R^6$ are both -H.

In preferred embodiments, $X^1$ is =O and $R^2$ is $-OR^4$, wherein $R^4$ is -H or $-C_{1-2}$alkyl; preferably H.

In preferred embodiments, when referring to Y, the 5- to 6-membered unsaturated heterocycle comprising 3 hetero atoms that are nitrogen is

a 5-membered unsaturated heterocycle comprising 3 hetero atoms that are selected from O and N, preferably wherein 2 or 3 heteroatoms are nitrogen, wherein the 5-membered unsaturated heterocycle is monosubstituted with =S or =O, preferably with =O;

a 6-membered heterocycle comprising 3 hetero atoms that are nitrogen, wherein the 6-membered heterocycle is di-substituted with =O or =S, preferably with =O.

In preferred embodiments, when referring to Y, the 5- to 6-membered unsaturated heterocycle comprising 3 hetero atoms that are nitrogen is a 5-membered heterocycle comprising 3 hetero atoms that are nitrogen, wherein the 5-membered heterocycle is monosubstituted with =O.

In preferred embodiments, Y is selected from the group consisting of

| | | | |
|---|---|---|---|
| 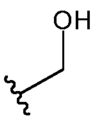, | 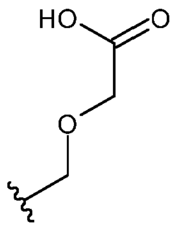, | 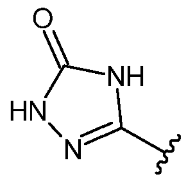, | 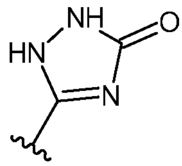, |
| -H, | 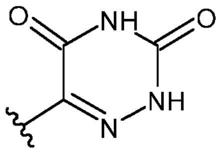, | 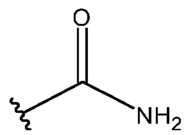, | 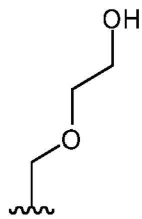, |
| 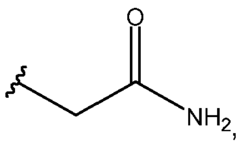, | 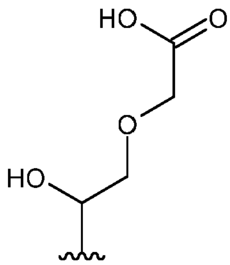, | 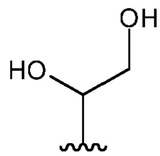, | 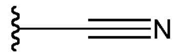, |
| 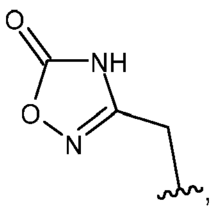, and | 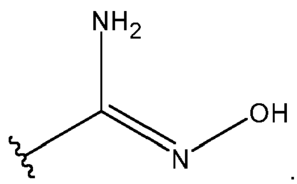. | | |

more preferably, wherein Y selected from the group consisting of

| | | | |
|---|---|---|---|
| 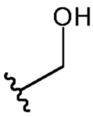, | 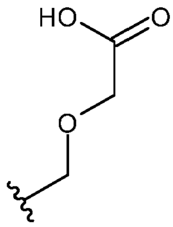, | 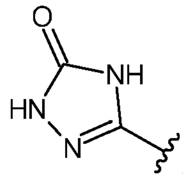, | 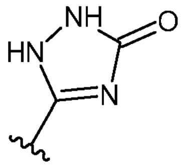, |
| 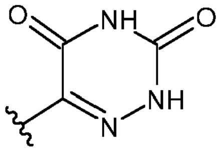, | 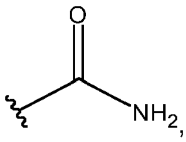, | 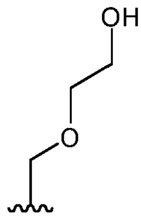, | 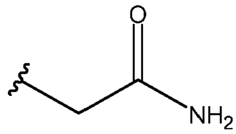 |
| 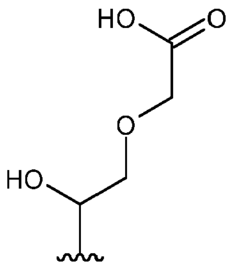 | 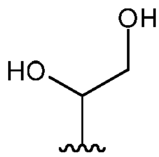, | 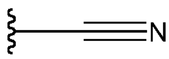, | 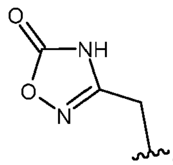, and |
| 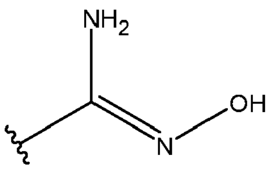, | | | |

even more preferably, wherein Y is selected from the group consisting of

| | | | |
|---|---|---|---|
| 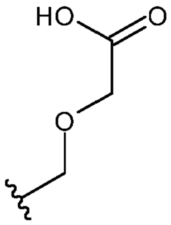 , | 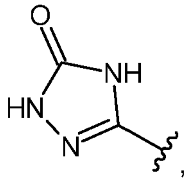 , | 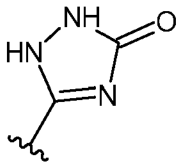 , | 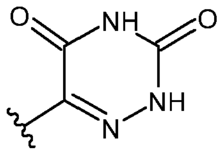 , |
| 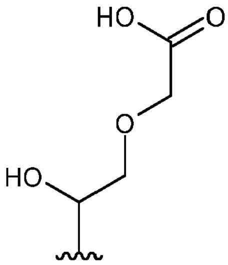 | 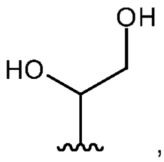 , | 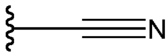 , | 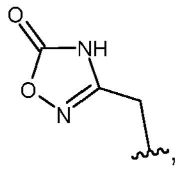 , and |
| 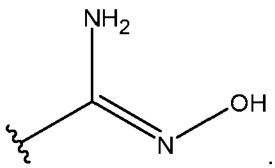 . | | | |

In more preferred embodiments, Y is selected from the group consisting of:

| | | |
|---|---|---|
| 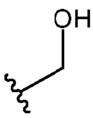 , | 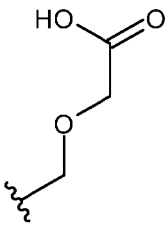 , | 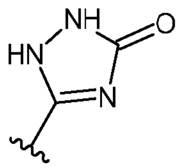 , |
| 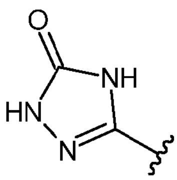 , | 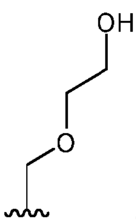 , or | -H, |

more preferably, wherein Y is selected from the group consisting of

| | | |
|---|---|---|
| , | , | , |
| , | . | |

In more preferred embodiments, Y is selected form the group consisting of

| | | |
|---|---|---|
| 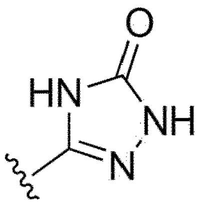 , | 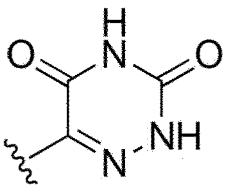 , | 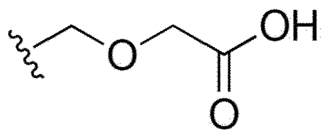 , |
| 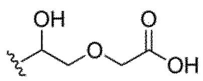 , | 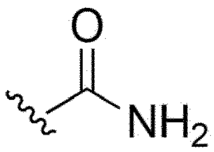 , | 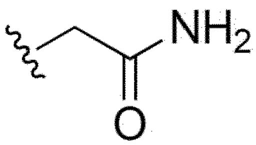 , or |
| 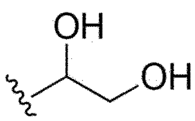 . | | |

In preferred embodiments, Z is an aliphatic group comprising two nitrogen atoms and 1 heterocycle, wherein the first of the two nitrogen atoms connects Z to and the second of the two nitrogen atom connects Z to , preferably to, and the two nitrogen atoms are heteroatoms in a 6- to 7-membered heterocycle with two heteroatoms, wherein the heterocycle is optionally substituted, preferably monosubstituted, with methyl. In preferred embodiments, said 6- to 7-membered heterocycle with two heteroatoms is a 6-membered heterocycle with two heteroatoms. In other preferred embodiments, said 6- to 7-membered heterocycle with two heteroatoms is a 7-membered heterocycle with two heteroatoms.

In preferred embodiments, **Z** is selected a group consisting of:

| | | |
|---|---|---|
| 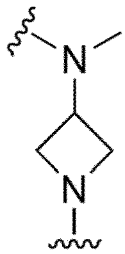 , | 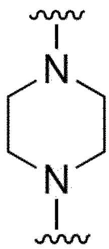 , | 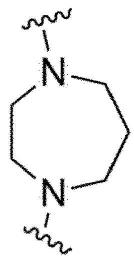 , |
| 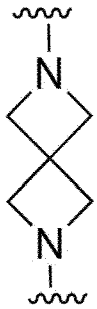 , | 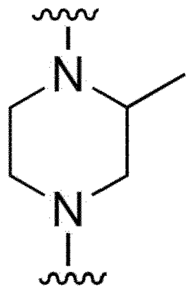 , or | 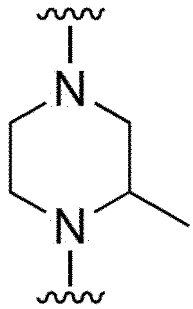 . |

In more preferred embodiments, Z is selected from , or.

In preferred embodiments, B is selected from -Cl or $-CF_3$. In particularly preferred embodiments, B is -Cl. In particularly preferred embodiments, B is $-CF_3$. In preferred embodiments, B is defined as in any other embodiment herein, with the exception that B is not $-CF_3$. In preferred embodiments, B is defined as in any other embodiment herein, with the exception that B is not -Cl.

In preferred embodiments, A is selected from -H or -F.

In preferred embodiments, X is -O-.

In preferred embodiments of the invention,

**A** is one or two substituents independently selected from -H, -F, -Cl, -Br, -I, $-CF_3$, $-OCF_3$, $-CF_2H$, and $-OCF_2H$;

**B** is one or two substituents independently selected from -F, -Cl, -Br, -I, $-CF_3$, - $OCF_3$, $-CF_2H$, and $-OCF_2H$;

**X** is selected from -S- and -O-;

**m** is 1;

**Y** is selected from:

| | | |
|---|---|---|
| 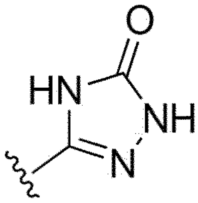 , | 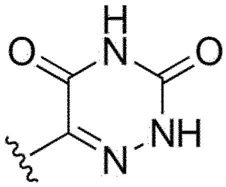 , | 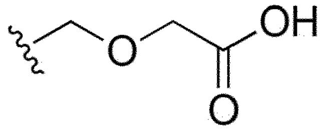 , |
| 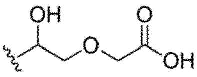 , | 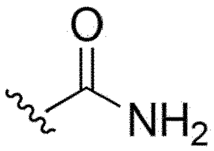 , | 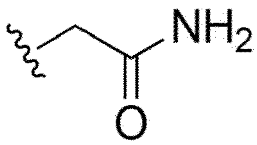 , or |
| 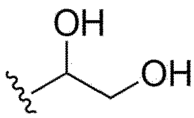 . | | |

and

Z is selected from:

| | | |
|---|---|---|
| 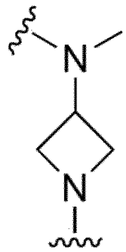 , | 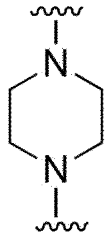 , | 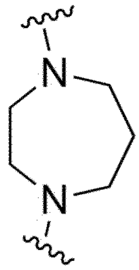 , |
| 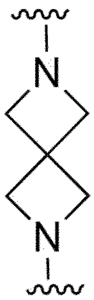 , | 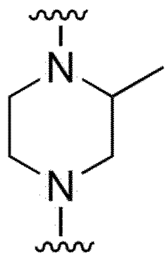 , or | 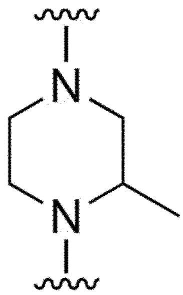 . |

In preferred embodiments, the compound has the formula II

(II),

wherein B, X, A, Y, m and Z are defined as in any of the embodiments herein.

In preferred embodiments, the compound has the formula III

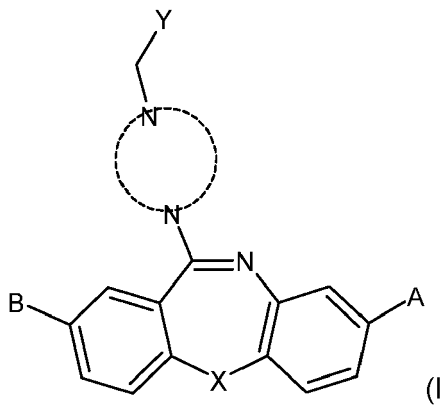

(III),

wherein B, X, A, Y, and Z are defined as in any of the embodiments herein.

In the context of the invention, the compounds are referred to with following numbering:

| Compound NO. | Structure | Compound NO. | Structure |
|---|---|---|---|
| 1, also referred to as Loxapine | 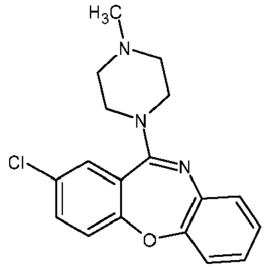 | 2 | 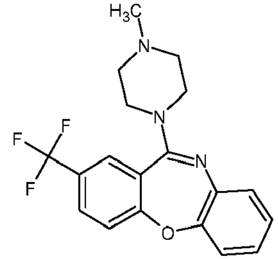 |
| 3 | 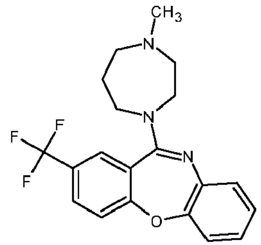 | 4 | 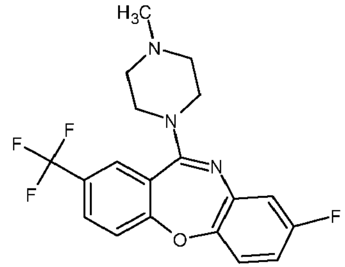 |
| 5 | 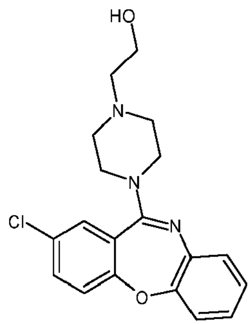 | 6 | 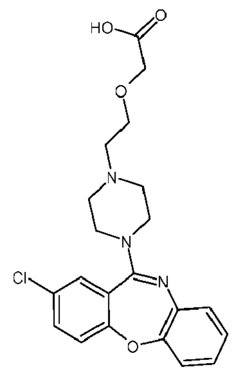 |

(continued)

| Compound NO. | Structure | Compound NO. | Structure |
|---|---|---|---|
| 7 | 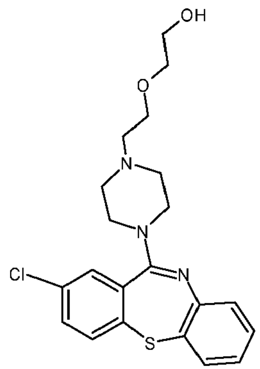 | 8 | 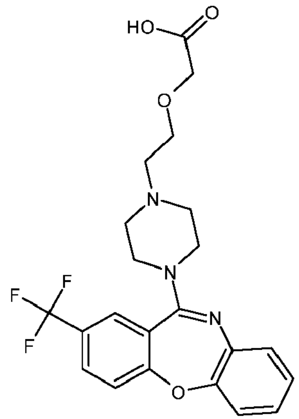 |
| 9 | 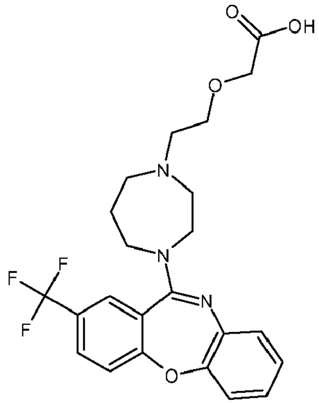 | 10 | 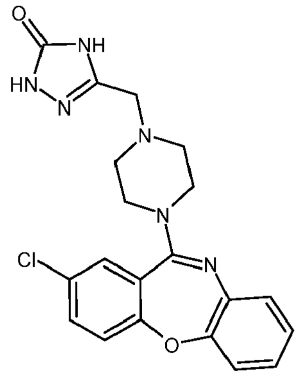 |
| 11 | | 12 | |

(continued)

| Compound NO. | Structure | Compound NO. | Structure |
|---|---|---|---|
| 13 | 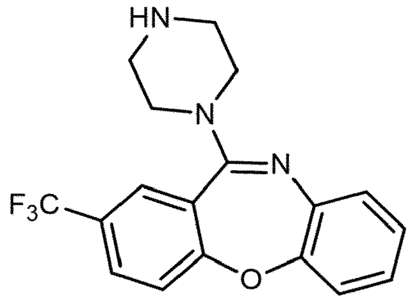 | 14 | 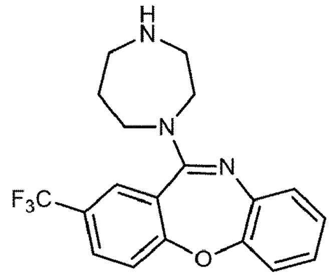 |
| 15 | | 16 | |
| 17 | | 18 | |
| 19 | | 20 | |
| 21 | | 22 | 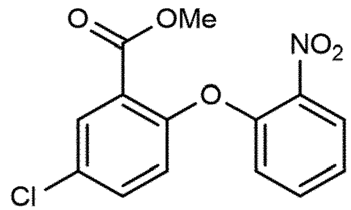 |
| 23 | 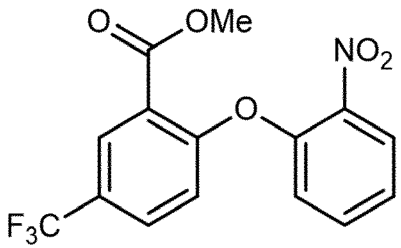 | 24 | 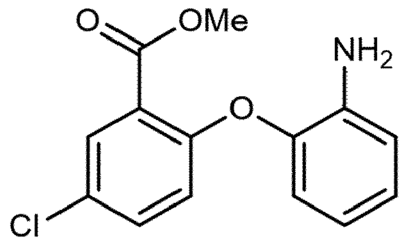 |

(continued)

| Compound NO. | Structure | Compound NO. | Structure |
|---|---|---|---|
| 25 | 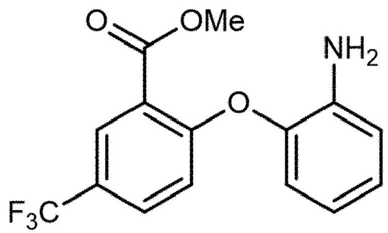 | 26 | 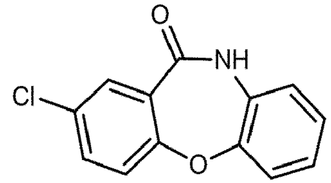 |
| 27 | 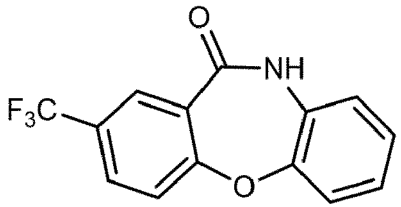 | 28 | 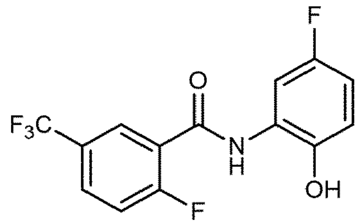 |
| 29 | 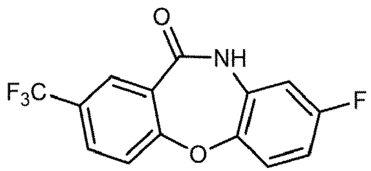 | 30 | 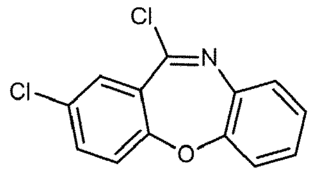 |
| 31 | | 32 | 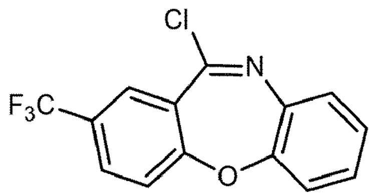 |
| 33 | 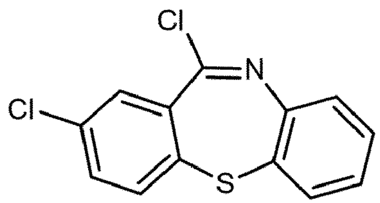 | 34 | 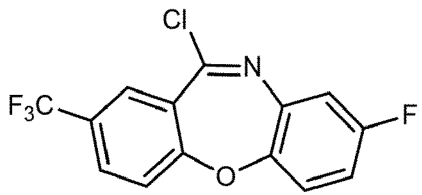 |

(continued)

| Compound NO. | Structure | Compound NO. | Structure |
|---|---|---|---|
| 35 | | 36 | |
| 37 | | 38 | |
| 39 | | 40 | |
| 41 | | 42 | |
| 43 | | | |

In particularly preferred embodiments, the compound is selected from the group consisting of compounds 1 to 21. Preferably, the compound is selected from the group consisting of compounds 2 to 21, preferably the compound is selected from the group consisting of compounds 2, 3, 4, 5, 6, 7, 8, 9, 10, 11, 12, 18, 19, 20, 21, more preferably the compound is selected from the group consisting of compounds 2, 3, 4, 5, 6, 7, 8, 9, and 10.

In particularly preferred embodiments, the compound is selected from the group consisting of compounds 6, 8, 9, 10, 11, 12, 15, 16, 17 ,18 ,19, 20 and 21, preferably the compound is selected from the group consisting of compounds 6, 8, 9, 10, 11, 12, 18 ,19, 20, 21, more preferably the compound is selected from the group consisting of the compounds 6, 8, 9, and 10.

In particularly preferred embodiments, the compound is selected from the group consisting of compounds 6, 7, 8, 9, 10, 11, 15, 16, 17,18,19, 20 and 21, preferably the compound is selected from the group consisting of compounds 6, 7, 8, 9, 10, 11, 18 ,19, 20 and 21, more preferably the compound is selected from the group consisting of compounds 6, 7, 8, 9, and 10.

In particularly preferred embodiments, the compound is selected from the group consisting of compounds 10, 11, 15, 18, 19 and 20. In particularly preferred embodiments, Y is

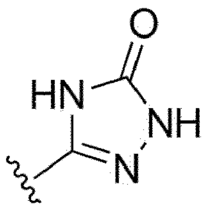

,

and optionally m is 1.

In preferred embodiments of the invention, the compounds are selected from the group consisting of compounds 3, 5-21; preferably the compounds are selected from the group consisting of compounds 3, 5-14; preferably the compounds are selected from the group of compounds 3, 5-11; more preferably the compounds are selected from the group of compounds 3, 5-10. In preferred embodiments, the compound is selected from the group of compounds 3, 5-10, 18-21. In preferred embodiments, the compound is any one of compounds 2 to 21.

In preferred embodiments of the invention, when X is -S-, then Y is not $-(CH)_2-O-CH_2-CH_2-OH$. In preferred embodiments of the invention, when X is -S-, B is -Cl, A is -H, m is 1, and Z is

then Y is not $-(CH)_2-O-CH_2-CH_2-OH$.

In preferred embodiments of the invention, when m is 0, then Y is not -H. In preferred embodiments of the invention, when m is 1, then Y is not -H. In preferred embodiments of the invention, when m is 0, and optionally when B is further Cl, then Y is not $-(CH_2)-(CH_2)-OH$. In preferred embodiments of the invention, when m is 1, and optionally when B is further Cl, then Y is not $-(CH_2)-OH$.

In preferred embodiments of the invention, the compound is not a compound selected from the group of

(Compound 1),

(Compound 2), (Compound 3),

(Compound 3),

(Compound 4),

(Compound 4),

(Compound 5), (Compound 7),

(Compound 13), and

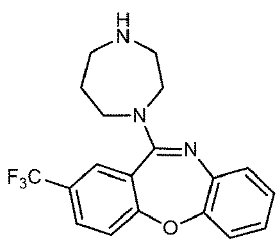 (Compound 14).

In preferred embodiments, the compound has an activity as an activator of a potassium channel in a cell, preferably of a potassium channel Slack ($K_{Na}1.1$). In the context of the invention, the term "Slack" or "Slack channel" refers to a class of sodium-activated potassium channels. These potassium channels are predominantly expressed in neuronal tissue where they are involved in neuronal excitability, burst firing and adaptation of firing rate. Preferably, the Slack channel is preferably encoded by the gene *Kcnt1.* Preferably, the slack channel refers to any isoform of the Potassium channel subfamily T member 1 disclosed in the UniProt entry Q5JUK3 and Q6ZPR4 (at the date of February $2^{nd}$, 2023).

In preferred embodiments, the compound activates the potassium channel with an $EC_{50}$ of less than 100 $\mu$M, preferable of between 0.5 and 80 $\mu$M. In this context, the $EC_{50}$ preferably refers to an $EC_{50}$ that is determined in an $Na^+$ free buffer with 2 mM $Ca^{2+}$, 2 mM $Mg^{2+}$ and 0.03% DMSO.

In preferred embodiments, the compound, which when administered to an animal does not penetrate the blood brain barrier (BBB), or which penetrates the BBB less than a reference compound, such as Loxapine.

In preferred embodiments, the compound does not bind the human dopamine receptor, more preferably wherein the compound binds the human dopamine receptor less compared to a reference compound, such as Loxapine.

In preferred embodiments of the invention, the compound binds to an off-target selected from the group consisting of alpha-1A adrenergic receptor ($\alpha_{1A}$), dopamine receptor $D_1$ ($D_1$), the short form of dopamine receptor $D_2$ ($D_{2S}$ or $D_{2Sh}$), histamine H1 receptor ($H_1$), a serotonin receptor (preferably 5-$HT_{2A}$ and 5-$HT_{2B}$), norepinephrine transporter (NET), serotonin transporter (SET), cannabinoid receptor 1 (CB1). In preferred embodiments of the invention, the compound binds to a target selected from the group consisting of alpha-1A adrenergic receptor ($\alpha_{1A}$), dopamine receptor $D_1$ ($D_1$), the short form of dopamine receptor $D_2$ ($D_{2S}$ or $D_{2Sh}$), histamine H1 receptor ($H_1$), a serotonin receptor (preferably 5-$HT_{2A}$ and 5-$HT_{2B}$), norepinephrine transporter (NET), and serotonin transporter (SET). In preferred embodiments of the invention, the compound binds to a target selected from the group consisting of dopamine receptor $D_1$ ($D_1$), Histamine H1 receptor ($H_1$), a serotonin receptor (preferably 5-$HT_{2A}$ and 5-$HT_{2B}$). By binding to such an off-target, the antipruritic effect of the compounds of the invention may be further increased. In preferred embodiments, the compound binds to a $H_1$ receptor (preferably, wherein the compound of the invention is an antagonist of $H_1$ receptor). In further preferred embodiments, the compound of the invention binds to a 5-HT receptor, more preferably a 5-$HT_2$ receptor, in particular a 5-$HT_{2A}$ receptor, preferably, wherein the compound is an agonist of the 5-HT receptor, 5-$HT_2$ receptor, and/or 5-$HT_{2A}$ receptor.

In preferred embodiments of the invention, the compound is Compound 6, Compound 8 Compound 10 or Compound 11. In preferred embodiments of the invention, the compound is Compound 6, Compound 8 or Compound 10. In more preferred embodiments of the invention, the compound is Compound 6 or Compound 10.

In preferred embodiments of the invention, the compound is isolated. In preferred embodiments, the compound is dissolved in a solvent or a dispersion. In preferred embodiments, the compound has a purity of at least 75%, optionally at least 90%, optionally at least 95%. In this context, the purity refers to the absence of any foreign material (in particular chemical substances) which may be present in a composition comprising the compound of the invention. Impurities may occur naturally, may be added or generated during the synthesis and/or purification of the compound of the invention. For example, impurities include a starting material, a solvent, an intermediate or a reactant, a degradation product of any of the foregoing or of a desired compound, leftovers of protecting groups after deprotection, and combinations thereof.

The compounds of the present invention may exist in tautomeric forms. Preferably, the depiction of a single tautomer, for example in a functional group or a compound, is understood to represent the compound or group in all its tautomeric forms. For example, if not indicated otherwise, the compounds 10, 11, 15, 17, 18, 19, and/or 20 preferably each refer to both the following tautomeric forms:

| | | | |
|---|---|---|---|
| Compound 10 tautomer 1 | 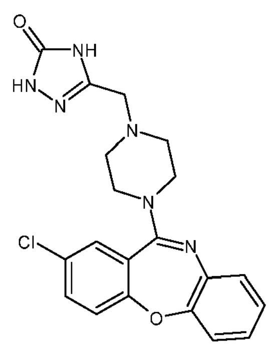 | Compound 10 tautomer 2 | 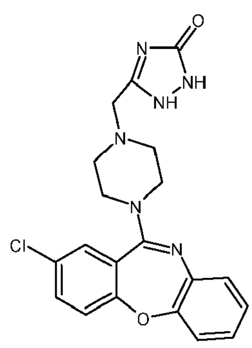 |

(continued)

| | | | |
|---|---|---|---|
| Compound 11 tautomer 1 | 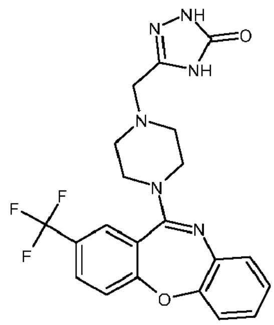 | Compound 11 tautomer 2 | 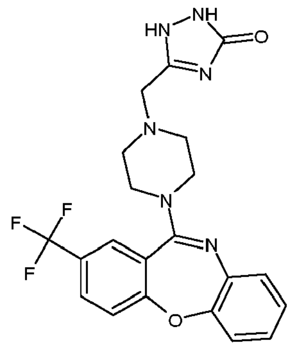 |
| Compound 15 tautomer 1 | 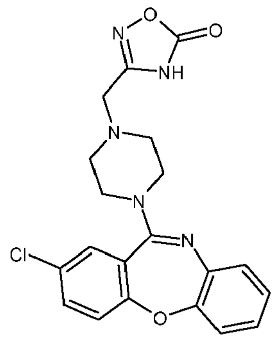 | Compound 15 tautomer 2 | 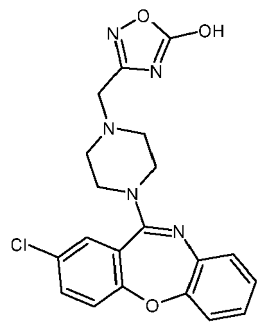 |
| Compound 17 tautomer 1 | 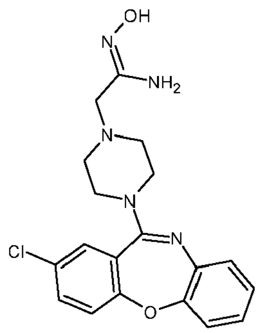 | Compound 17 tautomer 2 | 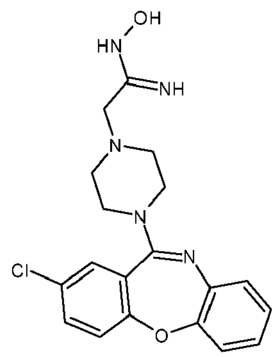 |
| Compound 18 tautomer 1 | 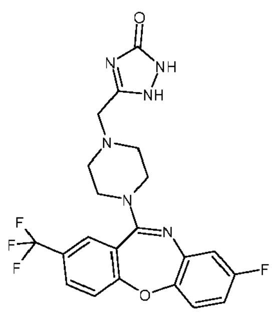 | Compound 18 tautomer 2 | 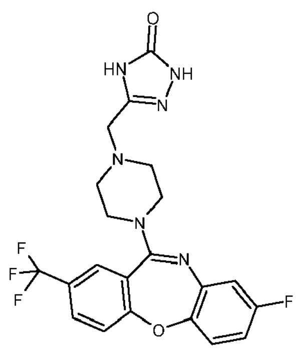 |

(continued)

| | | | |
|---|---|---|---|
| Compound 19 tautomer 1 | 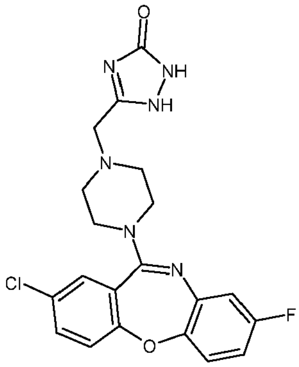 | Compound 19 tautomer 2 | 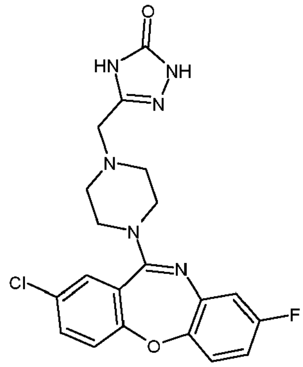 |
| Compound 20 tautomer 1 | 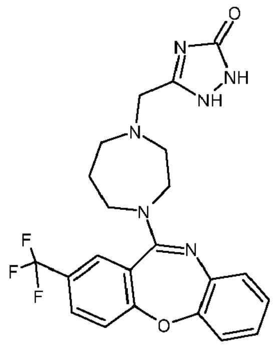 | Compound 20 tautomer 2 | 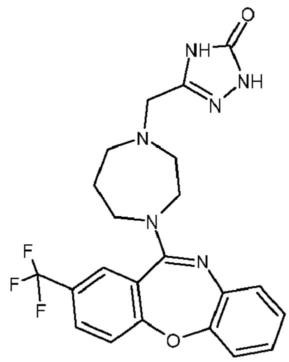 |

Preferably, Compound 10 is Compound 10 tautomer 1 or Compound 10 tautomer 2. Preferably, Compound 11 is Compound 11 tautomer 1 or Compound 11 tautomer 2. Preferably, Compound 15 is Compound 15 tautomer 1 or Compound 15 tautomer 2. Preferably, Compound 17 is Compound 17 tautomer 1 or Compound 17 tautomer 2. Preferably, Compound 18 is Compound 18 tautomer 1 or Compound 18 tautomer 2. Preferably, Compound 19 is Compound 19 tautomer 1 or Compound 19 tautomer 2. Preferably, Compound 20 is Compound 20 tautomer 1 or Compound 20 tautomer 2.

**In a second aspect,** the invention pertains to a pharmaceutical composition comprising the compound of any one of the aspects herein, or a salt, solvate, or ester thereof, and a pharmaceutically acceptable carrier or excipient.

Preferably, the pharmaceutically acceptable composition comprises the compound of any aspect of the invention, wherein the compound is in the form of a prodrug, a salt, a racemic mixture, a crystalline from, a polymorph, a solvate or combinations thereof.

The term "pharmaceutically acceptable carrier or excipient" means a carrier or excipient that is useful in preparing a pharmaceutical composition that is generally safe, non-toxic and neither biologically nor otherwise undesirable, and includes excipients that are acceptable for veterinary use as well as human pharmaceutical use. It includes solvents, emulsifiers, suspending agents, disintegrators, binding agents, stabilizing agents, diluents, gelling agents, preservatives, lubricants, surfactants and other similar carriers. Such a pharmaceutically carrier or excipient must be "acceptable" in the sense of being compatible with the other ingredients of a subject composition and not injurious to the patient.

In preferred embodiments of the pharmaceutical composition, the compound, or a salt, solvate, or ester thereof, is present in an amount of 0.1% w/w to 10% w/w, optionally in an amount of 1% w/w to 10% w/w. In preferred embodiments, the salt of the compound is a hydrochloride salt of the compound.

In preferred embodiments of the pharmaceutical composition, the composition is an oral composition, a topical composition, or an infusion. In preferred embodiments of the pharmaceutical composition, the composition is an oral composition, a topical composition, or a parenteral composition. In preferred embodiments, the composition is in the form of a patch, suppository, syringe, injector pen, spray bottle, mask, pastille and/or implant inhaler, nebulizer, creme and/or vaporizer. In preferred embodiments of the pharmaceutical composition is a unit dosage form. In particular preferred embodiments, the composition is in the form of a creme.

In preferred embodiments of the composition, the composition the dosage form is an extended-release dosage form. Preferably the extended release dosage form releases the compound over the course of 5 h, 10 h, 18 h, 24 h, 36 h, 48 h, 72 h, 1 week, 2 weeks, 1 month, 6 months, 12 months and/or 2 years. In this context, "over the course" encompasses a release, wherein the compound is continuously, periodically /or non-periodically released to the body

of the subject. Preferably, the extended release dosage form is an implant. Preferably, the implant comprises the compound of the first aspect of the invention.

**In a third aspect,** the invention pertains to a compound or composition for use in the treatment of a condition in a subject, the composition composition comprising an effective amount of the compound of the first aspect of the invention, or a salt, solvate, or ester thereof, wherein the condition is treatable by activating a potassium channel in a cell associated with the pathology of the condition, the treatment comprising administering the compound or composition to the subject in need thereof.

Preferably, the subject of the invention is an animal. In this context, the term "animal" includes humans. Preferably, the animal is a mammal or bird, most preferably a mammal. Preferably, the mammal is a human. Preferably, the mammal is a non-human primates, dog, cat, sheep, cattle, goat, pig, mouse, rat, rabbit or guinea pig.

In this context the term "treatment" includes the application or administration of a therapeutic agent (such as a compound of the invention) or procedure to a patient in need thereof, purpose to cure, heal, alleviate, relieve, alter, remedy, ameliorate, improve, affect or prevent the condition, the symptoms of the condition, or the predisposition toward the condition. Hence, the term "treatment" can include prophylactic treatment of a condition the symptoms of a condition.

Preferably, the application or administration comprises intravenous, intraperitoneal, subcutaneous, intramuscular, intrathecal, peridural, topical, oral, gastric and/or rectal administration. In preferred embodiments, the application or administration comprises intrathecal, and/or peridural, administration. In preferred embodiments, the application or administration comprises intravenous, intraperitoneal, subcutaneous, intramuscular, topical, oral, gastric and/or rectal administration. CNS administration, in particular intrathecal or peridural administration, is likely to result in a more potent analgesic effect in case of neuropathic pain compared to other types of administration such as than oral or i.p. administration.

In preferred embodiments, the condition is a pruritic condition, such as an acute or chronic pruritus. In preferred embodiments, the pruritic condition is associated with a second pathology, such as one selected from:

a dermatological disorder such as xerosis or xeroderma (dry skin), dermatitis or eczema (e.g., atopic dermatitis), psoriasis (e.g., plaque psoriasis), prurigo (e.g., prurigo nodularis), urticaria (e.g., chronic idiopathic urticaria), a connective tissue disorder (e.g., dermatomyositis), post-burn pruritus;

a kidney disorder (e.g., chronic kidney disease, chronic kidney failure or end-stage renal disease), dialysis (e.g., hemodialysis), uremic pruritus;

a hepato-biliary disorder (e.g., cholestasis, primary biliary cholangitis, primary sclerosing cholangitis, secondary sclerosing cholangitis, hepatitis, toxic liver disease, chronic liver disease or cirrhosis), cholestatic pruritus;

an endocrine disorder (e.g., hyperthyroidism or diabetes mellitus);

a metabolic disorder (e.g., iron deficiency or iron overload);

a benign or malignant neoplasm (e.g., a solid tumor, a carcinoma or a hematological neoplasm [e.g., Hodgkin's lymphoma, non-Hodgkin's lymphoma, myeloproliferative disease, or polycythemia vera]);

an infectious disease (e.g., a viral infection such as an infection with herpes simplex, herpes zoster, varicella, human immunodeficiency virus (HIV), hepatitis; a bacterial infection or a parasitosis);

a neurological disorder (e.g., degenerative neurological diseases, multiple sclerosis, brain tumors, postherpetic neuralgia, small-fiber neuropathies, brachioradial pruritus or notalgia paresthetica), neuropathic itch, neurogenic itch;

a psychiatric disease (e.g., depression, obsessive compulsive disorder, delusional disorder, eating disorder, or anxiety);

drug-induced pruritus (e.g., by opioids, antibiotics, antimalarial agents, ACE inhibitors, angiotensin receptor antagonists, anti-arrhythmic agents, antidepressants, antidiabetic drugs, antihypertensive drugs, anticonvulsants, anti-inflammatory drugs, betablockers, bronchodilators, calcium antagonists, diuretics, hormones, immunosuppressive drugs, antilipids, neuroleptics, plasma expanders, tranquillizers, or uricostatics);

pruritus in the elderly;

pruritus in pregnancy; or/and

chronic idiopathic pruritus.

In preferred embodiments of the invention, the pruritic condition is associated with second pathology, such as one selected from:

- a dermatological disorder such as xerosis or xeroderma (dry skin), dermatitis or eczema (e.g., atopic dermatitis), psoriasis (e.g., plaque psoriasis), prurigo (e.g., prurigo nodularis), urticaria (e.g., chronic idiopathic urticaria), a connective tissue disorder (e.g., dermatomyositis), post-burn pruritus;
- a kidney disorder (e.g., chronic kidney disease, chronic kidney failure or end-stage renal disease), dialysis (e.g., hemodialysis), uremic pruritus;
- a hepato-biliary disorder (e.g., cholestasis, primary biliary cholangitis, hepatitis, chronic liver disease or cirrhosis), cholestatic pruritus;
- a benign or malignant neoplasm (e.g., a solid tumor, a carcinoma or a hematological neoplasm [e.g., Hodgkin's lymphoma, non-Hodgkin's lymphoma or polycythemia vera]);
- neuropathic itch (e.g., brachioradial pruritus or notalgia paresthetica), neurogenic itch:
- drug-induced pruritus (e.g., by opioids),
- pruritus in the elderly, or/and
- chronic idiopathic pruritus.

In preferred embodiments, the myeloproliferative disease is a myeloproliferative neoplasm.

In preferred embodiments, the composition for use is the pharmaceutical composition of the second aspect of the invention. Additionally, the treatment may comprise applying or administering further antipruritic drugs before, simultaneously or after the application or administration of the compound or composition of the invention. Preferably, the further antipruritic drug is selected from a systemic, oral and/or topical drug. Preferably, the further antipruritic drug is selected from the group of corticosteroids, antihistamines, anesthetics, phosphodiesterase-4 inhibitors, capsaicin, μ-opioid receptor antagonists, antidepressants, immunosuppressants, anticonvulsants, janus kinase inhibitors, κ-opioid receptor agonist and Thalidomide- and/or Butorphanol-based drugs.

Preferably, the treatment according to the present invention is conducted before, simultaneously or after further antipruritic treatments, preferably wherein the further antipruritic treatment is selected from the group of applying or administering aluminum triacetate solution, olive oil, jewelweed, calamine lotion, sodium bicarbonate paste, ammonium hydroxide and/or papain-based topical creams, cannabis, pigweed (portulaca oleracaea), ashoka (sarco asoca or saraca asoca), fig (fificus carica) and/or cannabinoids.

In preferred embodiments, the condition is pain or a pain related noxious sensation in the subject.

In preferred embodiments, the pain or a pain related noxious sensation is selected from neuropathic pain induced by traumatic nerve injury, cancer and cancer treatments (e.g., chemotherapy), neurological conditions (e.g., multiple sclerosis), neurodegenerative conditions (e.g., Parkinson's disease), trigeminal neuralgia, diabetic peripheral neuropathy, stroke, shingles, HIV, Hansen's disease (leprosy), Guillain-Barre syndrome, blood vessel disease, vascular malformations, autoimmune conditions,

acute post-operative pain,

inflammatory pain, rheumatoid arthritis, osteoarthritis, and/or

nociplastic pain.

In preferred embodiments, the composition comprises at least one additional therapeutically active and/or pain reducing agent. Preferably, the at least one additional therapeutic agent is an agent for the treatment of the second pathology as mentioned herein.

**In a fourth aspect,** the invention pertains to a method of synthesizing a compound of the first aspect of the invention.

In this context the method of synthesizing a compound may include one or more synthesis steps. Preferably,

the synthesis step is a chemical reaction step and/or a purification step.

In particular, the method of providing a compound can comprise any of the synthesis steps disclosed herein and/or synthesis steps analogous thereto, which are readily known and available to one of ordinary skill in the art of organic synthesis.

In particular, the method of providing a compound can comprise any of the chemical reaction steps disclosed herein and/or chemical reaction steps analogous thereto, which are readily known and available to one of ordinary skill in the art of organic synthesis.

In particular, the method of providing a compound can comprise any of the purification steps disclosed herein and/or purification steps analogous thereto, which are readily known and available to one of ordinary skill in the art of organic synthesis.

Preferably, the chemical reaction step is followed or preceded by a purification step.

Purification in this context refers to any suitable method for removing an impurity fraction. Such purification methods are well known and include e.g., column chromatography, selective precipitation, trituration and elution of impurities with a suitable solvent in which the desired compound is not soluble, etc. The fraction of impurities removed may be such that the compound is prepared in substantially pure form; that is, for example, in a percentage purity as described above. In other embodiments, the compound of the invention is provided by said method in an impure form.

The method of synthesizing a compound according to the invention may be started, interrupted and continued at any point. Preferably, the method of synthesizing a compound according to the invention is interrupted after a chemical reaction step or a purification step. If possible, the method includes the isolation of by-products or intermediates of the chemical reaction steps of the invention.

A synthesis step, such as a chemical reaction step or a purification step, according to the invention may be conducted using any suitable solvent. For example, the choice of a solvent may depend on the stability of the products, the polarity of the solvent and/or the compound, the boiling point of the solvent, the acidity of the solvent, if a solvent is protic/aprotic and/or the biocompatibility of the solvent. Preferred solvents include aliphatic alcohols (such as methanol, ethanol, n-propanol, isopropanol), acetone, acetonitrile, ether, *N*,*N*-dimethylformamide (DMF), tetrahydrofuran (THF), dichloromethane, water or a mixture of two or more of these liquids. Preferably, a synthesis step according to the invention may be carried out under an inert gas such as nitrogen, helium, neon, argon, krypton, xenon, radon and/or sulphur hexafluoride. Moreover, the solvent, which is used in the synthesis step, for example in the chemical reaction step or the purification step, may be cleansed using such a gas.

In preferred embodiments, the method of synthesizing a compound according to the invention comprises a synthesis step, preferably a chemical reaction step, of synthesizing a lactam core. Preferably, said lactam core comprises the structure

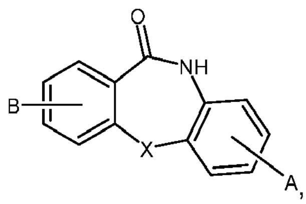

wherein A, X and B are defined as in any embodiment herein.

In preferred embodiments, the lactam core of the invention is synthesized according to Synthesis Route A or Synthesis Route B comprising the corresponding chemical reaction steps shown in Table 1111Table 11. The individual chemical reaction steps in Synthesis Routes A and Synthesis Route B are preferably preformed consecutively, therefore in the order

- Chemical Reaction Step 1a → Chemical Reaction Step 2a 4 Chemical Reaction Step 3a, or
- Chemical Reaction Step 1b → Chemical Reaction Step 2b.

**Table** 11114: Chemical Reaction Steps of Synthesis Route A and Synthesis Route B.

| **Synthesis Route A** |
|---|
| 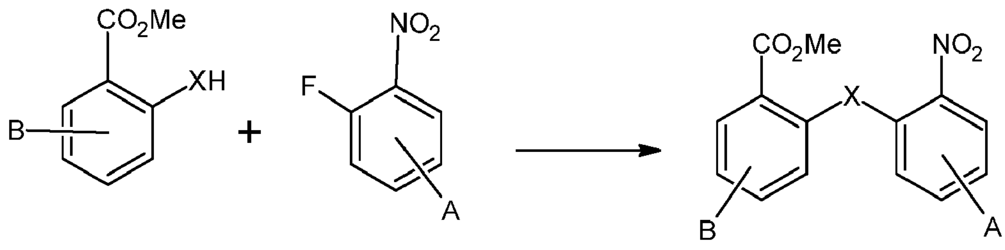<br>Chemical Reaction Step 1a |
| 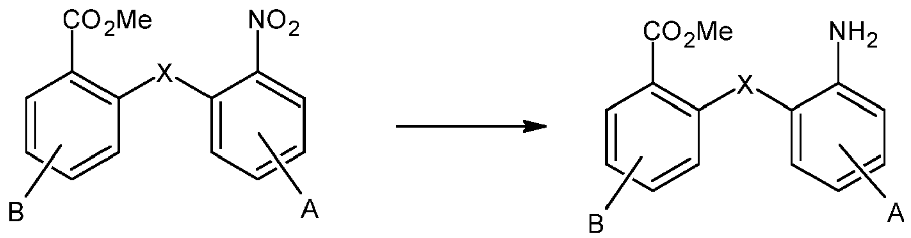<br>Chemical Reaction Step 2a |
| 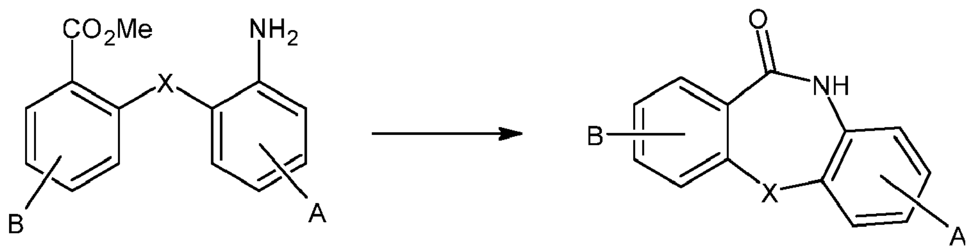 |
| Chemical Reaction Step 3a |
| **Synthesis Route B** |
| 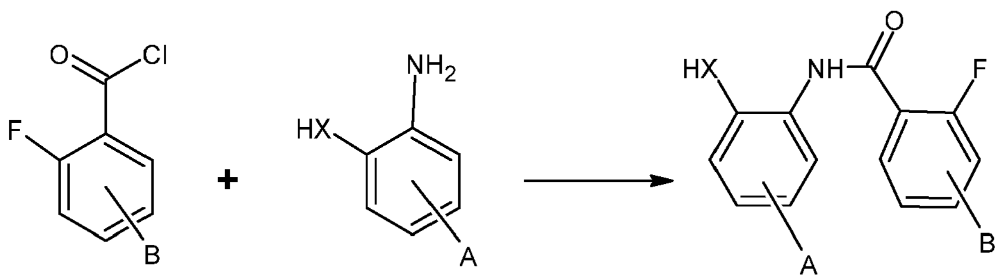<br>Chemical Reaction Step 1b |

(continued)

| Synthesis Route B |
|---|
| 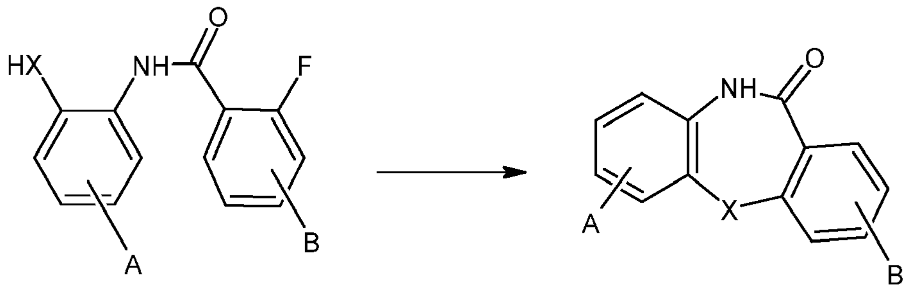 Chemical Reaction Step 2b |

In further preferred embodiments, the method of synthesizing a compound according to the invention comprises a synthesis step, preferably a chemical reaction step, of converting a lactam core to an imidoyl chloride derivative thereof and/or converting the lactam core and/or the imidoyl chloride derivative thereof to a compound of the invention. Said converting a lactam core to an imidoyl chloride derivative thereof and/or converting the lactam core and/or the imidoyl chloride derivative thereof to a compound of the invention is preferably conducted using the Synthesis Route C with their corresponding Chemical Reaction steps shown in Table 2222Table 22. Preferably, the individual Chemical Reaction steps in Synthesis Route C are performed consecutively, therefore in the order of
Chemical Reaction Step 1c → Chemical Reaction Step Reaction 2c.

**Table** 2222: **Chemical reaction steps of Synthesis Route C**

| Synthesis Route C |
|---|
| 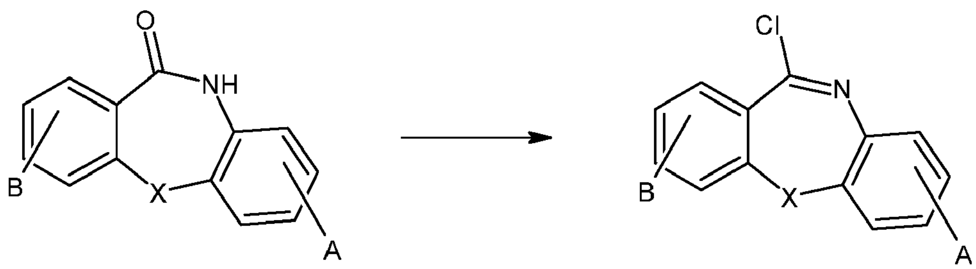 Chemical Reaction Step 1c |
| 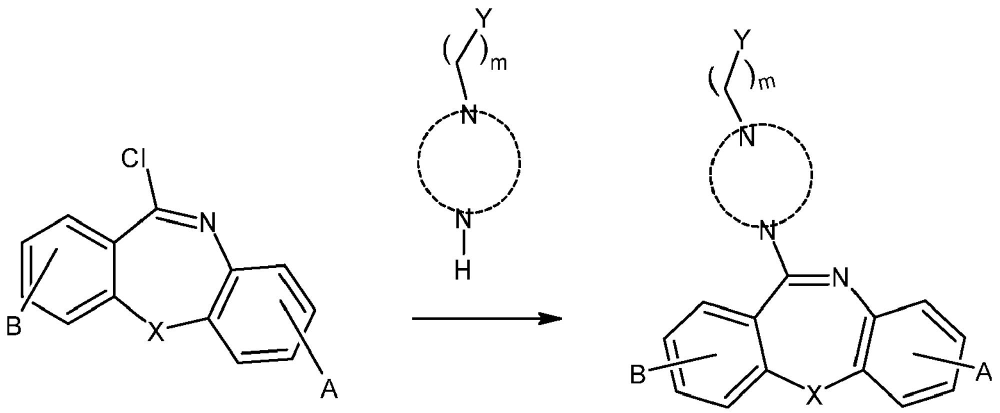 Chemical Reaction Step 2c, |

(continued)

| **Synthesis Route C** | | |
|---|---|---|
| wherein | 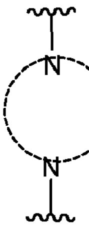 , | m, and Y are defined according to any other aspect of the invention. |

In preferred embodiments, the method of synthesizing a compound according to the invention comprises conducting the Synthesis Route A followed by conducting the Synthesis Route C. In preferred embodiments, the method of synthesizing a compound according to the invention comprises conducting the Synthesis Route B followed by conducting the Synthesis Route C.

In preferred embodiments, Synthesis Route B comprises an additional Chemical Reaction Step 0b that is conducted before the Chemical Reaction Step 1b.

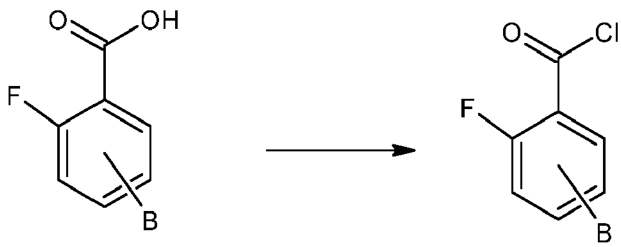 (Chemical Reaction Step 0b).

In preferred embodiments of the invention, when

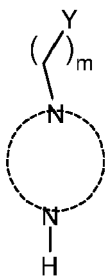 is 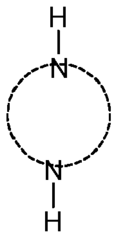 ,

preferably when

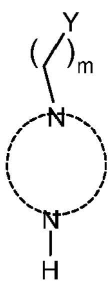

is piperazine or homopiperazine, the Synthesis Route C comprises a Chemical Reaction Step 3c after the Chemical Reaction Step 2c,

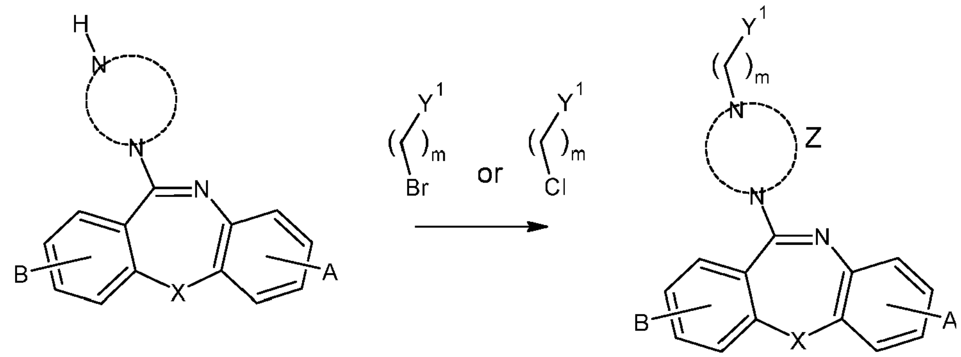


(Chemical Reaction Step 3c),

wherein Y' is defined as Y with the additional feature that Y' is not H.

In preferred embodiments, the method of synthesizing a compound according to the invention comprises starting the synthesis at any synthesis step, preferably at any chemical reaction step disclosed herein or at a purification step prior or after any chemical reaction step disclosed herein. In preferred embodiments, the method of synthesizing a compound according to the invention comprises conducting a reaction with a reaction intermediate or reaction product of a synthesis step, preferably a chemical reaction step, disclosed herein, preferably in the Synthesis Routes A, B and/or C.

In preferred embodiments, the method of synthesizing a compound according to the invention comprises conducting at least one synthesis step, preferably at least one chemical reaction step, disclosed herein, preferably in the Synthesis Routes A, B and/or C.

In preferred embodiments of the invention, the Chemical Reaction Step 1a is conducted in a solvent containing potassium carbonate. In preferred embodiments, Chemical Reaction Step 1a is conducted in *N*,*N*-dimethylformamide (DMF). In preferred embodiments, Chemical Reaction Step 1a is conducted at 100 to 140 °C most preferably at 120 °C.

In preferred embodiments, Chemical Reaction Step 2a is conducted using a reduction agent, preferably wherein the reduction agent is $SnCl_2 \cdot 2H_2O$, and optionally wherein the Chemical Reaction Step 2a is conducted in a mixture of ethanol/conc. HCl 1:1.

In preferred embodiments, the Chemical Reaction Step 3a comprises treatment of

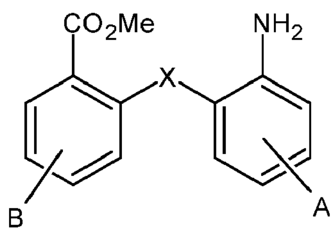

with an acid, more preferably wherein the acid is sulfuric acid, most preferably concentrated sulfuric acid. In preferred embodiments, the reaction is conducted in DMF. In preferred embodiments, Chemical Reaction Step 3a is conducted in DMF at 100 to 140 °C, most preferably at 120 °C.

In preferred embodiments the Chemical Reaction Step 0b comprises the reaction of addition of thionyl chloride to

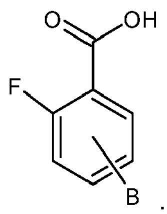

In preferred embodiments of the invention, the Chemical Reaction Step 1b comprises the dropwise addition of

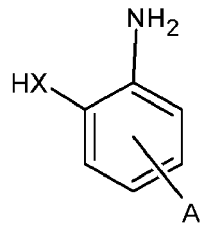

to a solution of

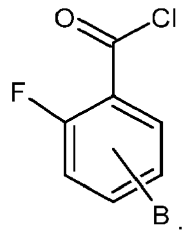

In preferred embodiments, the Chemical Reaction Step 1b is -20 to 50°C, more preferably from -10 to 30 °C, most preferably at 0 °C.

In preferred embodiments of the invention, the Chemical Reaction Step 2b is conducted in a basic solution, preferably wherein the basic solution comprises NaOH. In preferred embodiments, Chemical Reaction Step 2b is conducted in DMF. In preferred embodiments of the invention, Chemical Reaction Step 2b is conducted at 120 to 170 °C, more preferably at 140 to 160 °C, most preferably at 150 °C.

In preferred embodiments, Chemical Reaction Step 1c is conducted in phosphorus oxychloride, more preferably under reflux, and optionally, wherein the phosphorus oxychloride comprises N,N-dimethylaniline.

In preferred embodiments of the invention, the Chemical Reaction Step 2c is conducted in p-xylene, optionally wherein the reaction is conducted at 110 to 150 °C, preferably at 120 to 140 °C.

In preferred embodiments of the invention, the Chemical Reaction Step 3c is conducted with an additional base in the reaction mixture, preferably wherein the additional base is triethylamine.

In preferred embodiments, in particular when referring to the first and the fourth aspect of the invention, A is one substituent. In preferred embodiments, A is two substituents. In preferred embodiments, B is one substituent. In preferred embodiments, B is two substituents. Preferably, when B is two substituents, B is a substituent at the carbon positions 2 and 3, more preferably B is the substituent -Cl at the carbon positions 2 and 3. In preferred embodiments, A and B are each one substituent.

In preferred embodiments, in particular when referring to the first and the fourth aspect of the invention, A is one substituent. In preferred embodiments, A is two substituents. In preferred embodiments, B is one substituent. In preferred embodiments, B is two substituents. Preferably, when B is two substituents, B is a substituent at the carbon positions 3 and 4, more preferably B is the substituent -Cl at the carbon positions 3 and 4. In preferred embodiments, A and B are each one substituent.

In the context of the invention, with respect to the structure (I), when referring to the carbon positions of A and/or B, the numbering preferably refers to the following:

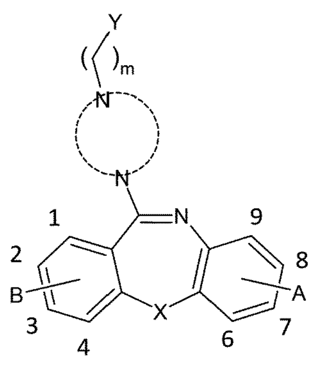

Preferably, in the Synthesis Routes A-C, the positions of the substituents A and B for each the respective reactants is selected in agreement with the positioning of the substituents A and B in the structure of (I).

In preferred embodiments of the invention, when referring to the chemical reaction steps of the invention,

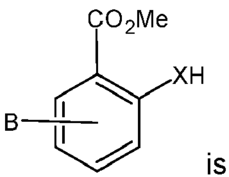 is 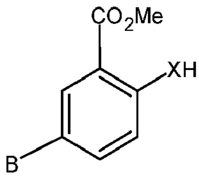, 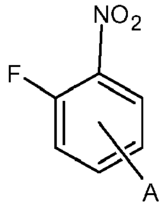 is 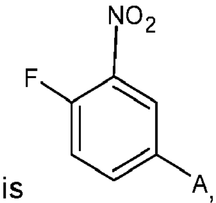,

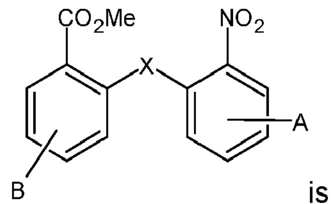 is 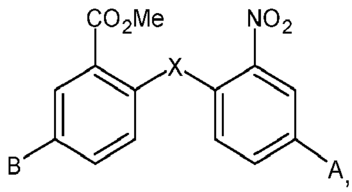,

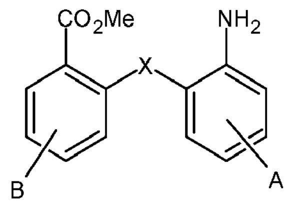 is 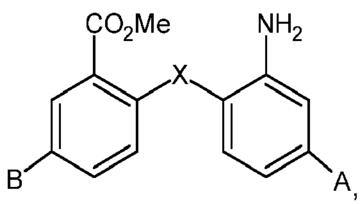,

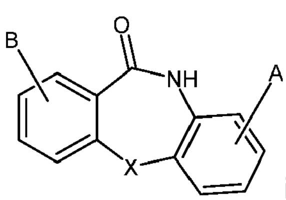 is 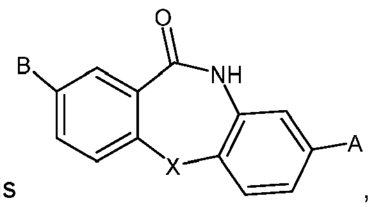, 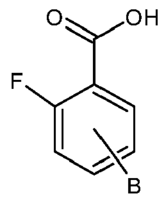 is 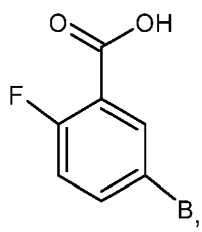,

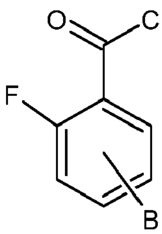 is 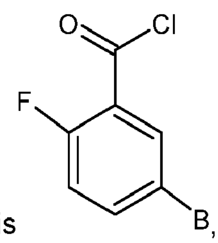, 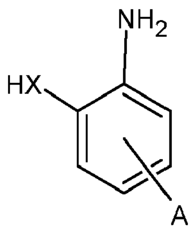 is 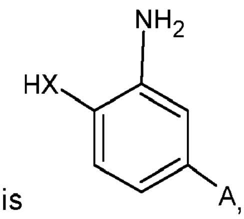,

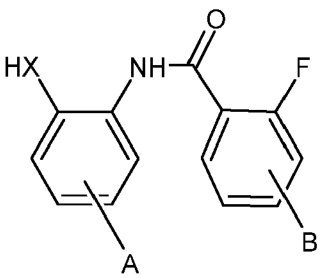 is 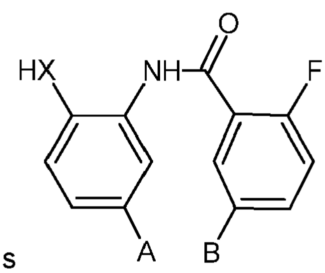,

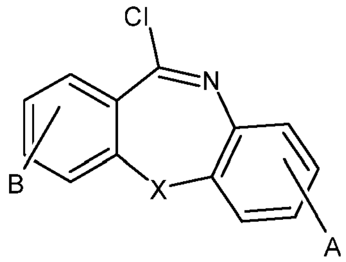 is, 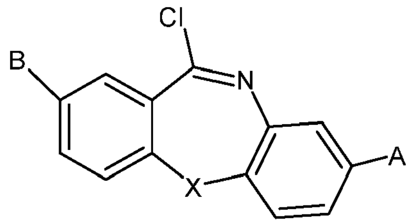

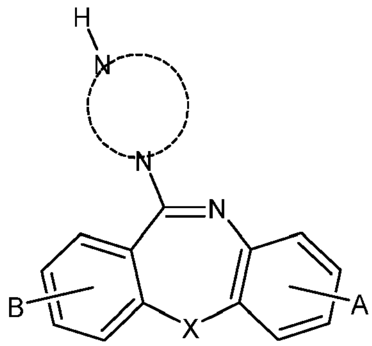 is, 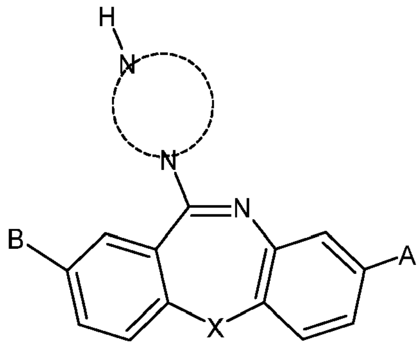 ,

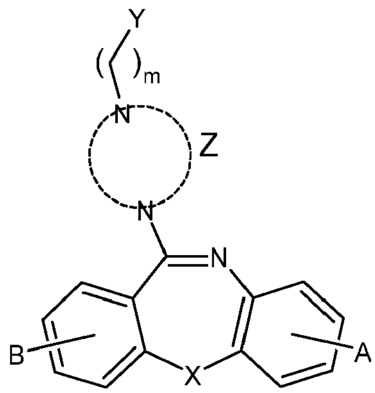 is 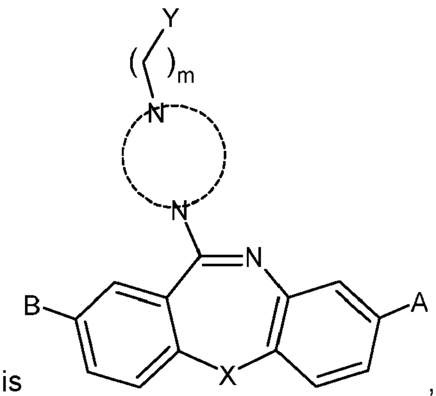 ,

and

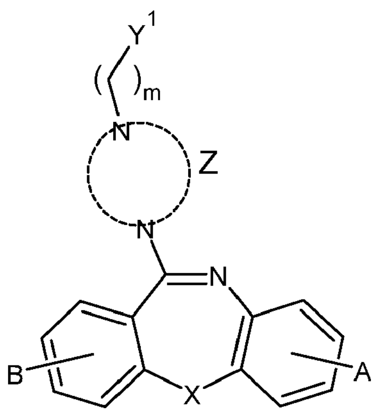 is 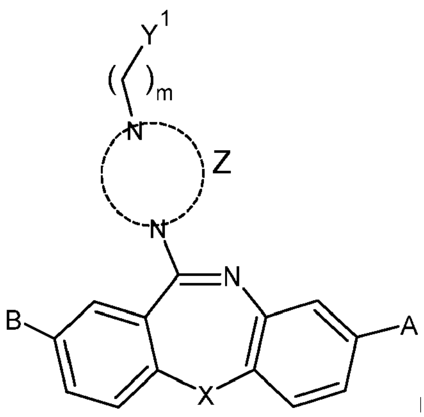

respectively.

The terms "of the [present] invention", "in accordance with the invention", "according to the invention" and the like, as used herein are intended to refer to all aspects and embodiments of the invention described and/or claimed herein.

As used herein, the term "comprising" is to be construed as encompassing both "including" and "consisting of",

both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated.

It is to be understood that application of the teachings of the present invention to a specific problem or environment, and the inclusion of variations of the present invention or additional features thereto (such as further aspects and embodiments), will be within the capabilities of one having ordinary skill in the art in light of the teachings contained herein.

Unless context dictates otherwise, the descriptions and definitions of the features set out above are not limited to any particular aspect or embodiment of the invention and apply equally to all aspects and embodiments which are described.

All references, patents, and publications cited herein are hereby incorporated by reference in their entirety.

BRIEF DESCRIPTION OF THE FIGURES AND SEQUENCES

The figures show:

**Figure 1: Expression of Kcnt1 and critical itch receptors across sensory neuron subsets from published single-cell RNA-seq data.** (**A**) Expression pattern in mouse DRG neurons (6-8 week old)[4]. (**B**) Expression pattern in human DRG neurons (24-65 year old)[16]. Downloaded from: https://sensoryomics.shinyapps.io/RNA-Data/. (**C**) Expression pattern in non-human primate DRG neurons (5-14 year old)[17]. Downloaded from: https://ernfors-group.shinyapps.io/macaquedr.

**Figure 2: Establishing a modified version of the FluxOR assay.** Cultured HEK293 cells stably expressing human Slack (HEK-Slack cells) were incubated with compounds of the invention in different buffers. (**A**) In the buffer provided with the FluxOR assay kit, Slack activation (indicated as increased F/F(Baseline) ratio) was detected after incubation with both Loxapine and vehicle (FluxOR assay buffer containing 0.03% DMSO). As Slack is activated by $Na^+$, the vehicle-induced Slack activation was most likely mediated by $Na^+$ present in the FluxOR assay buffer and in the vehicle. (**B**) In a $Na^+$-free buffer (with replacement of NaCl by choline chloride), Slack activation was observed after incubation with 30 μM Loxapine and 140 mM NaCl, but not after incubation with vehicle ($Na^+$ free buffer with 2 mM $Ca^{2+}$, 2 mM $Mg^{2+}$ and 0.03% DMSO). (C) DMSO at a concentration of 0.03-3% did not activate Slack in a $Na^+$-free buffer with 2 mM $Ca^{2+}$ and 2 mM $Mg^{2+}$. (**D**) Dose-response experiments with Loxapine in a $Na^+$ free buffer with 2 mM $Ca^{2+}$, 2 mM $Mg^{2+}$ and 0.03% DMSO yielded an $EC_{50}$ value of 23.45 μM in the first preliminary experiments. (**E**) Dose-response experiments with the Slack inhibitor compound 31 (ref[18]) in a $Na^+$ free buffer with 2 mM $Ca^{2+}$, 2 mM $Mg^{2+}$ and 0.03% DMSO and pre-stimulation with 25 μM Loxapine revealed that compound 31 inhibited the F/F(Baseline) ratio in a dose-dependent manner ($IC_{50}$ = 2.1 μM), confirming that the readout depends on Slack. All further experiments with the FluxOR, which are presented in **Fig. 3A, B**, were performed using a $Na^+$ free buffer with 2 mM $Ca^{2+}$, 2 mM $Mg^{2+}$ and 0.03% DMSO. All conditions were measured at least in triplicate and data are shown as mean ± SD.

**Figure 3: New compounds are activators of Slack channels.** (**A**) and (**B**) Dose-response experiments with new compounds in a FluxOR potassium ion channel assay. HEK293 cells stably expressing human Slack (HEK-Slack cells) were assayed for a potassium channel-mediated thallium response using F!uxOR. Each compound was incubated at six concentrations. For each sample, the fluorescence value was calculated and then normalized to the maximum fluorescence value of Loxapine. For better comparability, data from the Loxapine measurements are presented in all five graphs. Each data point is the average of 3 replicates. Data represent the mean ± s.d. (**C**) Patch-clamp recordings confirm that new compounds evoke Slack-mediated potassium currents. Whole-cell voltage recordings on HEK-Slack cells were performed at baseline and after incubation with a new compound (50 μM),

Loxapine (lox; 50 μM) or vehicle (external solution containing 0.03% DMSO). Loxapine was incubated in each series of experiments as a positive control. Shown are current densities (pA/pF) of vehicle, Loxapine and new compounds relative to baseline at a voltage of +80 mV (fold increase). Corresponding Current-voltage curves from these recordings and representative outward potassium traces are presented in **Fig. 4.** ** $P < 0.01$, *** $P < 0.001$ vs vehicle (Kruskal-Wallis test with Dunn's correction); box-and-whisker plots represent maximum and minimum values, and the box shows the first, second (median) and third quartile values. (**D**) Correlation of relative EMAX values of new compounds obtained in the FluxOR assay (presented in (**A**) and (**B**)) with relative current densities obtained in the patch-clamp experiments (presented in (**C**)). Pearson correlation. (**E**) Time-dependent fluorescence/fluorescence$_{Baseline}$ ratios of the Dose-response experiments presented in (**B**) in comparison with Loxapine and vehicle.

**Figure 4: New compounds evoke Slack-mediated potassium currents.** Whole-cell voltage recordings on HEK-Slack cells were performed at baseline and after incubation with a new compound (50 μM), Loxapine (50 μM) or vehicle (external solution containing 0.03% DMSO). (**A**) Current-voltage (I-V) curves from patch-clamp experiments that are presented in **FIG. 3C.** Loxapine was incubated in each series of experiments as a positive control. *n* = 8-20 cells per group. Data are shown as mean ± SD. (**B**) Representative outward $K^+$ ($I_K$) traces of Loxapine at +80 mV. (**C**) Current-voltage (I-V) curves from patch-clamp experiments that are presented in **FIG. 3B.** Loxapine was incubated in each series of experiments as a positive control. *n* = 8-20 cells per group.

**Figure 5: *In vitro* and pharmacokinetic characterization of the new compounds.** Blood brain barrier (BBB) permeability of the compounds of the invention was estimated using a BBB-specific parallel artificial membrane permeability assay. The predicted extent of BBB permeability is reflected by log $P_e$ values, and the fraction of solute lost to the membrane in this assay is reflected by the membrane retention factor (MR). Note that a high MR value limits the predictive validity of a corresponding log $P_e$ value.

**Figure 6: *In vivo* Pharmacokinetic profiles of new compounds.** (**A**) Time courses of brain and plasma concentrations of the compounds 1 to 10 in mice. Animals were i.p. injected with 1 mg/kg of each compound in a cassette dosing procedure (with simultaneous administration of 3-4 compounds per animal) and plasma and brain levels were measured at different time points by LC-MS analysis. Note that the y-axes are scaled differently in all diagrams. Data are presented as mean ± s.e.m. of 3 mice per group. (**B**) *In vivo* brain/plasma ratio of the new compounds in mice. Note that Compound 6 and Compound 10 have a particularly low brain/plasma ratio, suggesting a limited brain penetration. Data represent the mean. Additional pharmacokinetic parameters are presented in Table 6666Table 66.

**Figure 7: *In vitro* pharmacology screening of Compound 6, Compound 10 and Loxapine.** Compounds were tested at a concentration of 10 μM in binding and enzyme and uptake assays of 44 targets (mostly human; except BZD, NMDA, MAO-A, $Ca^{2+}$ channel, Kv channel and $Na^+$ channel, which were from rat). Compound binding was calculated as a % inhibition of the binding of a radioactively labeled ligand (agonist or antagonist, as indicated in brackets) specific for each target. Compound enzyme inhibition effect was calculated as a % inhibition of control enzyme activity. Results showing an inhibition (or stimulation for assays run in basal conditions) higher than 50% are considered to represent significant effects of the test compounds and are presented in black. Results showing an inhibition or stimulation between 25% and 50% (indicative of weak to moderate effects) and those lower than 25% (considered mostly attributable to variability of the signal around the control level) are presented in grey. Measurements were performed in duplicate.

**Figure 8: Compound 6 and Compound 10 inhibit histamine-independent acute itch behavior and neuropathic pain behavior.** (**A**), (**B**), Motor function. Compound 6, Compound 10, Loxapine or vehicle (0.9% NaCl with 10% 2-hydroxypropyl-β-cyclodextrine) were i.p. administered and (**A**) an accelerating rotarod test followed by (**B**) a vertical pole test were performed 15 min thereafter. Note that Compound 6 and 10 did not affect the time spent on the rotarod or the vertical pole, and therefore did not inhibit motor coordination, whereas Loxapine dose-dependently inhibited the motor coordination in both models. n = 8 per group. Box-and-whisker plots represent maximum and minimum values, and the box shows the first, second (median) and third quartile values. Dotted lines indicate cutoff times. * $p < 0.05$, *** $p < 0.001$, Kruskal-Wallis test. (C)-(G), Acute itch behavior. Compound 6, Compound 10 or vehicle were i.p. administered and 15 min thereafter different pruritogens were s.c. administered into the nape of the neck and the number of scratching bouts was counted over 30 min. In each panel, the time course of scratching behavior is shown on the left and the sum of scratching bouts in 30 min is presented on the right. (**C**), Compound 6 and Compound 10 inhibited the scratching behavior induced by chloroquine in a dose-dependent manner. (**D**) Compound 6 inhibited the chloroquine-induced scratching behavior in WT mice but not in Slack$^{-/-}$ littermates. (**E**)

Compound 6 ameliorated the scratching behavior induced by SLIGRL, (**F**) but not by histamine. n = 7-8 per group. Data represent the mean ± s.e.m. * $p < 0.05$, ** $p < 0.01$, *** $p < 0.001$; one-way-ANOVA with Dunnett's correction (C), Kruskal-Wallis test with Dunn's correction (D) or unpaired t-test (E, F). (**G**) Neuropathic pain behavior. In the spared nerve injury (SNI) model, neuropathic pain was induced by surgery. Twenty-eight days thereafter, mechanical hypersensitivity of the affected hindpaw (determined using a Dynamic Plantar Aesthesiometer), which is indicated by decreased paw withdrawal latency time, was detected in all mice. Then Compound 10 or vehicle were i.p. administered and the mechanical sensitivity was assessed over 3 h. Note that Compound 10 inhibited the neuropathic pain behavior. (**H**) Effects of Compound 6 in WT mice pretreated with a Slack inhibitor. Compound 31 (30 mg/kg) or vehicle were i.p. administered 5 min prior to Compound 6 (30 mg/kg i.p.), and chloroquine was s.c. administered 15 min thereafter. Data indicate that the antipruritic effect of Compound 6 was partly antagonized by the Slack inhibitor. n = 7-8 per group. Data are shown as mean ± SEM. * $p < 0.05$, unpaired t-test.

**Figure 9: Effects of Compound 6 in models of motor function and acute itch do not show sex-related differences.** Breakdown of results for Compound 6 in male and female mice from (**A**) **Fig. 8A,** (**B**) **Fig. 8B,** (**C**) **Fig. 8C,** and (**D**) **Fig. 8F.** Statistical significance in a and b was assessed by Kruskal-Wallis test. Box-and-whisker plots represent maximum and minimum values, and the box shows the first, second (median) and third quartile values. Dotted lines indicate cutoff times. Statistical significance in c and f was assessed by one-way-ANOVA with Dunnett's correction. Data represent the mean ± s.e.m.

**Figure 10: Compound 6 inhibits persistent itch behavior.** (**A**)-(**C**) Efficacy of Compound 6 in the DNFB model of persistent itch. **(A)** Experimental diagram showing the induction of spontaneous itch behavior by topical application of 2,4-dinitrofluorobenzene (DNFB) to the nape of the neck (twice 14 days apart), i.p. drug delivery 105 min after the second DNFB application, and begin of videotaping 15 min thereafter. (**B**) Compound 6 significantly reduced the number of scratching bouts and (**C**) the number of head shakes as compared to vehicle, n = 6-7 per group. (**D**)-(**F**) Efficacy of Compound 6 in the MC903 model of persistent itch. (**D**) Experimental diagram showing the induction of spontaneous scratching by topical application of MC903 to the nape of the neck (once daily over 7 days), i.p. drug delivery at day 8, and begin of videotaping 15 min thereafter. In comparison to vehicle, Compound 6 (**E**) significantly reduced the number of scratching bouts and (**F**) the number of head shakes. n = 8 per group. Data represent the mean ± s.e.m. * $p < 0.05$, ** $p < 0.01$, unpaired t-test.

**Figure 11: Sex-related effects of Compound 6 and correlation of behavioral outcomes in chronic itch models.** (**A**), (**B**) Effects of Compound 6 in models of chronic itch do not show sex-related differences. Breakdown of results in male and female mice from **(A) Fig. 10B and 10C** (DNFB model) and **(B) Fig. 10E and 10F** (MC903 model). Data represent the mean ± s.e.m. (**C**), (**D**), Correlation of number of head shakes with number of scratching bouts from **(C) Fig. 10B and 10C**, (D) **Fig. 10E and 10F** and (**E**) combination thereof. Statistical significance was assessed by a Pearson correlation.

**Figure 12: Potential side effects of Compound 6.** Pulse oximetry on non-anesthetized mice using a MouseOX Plus device revealed that i.p. delivery of Compound 6 or vehicle did not alter (**A**) the heart rate or (**B**) the breath rate, whereas morphine significantly lowered both parameters (n = 6 per group). * $p < 0.05$, ** $p < 0.01$, *** $p < 0.001$ vs vehicle, two-way MC ANOVA and Dunnett test.

**Figure 13: Compound 6 reduces neuronal excitability of itch-sensitive sensory neurons.** Cultured DRG neurons from mice were incubated overnight with an inflammatory soup followed by whole cell current-clamp recordings on IB4-binding neurons. (**A**) Recordings from a DRG neuron showing action potential (AP) firing in response to current injections (200-950 pA at 150 pA intervals, 1000-ms duration) at baseline, in the presence of Compound 6 (50 μM), and after wash-out. (**B**) Group data show that Compound 6 significantly blocked AP firing compared to baseline; n = 6 neurons. (**C**) Recordings from a DRG neuron showing a single AP evoked by injections of small currents (0-220 pA at 20 pA intervals, 10-ms duration) at baseline and in the presence of Compound 6 (50 μM). (**D**), (**E**) Group data indicate that Compound 6 (**D**) significantly increased the rheobase (amount of current required to generate an AP; and (**E**) reduced the AP amplitude; n = 7 neurons. Data represent the mean ± s.e.m. * $p < 0.05$, ** $p < 0.01$, *** $p < 0.001$, paired t-test.

EXAMPLES

Certain aspects and embodiments of the invention will now be illustrated by way of example and with reference to the description, figures and tables set out herein. Such examples of the methods, uses and other aspects of the present invention are representative only, and should not be taken to limit the scope of the present invention to only

such representative examples.

The examples show:

**Example 1: Synthesis of Slack-activating compounds**

A series of new Slack-activating compounds were developed based on the antipsychotic drug Loxapine. Synthesis of these new compounds was performed by linear synthesis Routes A - C under argon atmosphere as described below.

Synthesis Route A: The Loxapine scaffold contains a tricyclic core and a piperazine ring substituted by an alkyl residue. Starting from methyl esters of salicylic acid and 2-fluoronitrobenzene derivatives, diarylethers were obtained by nucleophilic aromatic substitution reaction. Subsequent reduction of the nitro group by tin(II)chloride enabled intramolecular amide bond formation.

Synthesis Route A

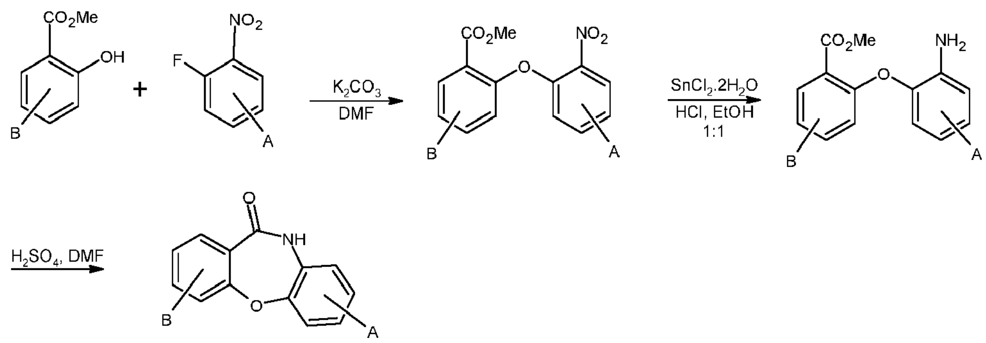


Synthesis Route B: Alternatively, amide bond was first formed between 2-fluorobenzoic acids and 2-aminophenol derivatives, followed by intramolecular nucleophilic aromatic substitution to afford the same lactam core.

Synthesis Route B

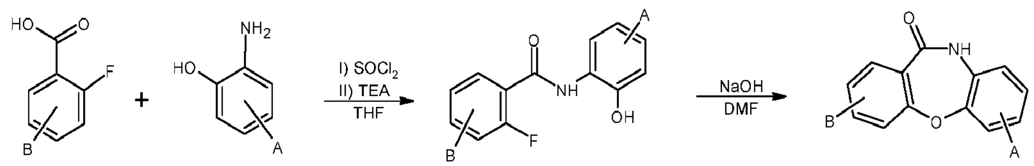


Synthesis Route C: The lactam was then converted to an imidoyl chloride by $POCl_3$. In the last step, substituted piperazines displaced the chloride to afford the desired Loxapine derivatives.

Synthesis Route C

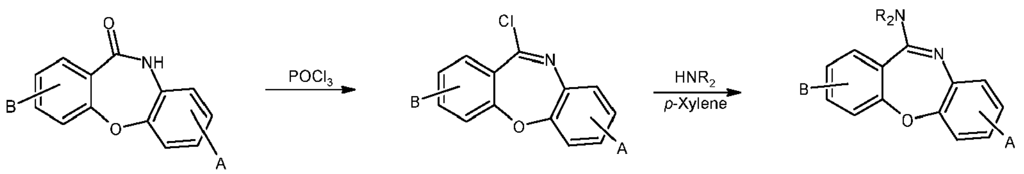

**Synthesis Route A - General synthesis of lactam starting from methyl esters of salicylic acid and 2-fluoronitrobenzene derivatives.**

GP1 - Formation of the diarylether: Potassium carbonate (1.5 equiv) was added to a solution of the corresponding methyl salicylate (1.5 equiv) and the corresponding 2-fluoronitrobenzene (1.0 equiv) in DMF (1.5 M regarding 2-fluoronitrobenzene). The resulting solution was heated in an oil bath to 120 °C overnight. Solvents were evaporated under reduced pressure and the residue taken into water and extracted three times with ethyl acetate. The combined organic phases were dried over magnesium sulfate, filtered, and evaporated. The resulting crude product was purified by flash chromatography.

GP2 - Reduction of the nitro group: A solution of $SnCl_2 \cdot 2H_2O$ (4.0 equiv) in conc. HCl (3 M) was added to a solution of the corresponding methyl 2-(2-nitrophenoxy)benzoate derivative (1.0 equiv) in a mixture of ethanol/conc. HCl 1:1 (0.5 M). The resulting solution was stirred at rt overnight. After that time, the temperature was set to 0 °C and the pH of the reaction solution made slightly basic by addition of sodium carbonate. The resulting solution was then extracted three times with ethyl acetate. The combined organic phases were dried over magnesium sulfate, filtered, and evaporated. The resulting crude product was purified by flash chromatography.

GP3 - Formation of the lactam ring via intramolecular condensation:

GP3I: A solution of the corresponding methyl 2-(2-aminophenoxy) benzoate derivative (1.0 equiv) in DMF (0.2 M) was treated with concentrated sulfuric acid (1.3 equiv) and heated in an oil bath at 120 °C overnight. After that time, the reaction was cooled to 0 °C and a few milliliters of water were added. The precipitated product was then filtered off and dried under vacuum to obtain the crude product, which was used in the next step without further purification.

**Synthesis Route B - General synthesis of lactam starting from 2-fluorobenzoic acids and 2-aminophenol derivatives.**

GP4 - Formation of the amide: To a solution of the corresponding 2-fluorobenzoic acid (1.0 equiv) in THF (1 M) freshly distilled thionyl chloride (2.0 equiv) was added and heated to reflux in an oil bath for 2 h. Thereafter, excess thionyl chloride and THF were removed under reduced pressure and the residue obtained was taken up in THF (2.5 M) again. This solution was added dropwise to a solution of the corresponding 2-aminophenol derivative (1.0 eq) and triethylamine (2 equiv) in THF (2.5 M referred to 2-aminophenol derivative) at 0 °C and the reaction mixture was stirred overnight at rt. After that time, the reaction mixture was concentrated under reduced pressure and the residue taken up in ethyl acetate. The organic phase was first washed with an aq HCl solution (2 M), water and saturated aq NaCl solution, then dried over magnesium sulfate, filtered, and evaporated. The resulting crude product was purified by flash chromatography.

GP5 - Formation of the lactam ring via intramolecular nucleophilic aromatic substitution: A solution of the corresponding 2-fluoro-N-(2-hydroxyphenyl)benzamide derivative (1.0 equiv) in DMF (0.25 M) was treated with freshly powdered NaOH (1.0 equiv) and heated in an oil bath at 150 °C for 5 h. After that time, the reaction was cooled to 0 °C and a few milliliters of water were added. The precipitated product was then filtered off and dried under vacuum to obtain the crude product, which was used in the next step without further purification.

**Synthesis Route C - General synthesis of Loxapine derivatives starting from lactam**

GP6 - Reaction with phosphorus oxychloride: The corresponding lactam (1.0 equiv) was dissolved in freshly distilled phosphorus oxychloride (0.5 M) and *N,N*-dimethylaniline (0.6 equiv) was added. The resulting mixture was heated to reflux in an oil bath for 5 h. Thereafter, excess phosphorus oxychloride was removed under reduced pressure and the residue obtained was taken up in toluene and washed once with cold water. The organic phase was dried over magnesium sulfate, filtered, and evaporated to give the crude product, which was immediately used in the next step without further purification.

GP7 - Formation of Loxapine derivatives: The corresponding amine (2.0 eq) was added to a solution of the appropriate imidoyl chloride (1.0 equiv) in p-xylene (0.15 M regarding imidoyl chloride). The resulting mixture was heated in an oil bath at 140 °C for 5 h. Solvents were evaporated, and the crude substance purified by preparative HPLC or flash chromatography. If unsubstituted piperazine or homopiperazine was employed, then alkylation of these derivatives was obtained by GP8.

GP8 - Alkylation of Loxapine derivatives: A mixture of Loxapine derivative (1 equiv), corresponding alkyl chloride (2 equiv.) and triethylamine (10 equiv.) in acetonitrile (0.15 M) was heated to reflux in an oil bath for 16 h. After completion, the reaction mixture was evaporated, and the residue purified by preparative HPLC or flash chromatography.

**Example 2: Characterization of synthesized compounds**

Using the Synthesis Routes as described above, the compounds were synthesized. For characterization of these compounds, NMR spectra were recorded with BrukerAvance DPX250, Bruker Avance 300, Bruker Avance 400, or Bruker Avance 500 all from Bruker (Karlsruhe, Germany) operating at ambient temperature. Proton spectra were recorded in $CDCl_3$ or DMSO-$d_6$ and $^1H$ NMR chemical shifts were referenced to the residual signals of $CHCl_3$ (at $\delta$ = 7.26 ppm) and DMSO-ds (at $\delta$ = 2.50 ppm). $^{13}C$ NMR chemical shifts were referenced against the central line of the solvent signal (for $CDCl_3$ at $\delta$ = 77.16 ppm and for DMSO-$d_6$ at $\delta$ = 39.52 ppm). Chemical shifts are given on $\delta$ scale (ppm). Coupling constants (J) are given in Hz. Multiplicities are indicated as follows: br (broad signal), s (singlet), d (doublet), t (triplet), q (quartet), quint or m (multiplet). ESI-MS were measured with LCMS-2020 from Shimadzu and HRMS with MALDI Orbitrap XL from Thermo Scientific. TLC was carried out on silica gel plates from Marcherey-Nagel (ALUGRAM®) and visualized with an UV lamp (254 nm and/or 366 nm). Purification of products was performed by flash chromatography using puriFlash XS420 and Silica HP 30 $\mu$m columns as stationary phase all from Interchim (Montluçon, France). Analytical and semipreparative HPLC was conducted by Shimadzu prominence with a SPD20A UV/Vis detector both from Shimadzu (Duisburg, Germany). Stationary phases were Luna 10 $\mu$m 100 Å, C18(2) (250 x 4.6 mm) and Luna 10 $\mu$m 100 Å, C18(2) (250 x 21.20 mm) from Phenomenex (Aschaffenburg, Germany) and eluent was a mixture of ACN and aq formic acid solution (0.1%). Flow rate was set to 1 mL/min and 21 mL/min. All compounds tested display a purity of >95% (254 nm).

**11-(4-Methylpiperazin-1-yl)-2-(trifluoromethyl)dibenzo[*b*,*f*][1,4]oxazepine (Compound 2)**

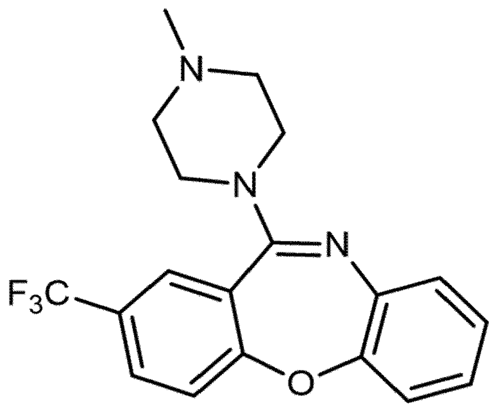

Synthesized according to GP7 from 11-chloro-2-(trifluoromethyl)dibenzo[*b*,*f*][1,4]oxazepine (63 mg, 0.210 mmol) and 1-methylpiperazine (46.6 $\mu$L, 0.420 mmol). Purification of the crude product by preparative HPLC yielded the title compound as a brown oil and formate salt (22 mg, 26%). 1H NMR (300 MHz, $CDCl_3$) $\delta$ 8.35 (s, 1H), 7.95 (br s, 1H), 7.70 (ddd, J = 8.5, 2.3, 0.5 Hz, 1H), 7.62 (d, J = 2.3 Hz, 1H), 7.36 (d, J = 8.5 Hz, 1H), 7.18-7.07 (m, 3H), 7.01 (td, J = 7.4, 2.0 Hz, 1H), 3.62 (br s, 4H), 2.69 (br s, 4H), 2.44 (s, 3H); 13C[1H] NMR (75 MHz, $CDCl_3$) $\delta$ 166.6, 163.2, 158.7, 151.4, 139.9, 129.7, 127.5, 127.2, 127.0, 126.0, 124.9, 124.1, 123.5, 122.1, 120.3, 54.0, 46.7, 45.3; HPLC-purity (254 nm): 96%; MALDI-HRMS: m/z calculated for $C_{19}H_{19}F_3N_3O$[M+H]+: 362.1474, found: 362.1488.

**11-(4-Methyl-1,4-diazepan-1-yl)-2-(trifluoromethyl)dibenzo[*b*,*f*][1,4]oxazepine (Compound 3)**

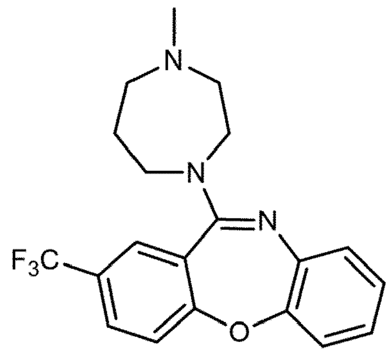

Synthesized according to GP7 from 11-chloro-2-(trifluoromethyl)dibenzo[*b*,*f*][1,4]oxazepine (63 mg, 0.212 mmol) and 1-methylhomopiperazine (49 mg, 0.423 mmol). Purification of the crude product by preparative HPLC yielded the title compound as a brown oil and formate salt (73 mg, 92%). 1H NMR (300 MHz, $CDCl_3$) δ 9.97 (br s, 1H), 8.51 (s, 1H), 7.68 (dd, J = 8.5, 1.9 Hz, 1H), 7.58 (d, J = 2.1 Hz, 1H), 7.35 (d, J = 8.5 Hz, 1H), 7.12-7.06 (m, 3H), 6.98-6.95 (m, 1H), 4.06-3.45 (m, 4H), 3.08-2.84 (m, 4H), 2.56 (s, 3H), 2.29 (br s, 1H), 2.01 (br s, 1H); 13C[1H] NMR (75 MHz, $CDCl_3$) δ 168.2, 162,8, 158.3, 151.2, 140.4, 129.4, 127,4, 127.0, 126.9, 126.2, 124,2, 124.0, 123.6, 122,2, 120,2, 56.9, 56.3, 49.1, 46.9, 45.4, 26.3; HPLC-purity (254 nm): 97%; MALDI-HRMS: m/z calculated for $C_{20}H_{21}F_3N_3O$[M+H]+: 376.1631, found: 376.1639.

**8-Fluoro-11-(4-methylpiperazin-1-yl)-2-(trifluoromethyl)dibenzo[*b*,*f*][1,4]oxazepine (Compound 4)**

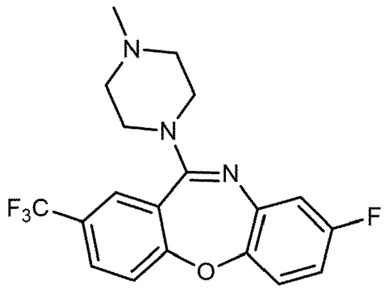

Synthesized according to GP7 from 11-chloro-8-fluoro-2-(trifluoromethyl)dibenzo[*b,f*][1,4]oxazepine (50 mg, 0.158 mmol) and 1-methylpiperazine (32 mg, 0.317 mmol). Purification of the crude product by preparative HPLC yielded the title compound as a yellow solid (25 mg, 42%). 1H NMR (300 MHz, $CDCl_3$) δ 7.68 (dd, J = 8.5, 1.9 Hz, 1H), 7.58 (d, J = 2.1 Hz, 1H), 7.35 (d, J = 8.5 Hz, 1H), 7.12-7.06 (m, 3H), 6.98-6.95 (m, 1H), 4.06-3.45 (m, 4H), 3.08-2.84 (m, 4H), 2.56 (s, 3H), 2.29 (br s, 1H), 2.01 (br s, 1H); 13C[1H] NMR (75 MHz, $CDCl_3$) δ 163.2, 161.5, 159.6, 159.4, 147.7, 141.6, 128.4, 127.7, 123.7, 124.1, 122.2, 120.1, 113.4, 110.9, 54.7, 47.3, 46.04; HPLC-purity (254 nm): >99%; MALDI-HRMS: m/z calculated for C19H18F4N30[M+H]+: 380.1381, found: 380.1377.

**2-(4-(2-Chlorodibenzo[*b,f*][1,4]oxazepin-11-yl)piperazin-1-yl)ethan-1-ol (Compound 5)**

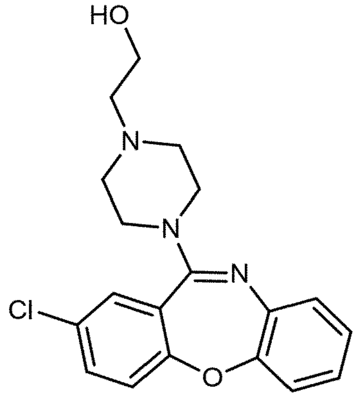

Synthesized according to GP7 from 2,11-dichlorodibenzo[b,t][1,4]oxazepane (198 mg, 0.750 mmol) and hydroxyethylpiperazine (195 mg, 1.50 mmol). Purification of the crude product by preparative HPLC yielded the title compound as a brown oil and formate salt (216 mg, 72%). 1H NMR (300 MHz, $CDCl_3$) δ 8.40 (s, 1H), 7.41 (dd, J = 8.6, 2.6 Hz, 1H), 7.31 (d, J = 2.6 Hz, 1H), 7.19 (d, J = 8.6 Hz, 1H), 7.16-7.07 (m, 3H), 7.05-6.99 (m, 1H), 6.51 (br s, 2H), 3.85-3.81 (m, 2H), 3.74 (br s, 4H), 2.98 (br s, 4H), 2.89-2.85 (m, 2H); 13C[1H] NMR (75 MHz, $CDCl_3$) δ 166.8, 159.3, 158.3, 151.7, 139.6, 133.0, 130.5, 128.8, 127.1, 125.9, 125.1, 124.5, 122.9, 120.2, 59.7, 56.9, 52.2, 45.8; HPLC-purity (254 nm): 99%; MALDI-HRMS: m/z calculated for $C_{19}H_{21}ClN_3O_2$[M+H]+: 358.1317, found: 358.1324.

**2-(2-(4-(2-Chlorodibenzo[*b,f*][1,4]oxazepin-11-yl)piperazin-1-yl)ethoxy)acetic acid (Compound 6)**

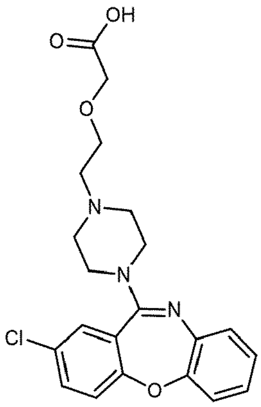

Synthesized according to GP8 from commercially purchased amoxapine (75 mg, 0.239 mmol), 2-(2-chloroethoxy)acetic acid (66 mg, 0.478 mmol) and triethylamine (0.34 mL, 2.39 mmol). Purification of the crude product by preparative HPLC yielded the title compound as a brown oil and formate salt (47 mg, 42%). 1H NMR (300 MHz, $CDCl_3$) δ 8.24 (br s, 1H), 7.41 (dd, J = 8.7, 2.4 Hz, 1H), 7.34 (d, J = 2.4 Hz, 1H), 7.18 (d, J = 8.7 Hz, 1H), 7.15-6.98 (m, 4H), 5.91 (br s, 2H), 4.23 (br s, 2H), 4.44-3.94 (m, 6H), 3.87-3.72 (m, 6H); 13C[1H] NMR (75 MHz, $CDCl_3$) δ 168.8, 164.7, 159.3, 159.0, 151.6, 139.1, 133.4, 130.8, 128.7, 127.2, 126.0, 125.7, 123.9, 123.0, 120.3, 61.4, 59.5, 55.8, 41.4; HPLC-purity (254 nm): 99%; MALDI-HRMS: m/z calculated for $C_{21}H_{23}ClN_3O_4$ [M+H]+: 416.1372, found: 416.1375.

**2-(2-(4-(2-Chlorodibenzo[*b,f*][1,4]thiazepin-11-yl)piperazin-1-yl)ethoxy)ethan-1-ol (Compound 7)**

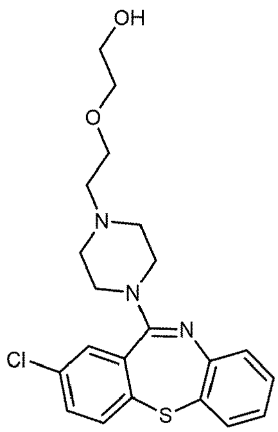

Synthesized according to GP7 from 2,11-dichlorodibenzo[b,f][1,4]thiazepine (119 mg, 0.425 mmol) and 1-[2-(2-hydroxyethoxy)ethyl]piperazine (147 μL, 0.850 mmol). Purification of the crude product by preparative HPLC yielded the title compound as a brown oil and formate salt (156 mg, 79%). 1H NMR (300 MHz, $CDCl_3$) δ 8.37 (s, 1H), 7.46-7.41 (m, 1H), 7.38 (dd, J = 7.7, 1.5 Hz, 1H), 7.33-7.27 (m, 2H), 7.29 (br s, 1H), 7.23-7.17 (m, 1H), 7.06 (dd, J = 8.0, 1.5 Hz, 1H), 6.93 (td, J = 7.3, 1.5 Hz, 1H), 3.88 (br s, 2H), 3.75 (t, J = 5.1 Hz, 2H), 3.70 (t, J = 4.8 Hz, 2H), 3.58 (t, J = 4.8 Hz, 2H), 3.55 (br s, 2H), 3.08-3.00 (m, 2H), 2.90 (t, J = 5.3 Hz, 2H), 2.90-2.82 (br s, 2H); 13C[1H] NMR (75 MHz, $CDCl_3$) δ 166.7, 158.9, 148.2, 138.1, 134.9, 134.8, 133.4, 132.9, 131.2, 129.4, 128.6, 127.3, 125.3, 123.6, 72.6, 66.1, 61.4, 57.2, 52.0, 45.1; HPLC-purity (254 nm): 95%; MALDI-HRMS: m/z calculated for $C_{21}H_{25}ClN_3O_2S$[M+H]+: 418.1351, found: 418.1350.

**2-(2-(4-(2-(Trifluoromethyl)dibenzo[*b,f*][1,4]oxazepin-11-yl)piperazin-1-yl)ethoxy)acetic acid (Compound 8)**

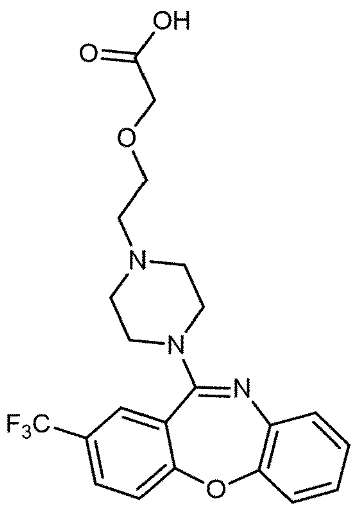

Synthesized according to GP8 from 11-(piperazin-1-yl)-2-(trifluoromethyl)dibenzo[*b,f*][1,4]oxazepine (75 mg, 0.102 mmol), 2-(2-chloroethoxy)acetic acid (28 mg, 0.205 mmol) and triethylamine (0.14 mL, 1.02 mmol). Purification of the crude product by flash chromatography (DCM/MeOH 95:5 to 9:1) yielded the title compound as a colorless solid (28 mg, 61%). 1H NMR (400 MHz, $CDCl_3$) δ 7.66 (dd, J = 8.5, 2.0 Hz, 1H), 7.56-7.56 (m, 1H), 7.31 (d, J = 8.5 Hz, 1H), 7.12-7.03 (m, 3H), 6.97 (td, J = 7.6, 1.8 Hz, 1H), 4.21 (s, 2H), 3.73-3.43 (m, 12H); 13C[1H] NMR (101 MHz, $CDCl_3$) δ 168.7, 163.4, 158.8, 151.5, 139.7, 128.5, 127.8, 127.4, 126.3, 125.4, 124.1, 123.6, 122.5, 120.5, 73.8, 69.7, 61.8, 44.3, 41.7; HPLC-purity (254 nm): 98%; MALDI-HRMS: m/z calculated for $C_{22}H_{23}F_3N_3O_4$ [M+H]+: 450.1635, found: 450.1637.

**2-(2-(4-(2-(Trifluoromethyl)dibenzo[*b,f*][1,4]oxazepin-11-yl)-1,4-diazepan-1-yl)ethoxy)acetic acid (Compound 9)**

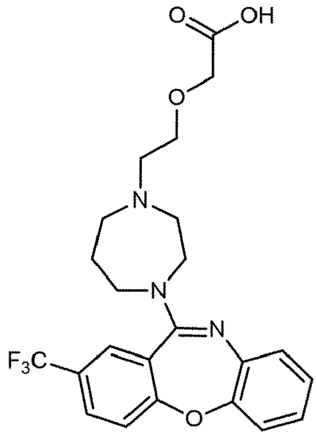

Synthesized according to GP8 from 11-(4-methyl-1,4-diazepan-1-yl)-2-(trifluoromethyl)dibenzo[*b*,*f*][1,4]oxazepine (40 mg, 0.111 mmol), 2-(2-chloroethoxy)acetic acid (31 mg, 0.221 mmol) and triethylamine (0.16 mL, 1.11 mmol). Purification of the crude product by flash chromatography (DCM/MeOH 95:5 to 9:1) yielded the title compound as a colorless solid (20 mg, 39%). 1H NMR (400 MHz, $CDCl_3$) $\delta$ 7.95-7.91 (m, 1H), 7.80-7.77 (m, 1H), 7.59 (t, J = 8.8 Hz, 1H), 7.22-7.18 (m, 1H), 7.11-6.93 (m, 3H), 4.63 (d, J = 4.8 Hz, 1H), 4.18-4.09 (m, 3H), 3.77-3.36 (m, 10H), 2.08-1.46 (m, 2H); 13C[1H] NMR (101 MHz, $CDCl_3$) $\delta$ 168.6, 162.4, 157.6, 150.7, 140.3, 129.9, 126.6, 126.3, 126.0, 126.0, 123.7, 123.6, 123.7, 122.4, 120.2, 72.5, 69.1, 68.9, 68.2, 67.6, 63.2, 60.0, 44,0; HPLC-purity (254 nm): 95%; MALDI-HRMS: m/z calculated for $C_{23}H_{25}F_3N_3O_4$ [M+H]+: 464.1792, found: 464.1788.

**5-((4-(2-Chlorodibenzo[*b*,*f*][1,4]oxazepin-11-yl)piperazin-1-yl)methyl)-1,2-dihydro-3H-1,2,4-triazol-3-one (Compound 10)**

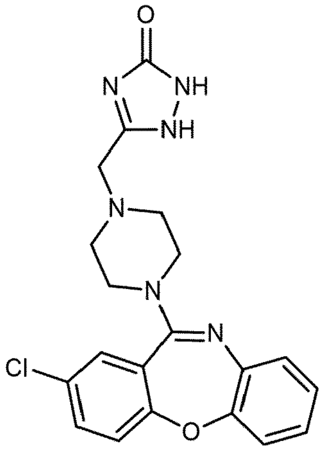

Synthesized according to GP8 from commercially purchased amoxapine (108 mg, 0.334 mmol), 3-(chloromethyl)-1H-1,2,4-triazol-5(4H)-one (66 mg, 0.478 mmol) and triethylamine (0.47 mL, 3.34 mmol). Purification of the crude product by flash chromatography (EtOAc/MeOH 9:1) yielded the title compound as a colorless solid (136 mg, 99%). 1H NMR (400 MHz, DMSO-$d_6$) $\delta$ 11.36 (br s, 1H), 11.26 (br s, 1H), 7.61 (dd, J = 8.7, 2.6 Hz, 1H), 7.42-7.39 (m, 2H), 7.18 (dd, J = 7.9, 1.2 Hz, 1H), 7.11-7.04 (m, 3H), 3.47 (br s, 4H), 3.36 (br s, 2H), 2.54 (br s, 4H); 13C[1H] NMR (101 MHz, $CDCl_3$) $\delta$ 158.7, 158.0, 156.2, 151.2, 144.3, 139.9, 133.0, 129.5, 128.7, 126.5, 125.8, 124.4, 124.2, 123.1, 120.2, 53.2, 52.0, 48.6; HPLC-purity (254 nm): 99%; MALDI-HRMS: m/z calculated for $C_{20}H_{20}ClN_6O_2$ [M+H]+: 411.1331, found: 411.1327.

**5-((4-(2-(Trifluoromethyl)dibenzo[*b*,*f*][1,4]oxazepin-11-yl)piperazin-1-yl)methyl)-1,2-dihydro-3H-1,2,4-triazol-3-one (Compound 11)**

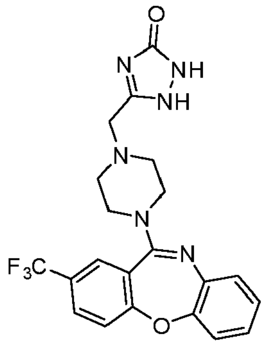

Synthesized according to GP8 from Compound 13 (20.0 mg, 0.0576 mmol), 3-chloromethyl)-1*H*-1,2,4-triazol-5(4*H*)-one (15.9 mg, 0.115 mmol), and triethylamine (80.3 μL, 0.576 mmol). Purification of the crude product by flash chromatography (DCM/MeOH 95:5 to 8:2) yielded the title compound (24.0 mg, 94%). $^1$H NMR (500 MHz, DMSO-$d_6$) δ 11.37 (s, 1H), 11.26 (s, 1H), 7.95 (dd, J = 8.7, 2.1 Hz, 1H), 7.70 (d, J = 2.2 Hz, 1H), 7.60 (d, J = 8.5 Hz, 1H), 7.23 (dd, J = 7.9, 1.3 Hz, 1H), 7.13-7.06 (m, 2H), 7.04-7.00 (m, 1H), 3.46 (br s, 4H), 3.35 (s, 2H), 2.53 (br s, 4H). $^{13}$C{$^1$H} (126 MHz, DMSO-$d_6$) δ 162.6, 158.1, 156.2, 150.8, 144.3, 139.9, 130.3, 126.8, 126.6, 126.2, 126.1, 123.6, 124.4, 123.6, 122.6, 120.4, 53.3, 52.0, 46.7; HPLC-purity (254 nm): 99%; MALDI-HRMS: m/z calculated for $C_{21}H_{20}F_3N_6O_2$ [M+H]$^+$: 445.1594, found: 445.1592.

**2-(2-(4-(2-Chlorodibenzo[*b,f*][1,4]thiazepin-11-yl)piperazin-1-yl)ethoxy)acetic acid (Compound 12)**

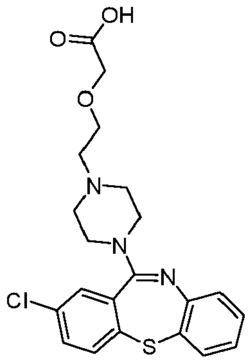

Synthesized according to GP8 from

2-chloro-11-(piperazin-1-yl)dibenzo[b,f][1,4]thiazepine (45 mg, 0.136 mmol), 2-(2-chloroethoxy)acetic acid (37.7 mg, 0.272 mmol), and triethylamine (191 μL, 1.36 mmol). Purification of the crude product by preparative HPLC yielded the title compound (19.6 mg, 33%). $^1$H NMR (300 MHz, $CDCl_3$) δ 7.46 (d, J = 8.3 Hz, 1H), 7.39 (dd, J = 7.7, 1.3 Hz, 1H), 7.35-7.31 (m, 2H), 7.24-7.18 (m, 1H), 7.07 (dd, J = 7.9, 1.2 Hz, 1H), 6.94 (td, J = 7.6, 1.4 Hz, 1H), 4.25 (s, 2H), 3.84-3.36 (m, 12H). $^{13}$C{$^1$H} NMR (75 MHz, $CDCl_3$) δ 168.5, 159.2, 148.1, 138.3, 135.1, 134.8, 133.5, 132.2, 131.3, 129.4, 128.5, 127.4, 125.3, 123.7, 73.6, 70.0, 61.7, 44.1, 41.5; HPLC-purity (254 nm): 99%; MALDI-HRMS: m/z calculated for $C_{21}H_{23}ClN_3O_3S$ [M+H]$^+$: 432.1143, found: 432.1148.

**11-(Piperazin-1-yl)-2-(trifluoromethyl)dibenzo[*b,f*][1,4]oxazepane (Compound 13)**

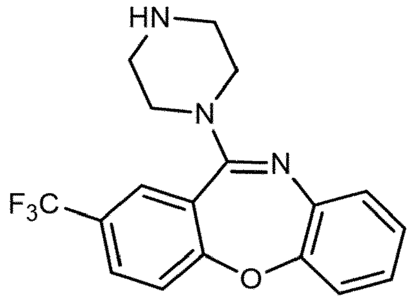

Synthesized according to GP7 from 11-chloro-2-(trifluoromethyl)dibenzo[b,fJ[1,4]oxazepine (267 mg, 0.897 mmol) and piperazine (155 mg, 1.79 mmol) in. Purification of the crude product by flash chromatography (DCM/MeOH 95:5 to 9:1) yielded the title compound as a yellow solid (240 mg, 77%). 1H NMR (400 MHz, $CDCl_3$) δ 8.77 (s, 2H), 8.47 (br s, 1H), 7.73 (d, J = 8.0 Hz, 1H), 7.64-7.60 (m, 1H), 7.37 (d, J = 8.0 Hz, 1H), 7.18-7.03 (m, 4H), 3.77-3.23 (m, 8H).

**11-(1,4-Diazepan-1-yl)-2-(trifluoromethyl)dibenzo[*b,f*][1,4]oxazepine (Compound 14)**

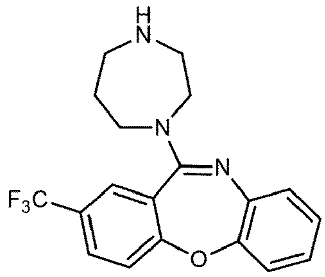

Synthesized according to GP7 from 11-chloro-2-(trifluoromethyl)dibenzo[b,f][1,4]oxazepine (267 mg, 0.897 mmol) and homopiperazine (183 mg, 1.79 mmol). Purification of the crude product by flash chromatography (DCM/MeOH 95:5 to 9:1) yielded the title compound as a yellow solid (178 mg, 55%). 1H NMR (400 MHz, $CDCl_3$) δ 7.69 (dd, J = 8.6, 2.0, 1H), 7.63 (d, J = 1.9 Hz, 1H), 7.36 (d, J = 8.5 Hz, 1H), 7.13-7.06 (m, 3H), 7.99-6.95 (m, 1H), 3.98-3.13 (m, 9H), 2.14-1.90 (m, 2H).

**3-((4-(2-Chlorodibenzo[*b,f*][1,4]oxazepin-11-yl)piperazin-1-yl)methyl)-1,2,4-oxadiazol-5(4*H*)-one (Compound 15)**

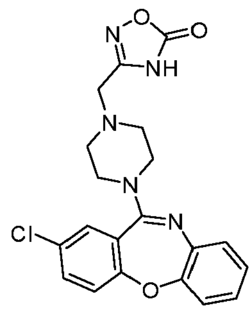

To a solution of **Compound 17** (22.0 mg, 0.0570 mmol) in EtOH (2.0 mL) a sodium methoxide solution in MeOH (5 M, 39.1 μL, 0.171 mmol) and diethyl carbonate (27.9 μL, 0.228 mmol) were added at rt and reaction mixture was heated to reflux overnight. Purification of the crude product by preparative HPLC yielded the title compound (11.0 mg, 47%). $^1$H NMR (400 MHz, DMSO-$d_6$) δ 7.62 (dd, J = 8.7, 2.6 Hz, 1H), 7.43 (d, J = 2.4 Hz, 1H), 7.40 (d, J = 9.6 Hz, 1H), 7.20-7.17 (m, 1H), 7.12-6.98 (m, 3H), 3.53-3.48 (m, 6H), 2.60 (br s, 4H). $^{13}$C{$^1$H} (101 MHz, DMSO-$d_6$) δ 159.9, 158.7, 158.0., 157.4, 151.2, 139.9, 133.0, 129.5, 128.7, 126.5, 125.8, 124.4, 124.3, 123.1, 120.2, 51.9, 51.2, 46.6. MALDI-HRMS: m/z calculated for $C_{20}H_{19}ClN_5O_3$ $[M+H]^+$: 412.1171, found: 412.1171.

**2-(4-(2-Chlorodibenzo[*b,f*][1,4]oxazepin-11-yl)piperazin-1-yl)acetonitrile (Compound 16)**

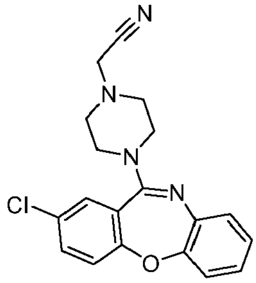

Synthesized according to GP8 from Amoxapine (150 mg, 0.464 mmol), bromoacetonitrile (66.6 μL, 0.927 mmol), and triethylamine (653 μL, 4.64 mmol). Purification of the crude product by flash chromatography (DCM/MeOH 99:1 to 95:5) yielded the title compound (75.1 mg, 46%). $^1$H NMR (400 MHz, $CDCl_3$) δ 7.39 (dd, J = 8.5, 2.6 Hz, 1H), 7.32 (d, J = 2.2 Hz, 1H), 7.20-7.15 (m, 2H), 7.12-7.06 (m, 2H), 7.03-6.98 (m, 1H), 3.59-3.51 (m, 6H), 2.71 (s, 4H). $^{13}$C{$^1$H} (101 MHz, $CDCl_3$) δ 159.5, 159.0, 152.0, 139.6, 132.9, 130.5, 129.1, 127.2, 126.0, 125.1, 124.8, 122.9, 120.3, 114.6, 51.6, 47.2, 46.1. MALDI-HRMS: m/z calculated for $C_{19}H_{18}ClN_4O$ $[M+H]^+$: 353.1164, found: 353.1165.

**2-(4-(2-Chlorodibenzo[b,f][1,4]oxazepin-11-yl)piperazin-1-yl)-Ar-hydroxyacetimidamide (Compound 17)**

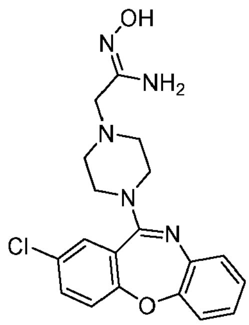

To a solution of **Compound 16** (50.0 mg, 0.140 mmol) in EtOH (1.0 mL) an aq hydroxylamine solution (50 wt%, 23.2 μL, 0.379 mmol) was added at rt and reaction mixture was heated to reflux overnight. Purification of the crude product by flash chromatography (DCM/MeOH 99:1 to 95:5) yielded the title compound (35.4 mg, 65%). $^1$H NMR (400 MHz, DMSO-$d_6$) δ 9.07 (br s, 1H), 7.61 (dd, J = 8.7, 2.6 Hz, 1H), 7.41-7.37 (m, 2H), 7.20-7.17 (m, 1H), 7.10-6.98 (m, 3H), 5.30 (s, 2H), 3.47 (br s, 4H), 2.92 (s, 2H). 2.54-2.46 (m, 4H). $^{13}$C{$^1$H} (101 MHz, DMSO-$d_6$) δ 158.6, 158.1, 151.2, 149.9, 139.9, 132.9, 129.5, 128.7, 126.5, 125.8, 124.5, 124.2, 123.1, 120.2, 57.8, 52.1, 46.8. MALDI-HRMS: m/z calculated for $C_{19}H_{21}ClN_5O_2$ $[M+H]^+$: 386.1378, found: 386.1371.

**5-((4-(8-Fluoro-2-(trifluoromethyl)dibenzo[*b,f*][1,4]oxazepin-11-yl)piperazin-1-yl)methyl)-1,2-dihydro-3*H*-1,2,4-triazol-3-one (Compound 18)**

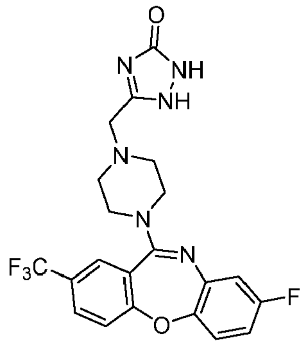

Synthesized according to GP8 from **Compound 187** (36.0 mg, 0.0985 mmol), 3-chloromethyl)-1*H*-1,2,4-triazol-5(4*H*)-one (27.1 mg, 0.197 mmol), and triethylamine (137 μL, 0.985 mmol). Purification of the crude product by preparative HPLC yielded the title compound (13.2 mg, 29%). $^1H$ NMR (500 MHz, DMSO-$d_6$) δ 11.36 (s, 1H), 11.26 (s, 1H), 7.97 (dd, J = 8.7, 2.1 Hz, 1H), 7.72 (d, J = 2.1 Hz, 1H), 7.61 (d, J = 8.5 Hz, 1H), 7.28-7.24 (m, 1H), 6.87-6.80 (m, 2H), 3.49 (br s, 4H), 3.36 (s, 2H), 2.60-2.44 (m, 4H). $^{13}C\{^1H\}$ (126 MHz, DMSO-$d_6$) δ 162.5, 160.7, 158.7, 158.5, 156.2, 147.1, 144.3, 141.48, 128.7, 126.3, 124.6, 123.3, 122.5, 121.4, 112.3, 110.3, 53.2, 51.9, 45.7. MALDI-HRMS: *m*/*z* calculated for $C_{21}H_{19}F_4N_6O_2$ $[M+H]^+$: 463.1500, found: 463.1499.

**5-((4-(2-Chloro-8-fluorodibenzo[*b*,*f*][1,4]oxazepin-11-yl)piperazin-1-yl)methyl)-1,2-dihydro-3H-1,2,4-triazol-3-one (Compound 19)**

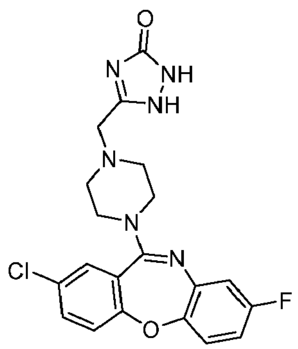

Synthesized according to GP8 from **Compound 175** (50.0 mg, 0.151 mmol), 3-chloromethyl)-1*H*-1,2,4-triazol-5(4*H*)-one (41.5 mg, 0.115 mmol), and triethylamine (210 μL, 1.51 mmol). Purification of the crude product by preparative HPLC yielded the title compound (38.9 mg, 60%). $^1H$ NMR (400 MHz, DMSO-$d_6$) δ 11.38 (s, 1H), 11.26 (s, 1H), 7.44-7.16 (m, 5H), 6.83-6.76 (m, 2H), 3.56 (s, 4H), 3.35 (s, 2H), 2.59-2.41 (m, 4H). $^{13}C\{^1H\}$ (101 MHz, DMSO-$d_6$) δ 160.8, 158.5, 158.4, 156.2, 147.5, 144.3, 141.8, 133.1, 129.6, 128.8, 124.2, 123.0, 121.6, 112.2, 110.1, 53.2, 52.0, 46.5. MALDI-HRMS: m/z calculated for $C_{20}H_{19}ClFN_6O_2$ $[M+H]^+$: 429.1237, found: 429.1239.

**5-((4-(2-(Trifluoromethyl)dibenzo[b,f][1,4]oxazepin-11-yl)-1,4-diazepan-1-yl)methyl)-1,2-dihydro-3H-1,2,4-triazol-3-one (Compound 20)**

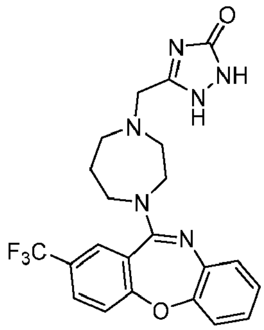

Synthesized according to GP8 from **Compound 14** (20.0 mg, 0.0553 mmol), 3-chloromethyl)-1*H*-1,2,4-triazol-5(4*H*)-one (15.2 mg, 0.111 mmol), and triethylamine (77.9 µL, 0.553 mmol). Purification of the crude product by flash chromatography (DCM/MeOH 95:5 to 8:2) yielded the title compound (16.8 mg, 67%). $^1H$ NMR (500 MHz, DMSO-$d_6$) δ 11.29 (s, 1H), 11.20 (s, 1H), 7.91 (dd, J = 8.6, 2.1 Hz, 1H), 7.77 (d, J = 2.0 Hz, 1H), 7.58 (d, J = 8.5 Hz, 1H), 7.20 (dd, J = 8.0, 1.4 Hz, 1H), 7.08-7.01 (m, 2H), 6.96-6.92 (m, 1H), 3.60 (s, 4H), 3.44 (s, 2H), 2.80-2.64 (m, 4H), 1.98-1.75 (m, 2H). $^{13}C\{^1H\}$ (126 MHz, DMSO-$d_6$) δ 162.2, 157.7, 156.2, 150.6, 145.2, 140.6, 129.7, 126.7, 126.4, 126.0, 125.7, 123.9, 123.7, 123.3, 122.3, 120.2. MALDI-HRMS: m/z calculated for $C_{22}H_{22}F_3N_6O_2$ $[M+H]^+$: 459.1751, found: 459.1752.

**2-(4-(8-Fluoro-2-(trifluoromethyl)dibenzo[*b,f*][1,4]oxazepin-11-yl)piperazin-1-yl)ethan-1-ol (Compound 21)**

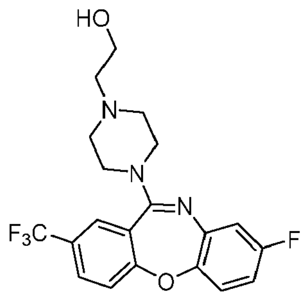

Synthesized according to GP7 from **Compound 232** (42.0 mg, 0.133 mmol) and 1-(2-hydroxyethyl)piperazine (33.3 µL, 0.266 mmol). Purification of the crude product by flash chromatography (DCM/MeOH 99:1 to 95:5) yielded the title compound (24.4 mg, 44%). $^1H$ NMR (400 MHz, $CDCl_3$) δ 7.72 (dd, J = 8.5, 1.8 Hz, 1H), 7.61 (d, J = 2.3 Hz, 1H), 7.36 (d, J = 8.8 Hz, 1H), 7.07-7.04 (m, 1H), 6.85-6.82 (m, 1H), 6.71-6.67 (m, 1H), 3.62 (br s, 4H), 3.17-2.93 (m, 5H). $^{13}C\{^1H\}$ (101 MHz, $CDCl_3$) δ 163.2, 160.5, 159.4, 147.7, 141.4, 129.9, 127.7, 127.1, 123.6, 124.1, 122.2, 120.9, 113.4, 110.4, 59.5, 57.9, 52.8, 47.5. MALDI-HRMS: *m*/*z* calculated for $C_{20}H_{20}F_4N_3O_2$ $[M+H]^+$: 410.1486, found: 410.1485

**Methyl 5-chloro-2-(2-nitrophenoxy)benzoate (Compound 22)**

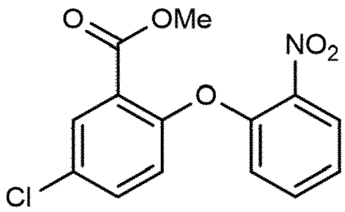

Synthesized according to GP1 from methyl 5-chlorosalicylate (2.80 g, 15.0 mmol), 2-fluoronitrobenzene (1.06 mL, 10.0 mmol) and potassium carbonate (2.07 g, 15 mmol). Purification of the crude product by flash chromatography (n-hexane/EtOAc 9:1 to 8:2) yielded the title compound as a colorless solid (3.07 g, 99%). 1H NMR (250 MHz, DMSO-$d_6$) δ 8.07 (dd, J = 8.1, 1.7 Hz, 1H) 7.92 (d, J = 2.7 Hz, 1H), 7.75 (dd, J = 8.8, 2.8 Hz, 1H) 7.68-7.60 (m, 1H), 7.37-7.30

(m, 1H), 7.27 (d, J = 8.8 Hz, 1H), 7.00 (d, J = 8.4, 1.1 Hz, 1H), 3.70 (s, 3H).

**Methyl 2-(2-nitrophenoxy)-5-(trifluoromethyl)benzoate (Compound** 23)

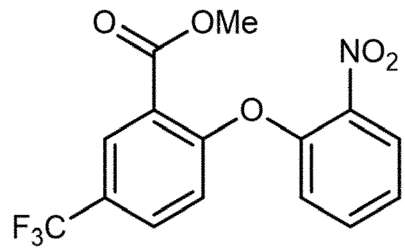

Synthesized according to GP1 from methyl 2-hydroxy-5-(trifluoromethyl)benzoate (991 mg, 4.50 mmol), 2-fluoronitrobenzene (317 μL, 3.00 mmol) and potassium carbonate (622 mg, 4.50 mmol). Purification of the crude product by flash chromatography (hexane/EtOAc 9:1 to 8:2) yielded the title compound as a colorless solid (615 mg, 60%). 1H NMR (250 MHz, $CDCl_3$) δ 8.27 (s, 1H) 8.03 (d, J = 8.2 Hz, 1H), 7.75 (d, J = 8.6 Hz, 1H), 7.56 (t, J = 8.2 Hz, 1H), 7.29 (t, J = 8.2 Hz, 1H), 7.08 (d, J = 8.6 Hz, 1H), 6.98 (d, J = 8.3 Hz, 1H), 3.85 (s, 3H).

**Methyl 2-(2-aminophenoxy)-5-chlorobenzoate (Compound 24)**

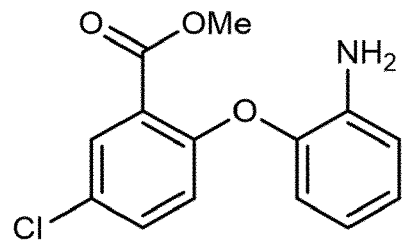

Synthesized according to GP2 from methyl 5-chloro-2-(2-nitrophenoxy)benzoate (954 mg, 3.10 mmol) and tin (II) chloride dihydrate (2.80 g, 12.4 mmol). Purification of the crude product by flash chromatography (hexane/EtOAc 9:1 to 8:2) yielded the title compound as a colorless solid (635 mg, 74%). 1H NMR (300 MHz, $CDCl_3$) δ 7.82 (d, J = 2.6 Hz, 1H), 7.33 (dd, J = 8.7, 2.6 Hz, 1H), 7.04-6.97 (m, 1H), 6.88-6.80 (m, 3H), 6.71 (td, J = 7.7, 1.5 Hz, 1H), 3.89 (s, 3H), 3.64 (br s, 2H).

**Methyl 2-(2-aminophenoxy)-5-(trifluoromethyl)benzoate (Compound 25)**

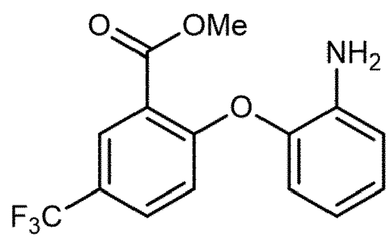

Synthesized according to GP2 from methyl 2-(2-nitrophenoxy)-5-(trifluoromethyl)benzoate (334 mg, 0.980 mmol) and tin (II) chloride dihydrate (900 mg, 3.91 mmol). Purification of the crude product by flash chromatography (hexane/EtOAc 9:1 to 8:2) yielded the title compound as a colorless solid (171 mg, 56%). 1H NMR (250 MHz, $CDCl_3$) δ 8.12 (d, J = 2.1 Hz, 1H), 7.61 (dd, J = 8.7, 2.4 Hz, 1H), 7.10-7.02 (m, 1H), 6.98-6.93 (m, 2H), 6.84 (dd, J = 7.9, 1.5 Hz, 1H), 6.79-6.72 (m, 1H), 3.99 (br s, 2H), 3.94 (s, 3H).

**2-Chlorodibenzo[b,f][1,4]oxazepin-11(10H)-one (Compound 26)**

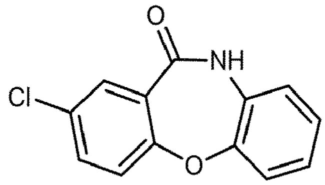

Synthesized according to GP3 from methyl 2-(2-aminophenoxy)-5-chlorobenzoate (630 mg, 2.27 mmol) and concentrated sulphuric acid (150 μL, 2.81 mmol). After filtration the title compound was obtained as a colorless solid (508 mg, 90%). 1H NMR (250 MHz, DMSO-d6) δ 10.66 (s, 1H), 7.72 (d, J = 2.5 Hz, 1H) 7.67 (dd, J = 8.6, 2.8 Hz, 1H), 7.40 (d, J = 8.6 Hz, 1H) 7.34 (dt, J = 7.1, 1.2 Hz, 1H), 7.21-7.10 (m, 3H).

**2-(Trifluoromethyl)dibenzo[b,f][1,4]oxazepin-11(10H)-one (Compound 27)**

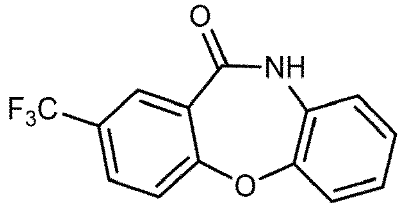

Synthesized according to GP3 from methyl 2-(2-aminophenoxy)-5-(trifluoromethyl)benzoate (166 mg, 0.533 mmol) and concentrated sulphuric acid (35.3 μL, 0.663 mmol). After filtration the title compound was obtained as a colorless solid (73 mg, 49%). 1H NMR (250 MHz, DMSO-$d_6$) δ 10.77 (s, 1H), 8.05-7.98 (m, 2H) 7.59 (d, J = 8.3 Hz, 1H), 7.39 (dt, J = 7.3, 1.2 Hz, 1H), 7.23-7.13 (m, 3H).

**2-Fluoro-*N*-(5-fluoro-2-hydroxyphenyl)-5-(trifluoromethyl)benzamide (Compound 28)**

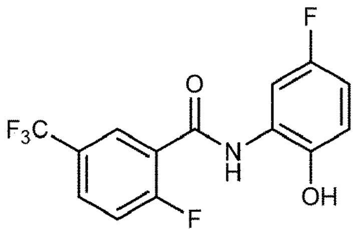

Synthesized according to GP4 from 2-fluoro-5-(trifluoromethyl)benzoic acid (1.50 g, 7.06 mmol), thionyl chloride (1.04 mL, 14.1 mmol), 2-amino-4-fluorophenol (925 mg, 7.06 mmol) and triethylamine (1.98 mL, 14.1 mmol). Purification of the crude product by flash chromatography (n-hexane/EtOAc 99:1 to 2:3) yielded the title compound as a red solid (1.43 g, 64%). 1H NMR (400 MHz, DMSO-$d_6$) δ 8.17-8.13 (m, 1H), 8.05-7.91 (m, 2H), 7.66-7.59 (m, 1H), 6.93-6.80 (m, 2H).

**8-Fluoro-2-(trifluoromethyl)dibenzo[*b*,*f*][1,4]oxazepin-11(10*H*)-one (Compound 29)**

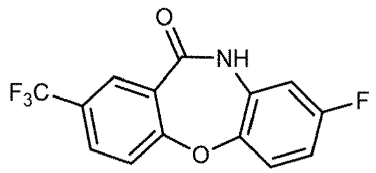

Synthesized according to GP5 from 2-fluoro-*N*-(5-fluoro-2-hydroxyphenyl)-5-(trifluoromethyl)benzamide (196 mg, 0.618 mmol), and sodium hydroxide (25 mg, 0.618 mmol). After filtration the title compound was obtained as a light-brown solid (168 mg, 92%). 1H NMR (250 MHz, DMSO-$d_6$) δ 10.85 (s, 1H), 8.04-8.00 (m, 2H), 7.61-7.58 (m, 1H), 7.48-7.40 (m, 1H), 7.06-6.97 (m, 2H).

**2,11-Dichlorodibenzo[*b*,*f*][1,4]oxazepane (Compound 30)**

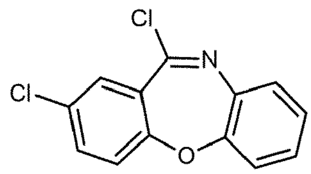

Synthesized according to GP6 from 2-chlorodibenzo[*b*,*f*][1,4]oxazepin-11(10*H*)-one (558 mg, 2.27 mmol), phosphorus oxychloride and *N*,*N*-dimethylaniline (173 μL, 1.36 mmol). After work up the title compound was obtained as brown solid (598 mg, 99%).

11-Chloro-2-(trifluoromethyl)dibenzo[b,fJ[1,4]oxazepine (Compound 32)

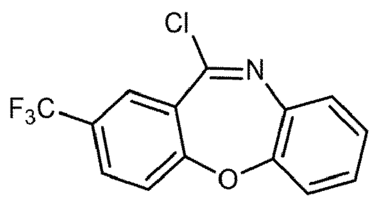

Synthesized according to GP6 from 2-(trifluoromethyl)dibenzo[b,f][1,4]oxazepin-11(10*H*)-one (73 mg, 0.261 mmol), phosphorus oxychloride and *N*,*N*-dimethylaniline (20.3 μL, 0.160 mmol). After work up the title compound was obtained as a brown solid (64 mg, 82%).

**2,11-Dichlorodibenzo[*b*,*f*][1,4]thiazepine (Compound 33)**

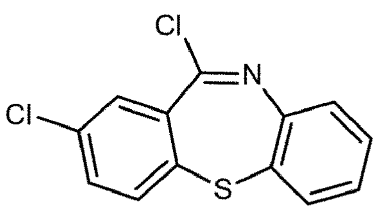

Synthetized according to GP6 from commercially purchased 2-chlorodibenzo[*b*,*f*][1,4]thiazepin-11(10*H*)-one (125 mg, 0.478 mmol), phosphorus oxychloride and *N*,*N*-dimethylaniline (36.8 μL, 0.290 mmol). After work up the title compound was obtained as a brown solid.

**11-Chloro-8-fluoro-2-(trifluoromethyl)dibenzo[b,fJ[1,4]oxazepine (Compound 34)**

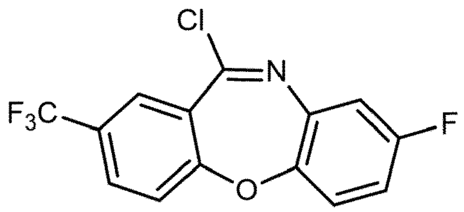

Synthetized according to GP6 from 8-fluoro-2-(trifluoromethyl)dibenzo[*b*,*f*][1,4]oxazepin-11(10*H*)-one (120 mg, 0.404 mmol), phosphorus oxychloride and *N*,*N*-dimethylaniline (31.0 μL, 0.242 mmol). After work up the title compound was obtained as a light-brown solid.

**Methyl 5-chloro-2-(4-fluoro-2-nitrophenoxy)benzoate (Compound 35)**

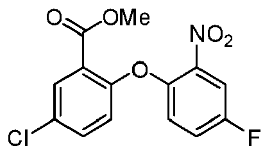

Synthesized according to GP1 from methyl 5-chlorosalicylate (700 mg, 3.75 mmol), 2,5-difluoronitrobenzene (271 μL, 2.50 mmol) and potassium carbonate (524 mg, 3.75 mmol). Purification of the crude product by flash chromatography (n-hexane/EtOAc 9:1 to 8:2) yielded the title compound (811 mg, 99%). $^1$H NMR (250 MHz, $CDCl_3$) δ 7.97 (d, J = 2.7 Hz, 1H), 7.73 (dd, J = 7.7, 3.1 Hz, 1H), 7.50 (dd, J = 8.8, 2.7 Hz, 1H), 7.27-7.19 (m, 1H), 6.99 (d, J = 8.7 Hz, 1H), 6.87 (dd, J = 9.2, 4.4 Hz, 1H), 3.81 (s, 3H).

**Methyl 2-(2-amino-4-fluorophenoxy)-5-chlorobenzoate (Compound 36)**

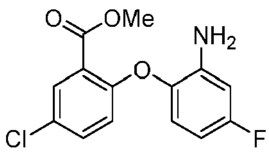

Synthesized according to GP2 from **Compound 50** (1.02 g, 3.14 mmol) and tin (II) chloride dihydrate (2.89 g, 12.6 mmol). Purification of the crude product by flash chromatography (n-hexane/EtOAc 9:1 to 8:2) yielded the title compound (630 mg, 68%). $^1$H NMR (250 MHz, $CDCl_3$) δ 7.80 (d, J = 2.7 Hz, 1H), 7.34 (dd, J = 8.9, 2.7 Hz, 1H), 6.87-6.80 (m, 2H), 6.52 (dd, J = 9.9, 2.9 Hz, 1H), 6.44-6.35 (m, 1H), 3.97 (br s, 2H), 3.91 (s, 3H).

**2-Chloro-8-fluorodibenzo[*b*,*f*][1,4]oxazepin-11(10H)-one (Compound 37)**

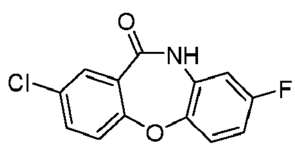

Synthesized according to GP3I from **Compound 68** (625 mg, 2.11 mmol) and concentrated sulphuric acid (141

μL, 2.64 mmol). After work up the title compound was obtained (499 mg, 90%). $^1$H NMR (250 MHz, DMSO-$d_6$) δ 10.73 (br s, 1H), 7.72 (d, J = 2.3 Hz, 1H), 7.68 (dd, J = 8.4, 1.4 Hz, 1H), 7.43-7.36 (m, 2H), 7.04-6.94 (m, 2H).

**2,11-Dichloro-8-fluorodibenzo[*b,f*][1,4]oxazepine (Compound 38)**

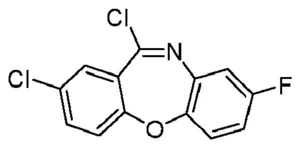

Synthesized according to GP6 from **Compound 90** (150 mg, 0.569 mmol), phosphorus oxychloride and *N*,*N*-dimethylaniline (43.1 μL, 0.340 mmol). After work up the title compound was obtained (138 mg, 86%).

**2-Chloro-8-fluoro-11-(piperazin-1-yl)dibenzo[*b,f*][1,4]oxazepine (Compound 39)**

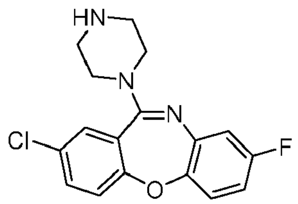

Synthesized according to GP7 from **Compound 115** (94.6 mg, 0.335 mmol) and piperazine (57.8 mg, 0.671 mmol). Purification of the crude product by flash chromatography (DCM/MeOH 99:1 to 8:2) yielded the title compound (74.1 mg, 67%). $^1$H NMR (400 MHz, $CDCl_3$) δ 7.72 (dd, J = 8.5, 1.8 Hz, 1H), 7.61 (d, J = 2.3 Hz, 1H), 7.36 (d, J = 8.8 Hz, 1H), 7.07-7.04 (m, 1H), 6.85-6.82 (m, 1H), 6.71-6.67 (m, 1H), 3.62 (br s, 4H), 3.17-2.93 (m, 5H). $^{13}$C{$^1$H} (101 MHz, $CDCl_3$) δ 161.5, 159.2, 159.1, 148.0, 141.2, 133.1, 130.7, 129.0, 124.5, 122.8, 120.8, 120.7, 112.2, 46.7, 44.6. MALDI-HRMS: m/z calculated for $C_{17}H_{16}ClFN_3$ $[M+H]^+$: 332.0960, found: 322.0959.

**8-Fluoro-11-(piperazin-1-yl)-2-(trifluoromethyl)dibenzo[b,fJ[1,4]oxazepine (Compound 40)**

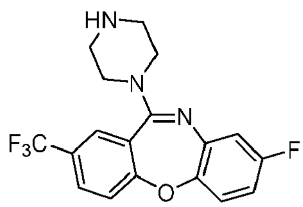

Synthesized according to GP7 from **Compound 232** (128 mg, 0.404 mmol) and piperazine (69.6 mg, 0.808 mmol). Purification of the crude product by flash chromatography (DCM/MeOH 99:1 to 8:2) yielded the title compound (77.3 mg, 52%). $^1$H NMR (400 MHz, $CDCl_3$) δ 7.72 (dd, J = 8.5, 1.8 Hz, 1H), 7.61 (d, J = 2.3 Hz, 1H), 7.36 (d, J = 8.8 Hz, 1H), 7.07-7.04 (m, 1H), 6.85-6.82 (m, 1H), 6.71-6.67 (m, 1H), 3.62 (br s, 4H), 3.17-2.93 (m, 5H). $^{13}$C{$^1$H} (101 MHz, $CDCl_3$) δ 163.3, 162.4, 159.4, 147.7, 141.4, 130.1, 127.8, 127.1, 124.7, 124.0, 122.5, 122.2, 120.9, 112.3, 45.3, 29.9. MALDI-HRMS: m/z calculated for $C_{18}H_{16}F_4N_3O$ $[M+H]^+$: 366.1224, found: 366.1226.

**2-Fluoro-*N*-(5-fluoro-2-hydroxyphenyl)-5-(trifluoromethyl)benzamide (Compound 41)**

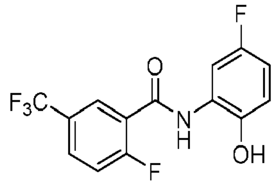

Synthesized according to GP4 from 2-fluoro-5-(trifluoromethyl)benzoic acid (1.50 g, 7.06 mmol), thionylchloride (1.0 mL, 14.1 mmol), 2-amino-4-fluorophenol (925 mg, 7.06 mmol), and triethylamine (2.0 mL, 14.1 mmol). Purification of the crude product by flash chromatography (n-hexane/EtOAc 99:1 to 4:6) yielded the title compound (1.43 g, 64%). $^1H$ NMR (250 MHz, $CDCl_3$) δ 8.15 (dd, J = 6.4, 2.1 Hz, 1H), 8.05-7.98 (m, 1H), 7.94 (dd, J = 10.4, 2.5 Hz, 1H), 7.63 (t, J = 9.6 Hz, 1H), 6.94-6.80 (m, 2H).

**8-Fluoro-2-(trifluoromethyl)dibenzo[*b,f*][1,4]oxazepin-11(10H)-one (Compound 42)**

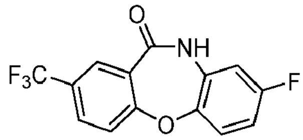

Synthesized according to GP5 from **Compound 230** (196 mg, 0.618 mmol) and sodium hydroxide (25 mg, 0.618 mmol). After work up the title compound was obtained (168 mg, 92%). $^1H$ NMR (250 MHz, DMSO-$d_6$) δ 10.85 (br s, 1H), 8.05-8.00 (m, 2H), 7.60 (d, J = 8.1 Hz, 1H), 7.48-7.40 (m, 1H), 7.06-6.97 (m, 2H).

**11-Chloro-8-fluoro-2-(trifluoromethyl)dibenzo[*b,f*][1,4]oxazepine (Compound 43)**

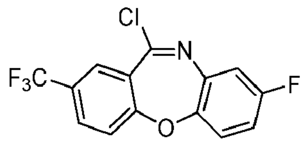

Synthesized according to GP6 from Compound 231 (120 mg, 0.404 mmol), phosphorus oxychloride and *N*,*N*-dimethylaniline (31.0 μL, 0.242 mmol). After work up the title compound was obtained (50.4 mg, 39%).

**Example 3: Functional activity testing**

Functional activity at human Slack was screened *in vitro* using a cell-based FluxOR assay in comparison to Loxapine as the reference activator. For that purpose, cultured HEK293 cells stably expressing human Slack (herein referred to as HEK-Slack cells) were incubated with compounds in a $Na^+$ free buffer to prevent compound-independent Slack activation by monovalent cations[7,19].

In first experiments, the inventors established that the modified FluxOR assay is capable to detect Slack activation by Loxapine (Fig. 2). Additionally, in dose-response experiments with the Slack inhibitor "Compound 31" (ref[18])

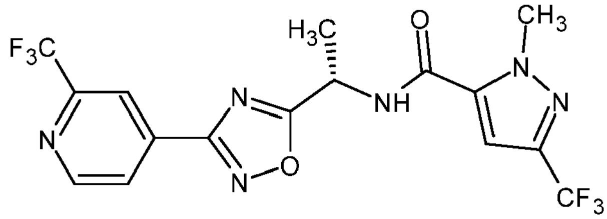
(Compound 31)

in a $Na^+$ free buffer with 2 mM $Ca^{2+}$, 2 mM $Mg^{2+}$ and 0.03% DMSO and pre-stimulation with 25 μM Loxapine revealed that compound 31 inhibited the F/F(Baseline) ratio in a dose-dependent manner ($IC_{50}$ = 2.1 μM), confirming that the readout depends on Slack. Subsequently, dose-response curves were conducted to determine the $EC_{50}$ and $E_{MAX}$ values.

In detail, in these experiments, the Slack-activating efficacy and potency of the novel compounds was determined using a commercial FluxOR™ Potassium Ion Channel Assay (Invitrogen). HEK293 cells stably expressing human Slack (herein referred to as HEK-Slack cells) were plated at a density of 50,000 cells per well of a poly-D-lysine coated (75 μg/mL, Sigma Aldrich) 96-well, black-walled microplate with clear bottom (Greiner Bio-One) in DMEM containing 10% FBS and 1% penicillin/streptomycin 24 hours prior to assaying.

On the experiment day, cells were loaded with FluxOR™ dye according to the manufacturer's protocol. A $Na^+$-free assay buffer containing 140 mM choline chloride, 5 mM KCl, 2 mM $CaCl_2$, 2 mM $MgSO_4$, 10 mM HEPES, 5.55 mM glucose, adjusted to pH 7.4 with KOH, was used. To reduce background fluorescence all assay components were complemented by 10% BackDrop™ Background Suppressor (Invitrogen). To reduce background fluorescence all assay components were complemented by 10% BackDrop™ Background Suppressor (Invitrogen).

After washing steps, cells were incubated with compounds for 30 min at 37 °C. All compounds were prepared as a 33.3 mM DMSO stock and diluted at the experimental day to a final concentration of 100, 50, 25, 12.5, 6.25, and 3.125 μM in assay buffer containing 0.03% DMSO. As a positive control 50 μM Loxapine was used. After stimulation with $Tl_2SO_4$ solution, the fluorescence was measured over 100 s (excitation 485 nm, emission 525 nm) using an Infinite M200 Microplate Reader (Tecan). Assays run in triplicate and the ratio to baseline fluorescence was calculated for all time points. Dose-response curves comprising six concentrations were performed in triplets using fluorescence/fluorescence$_{Baseline}$ ratios at the $100^{th}$ second. Values were calculated relative to Loxapine within each individual experiment as follows: $100 \times ((F/F_B(\text{Compound}) - F/F_B(\text{Vehicle}_{mean}) / (F/Fs(\text{Loxapine}) - F/F_B(\text{Vehicle}_{mean}))$. To generate $EC_{50}$ and $E_{MAX}$ values a standard, logistic, nonlinear regression analysis with GraphPad Prism 9.0 was used.

Out of 125 screened new compounds, 45 performed as Slack activators to a variable extent. The most interesting candidates exhibit a range of potencies between 3.2 and 62.5 μM), whereas Loxapine activated Slack with a potency of 20.7 μM **(Fig. 3A, B and** Table 3333Table 33). Efficacy ($E_{MAX}$) values ranged from 63.1% to 264% as compared to Loxapine **(Fig. 3A, B and** Table 3333Table 33). Considering that Loxapine at a low dose exerted Slack-dependent effects in mice[19] and that the FluxOR assay was performed in a buffer containing 2 mM $Ca^{2+}$ that inhibits Slack activity[13] the inventors concluded that the new compounds might be sufficient to activate Slack at standard doses *in vivo,* albeit their $EC_{50}$ values are in the micromolar range. For compounds 10, 11 and 18-21, the corresponding time-dependent fluorescence/fluorescence$_{Baseline}$ ratios are shown in **Fig. 3E** in comparison with Loxapine and vehicle.

**Table** 33333: **Structural features of the most promising new compounds and their Slack-activating properties.** Potency ($EC_{50}$) and efficacy ($E_{MAX}$; % relative to Loxapine) values are from a FluxOR assay in HEK-Slack cells that is shown in **Fig. 3A, B.** New compounds were measured in triplicate. $EC_{50}$ and $E_{MAX}$ values are presented as mean with 95% confidence interval.

| | **Substituen** | **B** | **E** | **A** | **X** | **n** | **FluxOR assay** | |
|---|---|---|---|---|---|---|---|---|
| | **Compound** | | | | | | $EC_{50}$ | $E_{MAX}$ |
| | | | | | | | μM | % |
| | **1 (Loxapine)** | Cl | 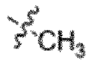 | H | O | 1 | 20.7 (16.3-26.4) | 100 |
| | **2** | $CF_3$ | 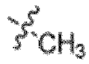 | H | O | 1 | 6.3 (5.3 - 7.4) | 114.3 (101.0 - 127.6) |
| | **3** | $CF_3$ | 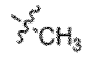 | H | O | 2 | 3.2 (2.3 - 4.5) | 63.1 (55.0 - 71.3) |
| | **4** | $CF_3$ | 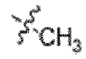 | F | O | 1 | 41.5 (36.2-47.6) | 199.1 (174.8 - 223.4) |
| | **5** | Cl | 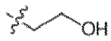 | H | O | 1 | 26.0 (21.7 - 31.1) | 222.8 (193.6 - 252.0) |
| | **6** | Cl | 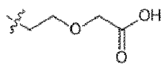 | H | O | 1 | 30.3 (20.8 - 44.1) | 96.2 (72.4 - 120.0) |
| | **7** | Cl | | H | S | 1 | 30.0 (23.9-37.7) | 87.4 (73.9 - 101.0) |
| | **8** | $CF_3$ | 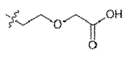 | H | O | 1 | 62.5 (49.3-79.2) | 177.0 (138.7 - 215.3) |
| | **9** | $CF_3$ | 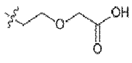 | H | O | 2 | 50.9 (46.0-56.3) | 186.4 (169.7-203.2) |
| | **10** | Cl | | H | O | 1 | 22.1 (19.9-24.5) | 120.2 (112 - 128.4) |
| | **11** | $CF_3$ | | H | O | 1 | 10.74 (9.0 - 12.9) | 199.4 (184.7-214.2) |
| | **18** | $CF_3$ | | F | O | 1 | 11.16 (8.4-14.9) | 126.8 (110.2-143.3) |
| | **19** | Cl | | F | O | 1 | 21.1 (18.1 - 24.5) | 117.4 (106.9 - 127.9) |
| | **20** | $CF_3$ | | H | O | 2 | 26.2 (21.0 - 32.8) | 128.8 (109.5-148.1) |
| | **21** | $CF_3$ | 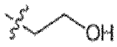 | F | O | 1 | 10.45 (8.9-12.3) | 264.2 (247.9 - 280.4) |

When interpreting the $EC_{50}$ values obtained in the FluxOR assay, which are in the lower micromolar range, it must be taken into account that these experiments were performed with an assay buffer containing 2 mM $Ca^{2+}$, which inhibits Slack activity. In such a setting, it is hardly possible to achieve Slack-activating $EC_{50}$ values in the lower nanomolar range. However, these values can still be used to estimate the efficacy of the compounds. As shown in further experiments on the inhibition of scratching behavior in mice after administration of Compound 6 and Compound 10 (compare example 9), the compounds with an EC50 as shown in Table 3333Table 33 sufficiently activate Slack *in vivo.*

The evaluation of analogs in this series of Slack activators in the FluxOR assay suggests that replacement of the chloro substituent at **B** by a trifluoromethyl group improved the activity (Compound 2). Additional introduction of a fluoro substituent at the other aryl ring (**A**) increased the efficacy but reduced the potency (Compound 4). In some cases,

sulfur analogues had comparable activity (Compound 7). Replacement of piperazine by homopiperazine improved the $EC_{50}$ value but decreased the $E_{MAX}$ value (Compound 3). Modification of the alkyl residue at E was generally better tolerated and held potential for tuning of Slack activity and pharmacological properties. However, these conservative substituents did not change the highly lipophilic nature of the original scaffold, which presumably leads to CNS exposure similar to Loxapine. The substitution of the methyl group at the piperazine ring offered the opportunity for substantial increase of polarity. Derivatives with aliphatic hydroxy groups exhibited good efficacy with acceptable $EC_{50}$ values (Compound 5). Finally, an ionizable carboxy group could be incorporated (Compound 6).

**Example 4: Slack activation by the new compounds was verified using whole-cell patch-clamp recordings in HEK-Slack cells.**

Generally, in these experiments, HEK-Slack cells were plated onto poly-D-lysine-coated (100 μg/mL, Sigma Aldrich) coverslips 1 day before experiments and cultured in DMEM containing 10% FBS and 1% penicillin/streptomycin in 5% $CO_2$ at 37 °C. Whole-cell voltage clamp recordings were acquired using an EPC 9 amplifier combined with Patchmaster software (HEKA Electronics, Lambrecht/Pfalz, Germany). Currents were sampled at 20 kHz and filtered at 5 kHz. Data analysis was performed using the Fitmaster software (HEKA Electronics). Membrane potential was held at -70 mV and outward $K^+$ current ($I_K$) was evoked by depolarizing steps (500 ms duration) ranging from -120 to +120 mV in increments of 20 mV. The pipette solution contained 140 mM KCl, 2 mM $MgCl_2$, 5 mM EGTA, 10 mM HEPES, and was adjusted to pH 7.4 with KOH. The extracellular solution contained 140 mM NaCl, 5 mM KCl, 2 mM $CaCl_2$, 2 mM $MgCl_2$, 10 mM HEPES, and was adjusted to pH 7.4 with NaOH. The osmolarity of all solutions was adjusted with glucose to 290-300 mOsmol/L. Patch pipettes had a resistance of 6-8 MΩ and were obtained from borosilicate glass capillaries (Science Products) using a conventional puller (DMZ-Universal Puller, Zeitz Instruments).

After baseline measurements, new compounds or Loxapine solved in external solution containing 0.03% DMSO (each with a final concentration of 50 μM) were added to the bath without a continuous perfusion, and $K^+$ currents were measured within 5 min. Loxapine was used as a positive control whereas vehicle containing 0.03% DMSO served as a negative control. At a holding potential of -70 mV a series of 500 ms-long test pulses ranging from -120 to +120 mV in intervals of 20 mV were applied.

Fold increase values in patch-clamp experiments were determined by calculating baseline current density relative to current density after compound application. These patch-clamp recordings revealed that the new compounds (all except Compound 3) significantly increased the $I_K$ amplitude in comparison to vehicle (**Fig. 3C** and **Fig. 4A**; representative $I_K$ traces are shown in **Fig. 4B**).

At a voltage of +80 mV, current densities of new compounds were increased with a range between 2.04 to 14.77 fold compared to baseline (**Fig. 3C**). Notably, the efficacy of the new compounds in the patch-clamp recordings (i.e., the fold increase of current densities) significantly correlated with their efficacy in the FluxOR assay (**Fig. 3D**). In addition to the results shown in **Fig. 3C** and **Fig. 4A,** the following results were monitored in patch-clamp experiments for Compound 10 that were performed analogously as described before. For a Compound 10 concentration of 25 μM, a fold increase of 7.74 ± 1.525 compared to baseline was monitored, for a Compound 10 concentration of 50 μM, a fold increase of 12.54 ± 1.395 compared to baseline was monitored respectively.

Overall, the FluxOR assay and patch-clamp analyses in HEK-Slack cells indicate that the compounds according to the invention activate Slack *in vitro.*

**Example 5: Microsomal stability assay of Compound 10 in liver microsomes**

For Compound 10, the metabolic stability in liver microsomes as shown in Table 4444Table 44 was determined by the following assay. The test compound was dissolved in DMSO (1 mM). The solubilized test compound (5 μL, final concentration 10 μM) was preincubated at 37 °C in 432 μL of phosphate buffer (0.1 M, pH = 7.4) together with 50 μL of NADPH regenerating system (30 mM glucose-6-phosphate, 4 U/mL glucose 6-phosphate dehydrogenase, 10 mM NADP, 30 mM MgCl2). After 5 min, the reaction was started by the addition of 13 μL of microsome mix from the liver of Sprague-Dawley rats (Invitrogen; 20 mg protein/mL in 0.1 M phosphate buffer) in a shaking water bath at 37 °C. The reaction was stopped by adding 500 μL of ice-cold methanol at 0, 15, 30, and 60 min. The samples were centrifuged at 5000 × g for 5 min at 4 °C, and the test compound was quantified from the supernatants by HPLC: the composition of the mobile phase was adapted to the test compound in a range of MeOH 40-90% and water (0.1% formic acid) 10-60%. Flow rate: 1 mL/min; stationary phase: Purospher STAR, RP18, 5 μm, 125 × 4; precolumn: Purospher STAR, RP18, 5 μm, 4 × 4; detection wavelength: 254 and 280 nm; injection volume: 50 μL. Control samples were performed to check the test compound's stability in the reaction mixture. First control was without NADPH, which is needed for the enzymatic activity of the microsomes, second control was with inactivated microsomes (incubated for 20 min at 90 °C), and third control was without the test compound (to determine the baseline). The amounts of the test compound were quantified by an external calibration curve. Data are expressed as the mean ± SEM remaining compound from three independent

experiments. In vitro half-life was calculated by a logarithmic-linear transformation of the remaining amounts of nonmetabolized test compound versus time.

**Table** 44444: **Metabolic stability of Compound 10**

| Time | |
|---|---|
| 15 min | 55% |
| 30 min | 35% |
| 60 min | 16% |

**Example 6: Blood-Brain-Barrier specific Parallel Artificial Membrane Permeability Assay (PAMPA-BBB).**

BBB permeability was estimated using a BBB-specific parallel artificial membrane permeability assay (PAMPA-BBB). PAMPA-BBB is a non-cell based permeation model that predicts the passive permeability of drug molecules through phospholipid membranes using a porcine polar brain lipid extract.[20]

Following a slightly modified procedure as described by Müller et al.[21], log $P_e$ (effective permeability) values were determined for the prediction of the passive Blood-Brain-Barrier (BBB) permeability. Five v/v% DMSO in PBS (Phosphate Buffer Saline; 0.01 M, pH = 7.4) buffer solutions of each compound were prepared with the final nominal concentration of 100 μM. These solutions were treated with ultrasonic waves and then centrifuged in case the test compounds were insoluble at this concentration. Proceeding with saturated, homogenous solutions ($C_D(0)$) is sufficient because log $P_e$ only depends on relative concentrations.

A BBB specific PAMPA system was used to determine the log $P_e$ and membrane retention (MR). Each well of the top plate (MultiScreen MAIPNTR10; Millipore; Billerica, US) was carefully coated with 5 μL of porcine polar brain lipid extract (PBLE; Avanti Polar Lipids, Birmingham, US) solution, then 150 μL of the $C_D(0)$ solution was put on the membrane. The bottom plate (MultiScreen MDCPN2M50 ; Millipore, Billerica, US) was filled with 2050 μl PBS. PBLE solution consisted of 1 mg PBLE per 10 μL *n*-dodecane and 30 μL *n*-hexane. The donor plate was put on the acceptor plate and covered with a wet paper tissue and a plate lid. The sandwich system was incubated at 37 °C for 4 h. The plates were shaken and incubated in a Thermo Scientific™ MaxQ™ 4000 Benchtop Orbital Shaker (Gel, Belgium). After the incubation, PAMPA sandwich plates were separated and compound concentrations in donor ($C_D(t)$) and acceptor ($C_A(t)$) solutions were determined by UPLC-MS (Waters, Milford, US). The concentration of the donor solution at zero time point (CD(0)) was determined using the supernatant after centrifugation. At iso-pH conditions, the effective permeability and the membrane retention of drugs were calculated by the following equation[22]:

$$P_e = \frac{-2.303}{A\cdot(t-\tau_{SS})}\cdot\frac{(V_A\cdot V_D)}{(V_A+V_D)}\cdot\log\left[1-\left(\frac{V_A+V_D}{(1-MR)\cdot V_D}\right)\left(\frac{C_A(t)}{C_D(0)}\right)\right] \quad (1)$$

where $P_e$ is the effective permeability coefficient (cm/s), A is the filter area (0.3 $cm^2$), $V_D$ and $V_A$ are the volumes in the donor (0.15 $cm^3$) and acceptor phases (2.05 $cm^3$), t is the incubation time (s), $\tau_{SS}$ is the time (s) to reach steady-state (240 s), $C_D(t)$ is the concentration (mol/$cm^3$) of the compound in the donor phase at time t, $C_A(t)$ is the concentration (mol/$cm^3$) of the compound in the acceptor phase at time t, $C_D(0)$ is the concentration (mol/$cm^3$) of the compound in the donor phase at time 0, and MR is the estimated membrane retention factor (the estimated mole fraction of solute lost to the membrane):

$$MR = \left(1-\frac{C_D(t)}{C_D(0)}-\frac{V_A}{V_D}\cdot\frac{C_A(t)}{C_D(0)}\right) \quad (2)$$

As shown in **Fig. 5,** the new compounds demonstrated variable effective BBB permeability (log $P_e$) values and membrane retention factors (MR; indicating the fraction of solute bound to the membrane). Evaluation of the set of compounds in this assay revealed that substitution at the aromatic core, introduction of the carboxylic acid linker and introduction of the hydroxy linker displayed little influence on the permeability. The missing decrease of passive permeability for the zwitterionic carboxylic acid derivatives might result from intramolecular charge neutralization, like it is

known from cetirizine[23]. On the contrary, replacement of piperazine by homopiperazine increased the permeability. Considering that a high MR factor limits the predictive validity of the corresponding $logP_e$ value, the PAMPA-BBB *in vitro* data led the inventors to conclude that compounds Compound 6 and Compound 8 (with both $logP_e < -4.57$ and MR $\leq 0$) as well as Compound 10 (with $logP_e = -4.492$ and MR = 0.02) most likely have a low BBB permeability.

**Table 5555:** Brain permeability of the compounds of the invention according to the PAMPA-BBB model.

| | PAMPA (compare Fig. 5) | |
|---|---|---|
| **Activators** | MR | BBB permeability |
| | | log $P_e$ |
| Loxapine | 0.33 | -4.45 |
| Compound 8 | -0.01 | -4.58 |
| Compound 9 | -0.10 | -4.45 |
| Compound 4 | 0.74 | -4.67 |
| Compound 6 | -0.04 | -4.61 |
| Compound 7 | 0.19 | -4.27 |
| Compound 5 | 0.03 | -4.40 |
| Compound 2 | 0.64 | -4.58 |
| Compound 3 | 0.5 | -4.42 |
| Compound 10 | 0.02 | -4.492 |

**Example 7: Pharmacokinetic properties of new Slack activators by *in vivo* study.**

The compounds pharmacokinetic properties were examined *in vivo* by administering the compounds intraperitoneally (i.p.) at 1 mg/kg in mice, and determining plasma and brain levels over 8 h by LC-MS measurements.

The pharmacokinetic study was performed by Pharmacelsus (Saarbrücken, Germany). In total 27 male C57BL/6 mice (8 weeks old, 23-31 g body weight, purchased from Janvier Labs, France) were used. The animals were housed in a temperature-controlled room (20-24 °C) and maintained in a 12 h light/dark cycle. Food and water were available *ad libitum*. All experimental procedures were approved by and conducted in accordance with the regulations of the local Animal Welfare authorities (Landesamt für Gesundheit und Verbraucherschutz, Abteilung Lebensmittel- und Veterinärwesen, Saarbrücken).

The animals were divided into 3 groups (9 mice / group) and a cassette dosing was performed, in which the mice received a single dose of 1 mg/kg body weight of 3-4 compounds (dissolved in 0.9% NaCl containing 20% cyclodextrine) i.p. simultaneously. From each animal 2 blood samples were collected from the retrobulbar venous plexus under short isoflurane anesthesia (at 0.25 h and 0.5 h from 3 mice; at 1 h and 2 h from 3 mice; and at 4 h and 8 h from 3 mice). Li-heparin was used as anticoagulant. Plasma samples were obtained by centrifugation for 10 min at 3000 × g and 4 °C. Immediately after the last blood sample the animal was sacrificed by cervical dislocation and the brain was removed and frozen in liquid nitrogen. Plasma and brain samples were stored at -80°C until LC-MS analysis.

LC-MS analysis was performed as follows: For Loxapine, Compound 2 and Compound 6, the HPLC system consisted of a Surveyor Pump Plus pump and a Surveyor Plus Auto sampler (Thermo Fisher Scientific, USA), and mass spectrometry was performed on a TSQ Quantum Discovery MAX mass spectrometer equipped with an ESI (electro spray ionization) interface (Thermo Fisher Scientific, USA) in positive SRM mode, connected to a PC running the standard software Xcalibur 2.0.7. For all other compounds, the HPLC system consisted of a U-HPLC pump (Accela) and an AS Open auto sampler (Thermo Fisher Scientific, USA), and mass spectrometry was performed on a Q Exactive (Orbitrap) accurate mass spectrometer equipped with a heated electrospray (H-ESI) interface (Thermo Fisher Scientific, USA) connected to a PC running the standard software Chromeleon 7.2. For all compounds, the HPLC pump flow rate was set to 600 μl/min and the compounds were separated on an analytical column with a suitable pre-column. The pharmacokinetic analysis was performed applying a non-compartment model using the Kinetica 5.0 software (Thermo Scientific, Waltham, USA). All given parameters were obtained by trapezoid area calculation.

All compounds peaked in the plasma within 30 min of injection (Fig. 6A). Peak ($C_{max}$) plasma concentrations ranged from 25.6 to 315.5 ng/ml and the terminal elimination half-life ($t_{1/2z}$) ranged from 1.2 to 3.9 h (Table 6666Table 66). In the brain, highest levels were also measured within 30 min of injection and ranged up to 369 ng/ml (**Fig. 6A**).

The brain/plasma ratios are shown in Fig. 6B. Notably, Compound 6 and compound 10 have a low brain/plasma ratio of ≤ 0.3, suggesting that the brain penetration of these compounds is limited.

**Table 6666: Pharmacokinetic parameters of the new compounds in mice.** Animals were i.p. injected with 1 mg/kg of each compound and plasma and brain concentration at different time points were measured by LC-MS analysis. Presented are pharmacokinetic parameters based on plasma concentration. Time courses of plasma and brain concentration are shown in **Fig. 6A.** The brain/plasma ratio is shown in **Fig. 6B.**

| **Compound No.** | **6** | **9** | **8** | **2** | **4** | **Loxapine** | **7** | **5** | **3** |
|---|---|---|---|---|---|---|---|---|---|
| **Cmax (ng/ml)** | 240.4 | 242.2 | 315.5 | 25.6 | 40.0 | 41.6 | 87.2 | 44.0 | 32.3 |
| **tmax (h)** | 0.25 | 0.25 | 0.25 | 0.25 | 0.5 | 0.25 | 0.25 | 0.25 | 0.5 |
| **Cz (ng/ml)** | 13.3 | 0.3 | 31.4 | 3.3 | 4.1 | 3.2 | 0.3 | 0.4 | 1.0 |
| **tz (h)** | 8 | 8 | 8 | 8 | 8 | 8 | 8 | 8 | 8 |
| **t1/2z (h)** | 2.4 | 1.8 | 3.9 | 3.4 | 2.3 | 2.3 | 1.6 | 1.2 | 2.0 |
| **AUC(0-tz) (ng*h/ml)** | 419.7 | 122.6 | 675.1 | 67.8 | 108.2 | 105.2 | 71.2 | 49.6 | 55.4 |
| **AUC (0-inf) (ng*h/ml)** | 466.7 | 123.4 | 851.0 | 84.0 | 122.0 | 115.9 | 71.9 | 50.2 | 58.3 |
| **%AUCextra** | 10.1 | 0.6 | 20.7 | 19.3 | 11.4 | 9.2 | 0.9 | 1.3 | 5.0 |
| **Vz/f (ml/kg)** | 7,560 | 21,080 | 6,582 | 58,489 | 27,724 | 28,837 | 31,440 | 33,111 | 50,117 |
| **cuf (ml/ (h*kg))** | 2,143 | 8,107 | 1,175 | 11,911 | 8,195 | 8,630 | 13,915 | 19,909 | 17,158 |

**Example 8: Off target profile of Compound 6**

As Loxapine is a first-generation antipsychotic with substantial binding affinity to many receptors[24] the inventors examined the pharmacological profile of Compound 6 and Loxapine (both 10 μM) *in vitro* using a SafetyScreen44 panel (Eurofins) that screens the interaction of compounds with 44 targets.

In this panel, Loxapine (10 μM) showed substantial binding inhibition (higher than 50%) for 17 out of 44 targets including adrenergic ($\alpha_{1A}$ and $\alpha_{2A}$), dopamine ($D_1$ and $D_{2S}$), histamine ($H_1$ and $H_2$), muscarine ($M_1$, $M_2$ and $M_3$), serotonin (5-$HT_{1A}$, 5-$HT_{1B}$, 5-$HT_{2A}$, 5-$HT_{2B}$ and 5-$HT_3$) receptors, $Na^+$ channel, norepinephrine transporter (NET), and serotonin transporter (SET) (**Fig. 7),** thereby confirming the 'dirty' nature of first generation antipsychotics. Of note, considerably less off-target activities were observed with Compound 6 (10 μM), which exhibited substantial binding to 8 targets ($\alpha_{1A}$, $D_1$, $D_{2S}$, $H_1$, 5-$HT_{2A}$, 5-$HT_{2B}$, NET, and SET), and with Compound 10 (10 μM), which showed substantial binding inhibition for 5 targets ($CB_1$, $D_1$, $H_1$, 5-$HT_{2A}$, and 5-$HT_{2B}$). Considering that the majority of the targets affected by Compound 6 or Compound 10 are mainly expressed in the CNS (in particular, $D_1$, $D_{2S}$, 5-$HT_{2B}$, NET, and SET[25,21]; www.proteinatlas.org), and that both compounds show only limited brain penetration, these data suggest an improved pharmacological profile of Compound 6 and Compound 10 as compared to Loxapine. Furthermore, Compound 6 and Compound 10 (like Loxapine) did not measurably bind to human ether-à-go-go (hERG) or voltage-gated ($K_V$) potassium channels in the off-target screen (**Fig. 7**), indicating that this compound does not act as an unspecific modulator of potassium channels.

**Example 9: Compound 6 and Compound 10 profoundly inhibit itch-related behavior in mice**

Further studies were conducted to assess the *in vivo* efficacy of Compound 6 and Compound 10 in mice. In detail, experiments were performed in 8 to 16 weeks old C57BL/6N mice (Charles River, Sulzfeld, Germany) and animals with a genetic deletion of Slack ($Slack^{-/-}$mice[27]) of either sex. Animals were housed on a 12 h light/dark cycle with access to food and water ad libitum. All experiments adhered to the guidelines of the International Association for the Study of Pain and to the ARRIVE (Animal Research: Reporting on In Vivo Experiments) guidelines, and were approved by and conducted in accordance with the regulations of the local Animal Welfare authorities (Regierungspräsidium Darmstadt, Germany). All behavioral studies were conducted during the light cycle of the day at room temperature (20-24 °C) by

an observer blinded for the treatment of the animals and/or their genotype.

Accelerating rotarod and vertical pole test were performed as follows: Mice were placed on a rotarod treadmill (Ugo Basile, Italy) with increasing speed (4-40 rpm over 300 s), and were trained for 4-5 consecutive days. Only mice reaching the 300 seconds without falling off were included in the experiment. On the testing day, compounds were i.p. administered in a vehicle composed of 0.9% saline (Sigma-Aldrich) containing 10% 2-hydroxypropyl-β-cyclodextrine (ITW Reagents) and after 15 min the latency to fall off the accelerating rotarod was measured. Immediately thereafter, mice were placed head-upward on the top of a vertical pole with a rough surface (diameter 1 cm, height 40 cm) and the time until animals reached the ground was recorded (cut-off time 20 s). Means out of three trials for each time point were calculated for further analysis.

Treatments with Compound 6 and Compound 10 (3-30 mg i.p.) did neither impair the motor function in the accelerating rotarod test, a standard model of motor performance (Fig. 8A), nor in the vertical pole test that assesses basal ganglia related movement disorders (Fig. 8B). By contrast, Loxapine at a dose $\geq$ 0.39 mg/kg significantly reduced the ability of the mice to perform in both models (**Fig. 8A,B**), which is in line with its high CNS penetration and confirms earlier reports[28].

Acute itch behavior of the mice was monitored using a histamine-based, chloroquine-based and SLIGRL-based acute itch behavior model. In these models, three to four days before the experiment day, the fur on the dorsolateral aspect of the neck was shaved under brief isoflurane anesthesia. On the testing day, mice were habituated for 30 min in separate plexiglass cylinders (30 cm diameter). Compounds were dissolved in 0.9% saline containing 10% 2-hydroxypropyl-β-cyclodextrine and i.p. administered in a volume of maximal 200 μL. 15 min thereafter, the pruritogens chloroquine (200 μg), SLIGRL (100 μg) or histamine (800 μg), all dissolved in 0,9% saline (20 μL), were injected subcutaneously into the nape of the neck. Numbers of scratching bouts directed to the nape of the neck were assessed over 30 min by videotaping.

Antipruritic efficacy of Compound 6 and Compound 10 in a model of histamine-independent itch evoked by the antimalarial drug chloroquine: Importantly, a systemic treatment of the mice with Compound 6 (3, 10 or 30 mg/kg i.p.) or Compound 10 (3, 10 or 30 mg/kg i.p.) 15 min prior to s.c. injection of chloroquine into the nape of the neck was found to ameliorate the scratching behavior in a dose-dependent manner (**Fig. 8C**) in the histamine-independent itch-model, where itch was evoked by the antimalarial drug chloroquine. Further experiments supported the conclusion that the antipruritic effects were mediated by Slack activation, because Compound 6 (10 mg/kg) did not significantly alter chloroquine-induced scratching in animals with a genetic deletion of Slack ($Slack^{-/-}$ mice; **Fig. 8D**). The assumption that Slack is the primary receptor mediating activity *in vivo* is supported by the observation that action of Compound 6 was much reduced in Slack-/- mice and blocked by Slack inhibitor Compound 31 (**Fig. 8H**).

Together, supported by genetic and pharmacological evidence these data suggest that Compound 6 or Compound 10 treatment ameliorates chloroquine-induced itch by activation of Slack.

Antipruritic efficacy of Compound 6 in histamine-independent itch evoked by SLIGRL injection: The chloroquine-induced scratching in mice is driven by activation of MrgprA3 in the NP2 population of sensory neurons[4,29]. To further explore the antipruritic efficacy of Compound 6 in histamine-independent itch, the inventors assessed the behavioral response after s.c. injection of the peptide Ser-Leu-Ile-Gly-Arg-Leu (SLIGRL) that evokes scratching by activation of MrgprC11 (ref[30]), which is expressed in the NP2 and NP3 population[4]. Similar to the chloroquine model, Compound 6 (10 mg/kg i.p.) significantly inhibited the SLIGRL-induced scratching behavior (**Fig. 8E**). By contrast, scratching evoked by s.c. histamine was not affected by Compound 6 (**Fig. 8F**), pointing to a limited function of Slack in the processing of histamine-dependent itch. Moreover, this finding provides further evidence that the antipruritic effects of Compound 6 observed in histamine-independent models are not the result of motor impairment.

In these studies, no significant behavioral differences were observed between sexes (**Fig. 9 and Fig. 11).**

Furthermore, the effect of Compound 6 in chronic itch was examined in a model of allergic contact dermatitis[31]. In brief, the fur on the dorsolateral aspect of the neck was shaved under brief isoflurane anesthesia. Three to four days thereafter, the shaved skin area was painted with 100 μL of 0.15% 2,4-dinitrofluorobenzene (DNFB) in acetone/olive oil (3:1). After 10-11 days, the skin was shaved again and the DNFB solution was painted 3-4 days thereafter (i.e., two painting sessions 14 days apart). Ninety minutes after the second painting, mice were habituated for 15 min in separate plexiglass cylinders (30 cm diameter).

This treatment resulted in persistent itch behavior reflected by scratching the painted area and head-shaking that was significantly correlated with scratching.

Compounds or vehicle (10% 2-hydroxypropyl-β-cyclodextrine in 0.9% saline) were i.p. administered, and 15 min thereafter spontaneous scratching was recorded over 30 min by videotaping **(Fig. 10A).** I.p. treatment with Compound 6 after the second DNFB exposure significantly inhibited the number of scratching bouts and the number of head shakes as compared to vehicle-treated animals **(Fig. 10B and 10C).**

Similarly, the effect of Compound 6 was also examined in the MC903 model of skin inflammation, which has some characteristics of allergic contact dermatitis and atopic dermatitis[32].

The fur on the dorsolateral aspect of the neck was shaved under brief isoflurane anesthesia. Five days thereafter,

20 μL MC903 (0.2 mM; Calcipotriol, Tocris) solved in absolute ethanol was applied to the skin at the back of the neck in brief anesthesia for 7 consecutive days. On day 8, mice were habituated for 30 min in separate plexiglass cylinders (30 cm diameter). Compounds or vehicle (10% 2-hydroxypropyl-β-cyclodextrine in 0.9% saline) were i.p. administered, and 15 min thereafter spontaneous scratching was recorded over 30 min by videotaping **(Fig. 10D).** In these studies, i.p. Compound 6 significantly ameliorated both scratching and head shaking **(Fig. 10E and 10F).** These findings are important, because they indicate that Slack activators can ameliorate pruritus that has already established.

Finally, potential side effects of Compound 6 were assessed by measuring cardiovascular functions by pulse oximetry. A pulse oximeter (MouseOX Plus, Starr Life Sciences Corp) was used on conscious, freely moving mice. After 3 consecutive days of habituating the mice to the collar clip for at least 30 min, baseline measurements were performed. Then compounds were i.p. administered and after a resting time of 5 min the heart rate, respiratory rate and arterial $O_2$ saturation was recorded over 30 min. All datasets were sampled at 1 Hz, error-corrected according to manufacturer's instructions and averaged over 300 s for each time point.

In these pulse oximetry measurements on conscious, freely moving mice i.p. administration of Compound 6 (10 mg/kg) did neither affect the heart rate (**Fig. 12A**) nor the breath rate (**Fig. 12B**) during an observation period of 30 min. By contrast, both parameters were significantly reduced after i.p. administration of morphine (10 mg/kg), which was used as a positive control in this experiment.

Altogether, these results highlight that Compound 6 effectively inhibits the itch behavior of mice in multiple models at a well-tolerated dose.

**Example 9: Compound 6 inhibits itch-sensitive sensory neurons**

To determine if the antipruritic effect of Compound 6 occurs directly at the neuronal level, whole-cell patch-clamp electrophysiology was used to measure excitability of DRG neurons.

A DRG neuron primary cell culture was prepared and stimulated with an inflammatory soup overnight to induce hyperexcitability. In brief, naive C57BL/6N mice (age 4-8 weeks) were killed by $CO_2$ inhalation and lumbar (L1 - L5) DRGs were transferred to hanks' balanced salt solution (Gibco, Thermo Fisher Scientific). After incubation with 500 U/mL collagenase IV and 2.5 U/mL dispase II (both from Sigma Aldrich) for 60 min, including carefully shaking every 20 min, DRGs were washed and gently triturated twice with a fire-polished Pasteur pipette in neurobasal medium (Gibco, Thermo Fisher Scientific) including 10% FBS and 0.5 mM GlutMax (Gibco, Thermo Fisher Scientific). Dissociated DRGs were seeded onto poly-D-lysine-coated (100 μg/mL) coverslips and cultured in neurobasal medium supplemented with 2% B27 (Gibco, Thermo Fisher Scientific), 1% penicillin/streptomycin and 0.5 mM GlutMax in 5% $CO_2$ at 37 °C.

In order to simulate a pathological state, DRG neuronal cultures were incubated with an inflammatory soup (histamine: 10 μM), $PGE_2$: 10 μM), serotonin: 10 μM, bradykinin: 10 μM)[33] overnight and performed recordings on cells that bind isolectin B4, a marker of nonpeptidergic C-fiber neurons including the itch-sensitive neuronal populations (NP1-NP3)[4] in mice. Before recording, DRG neurons were preincubated with 10 μg/mL FITC-conjugated IB4 (Sigma-Aldrich) for 5 - 10 min to select for Slack-expressing neurons.

Whole-cell current clamp recordings were obtained using an EPC 9 amplifier combined with Patchmaster software (HEKA Electronics, Lambrecht/Pfalz, Germany). General settings, pipette and extracellular solutions were used as described above. Evoked action potentials (AP) were elicited by 10 ms current injections starting at 0 pA in 20 pA increments to determine electrophysiological parameters. Action potential (AP) firing was induced by depolarizing current pulses (200 - 950 pA at 150 pA intervals, 1000 ms duration). Recordings were performed at baseline and following a 1 min incubation with 50 μM Compound 6 or vehicle (external solution containing 0.03% DMSO). Number of APs before and after compound application were counted at the same current injection step.

DRG neurons from the above-described cultures displayed pronounced hyperexcitability, as indicated by spontaneous action potential (AP) firing after injecting currents (200-950 pA; **Fig. 13A**). Notably, application of Compound 6 (50 μM) caused a profound decrease in the number of AP fired (**Fig. 13B**). Furthermore, after injection of small currents (0-220 pA), the rheobase (i.e., the amount of current required to generate an AP) was significantly increased in presence of Compound 6 (**Fig. 13D**) and the AP amplitude was significantly lower (**Fig. 13E**). These findings confirm that Compound 6 inhibits itch-sensitive DRG neurons in mice.

**Example 10: Chemicals/Drugs and Cell Cultures**

Chemicals/Drugs: Loxapine, pregabalin, chloroquine, histamine, $PGE_2$, serotonin and bradykinin were purchased from Sigma-Aldrich. All indicated concentrations refer to pure substances. Reagents and solvents for the synthesis of Loxapine derivatives were obtained from Acros Organics (Gel, Belgium), Alfa Aesar GmbH & Co KG (Karlsruhe, Germany), BLDPharm Inc. (NuiNan, China), Fluorochem Ltd. (Hadfield, UK), Sigma-Aldrich (Munich, Germany), and TCI Europe N.V. (Zwijndrecht, Belgium).

Cell cultures: HEK293 cells stably transfected with human Kcnt1 (herein referred to as HEK-Slack cells; SB-HEK-KCa4.1; SB Drug Discovery, Lanarkshire, UK) were maintained in Dulbecco's modified Eagle's medium-Glutamax with 10% fetal calf serum and 1% penicillin/streptomycin, supplemented with 0.6 mg/mL G-418 (all from Gibco/Thermo Fisher Scientific) in 5% $CO_2$ at 37 °C. Cells were passaged every 4 to 5 days from P11 to P35 depending on confluence.

Statistical analysis. Statistical analysis was performed in Prism 9 (GraphPad). Normally distributed data are expressed as the mean ± standard error of the mean (SEM) and nonparametric data are presented as median and interquartile range. To identify and remove potential outliers the ROUT method with a Q (maximum desired false discovery rate) of 1% was used. The statistical test and number of replicates for each analysis is indicated in the figure legends or the main text. For all tests, a probability value P < 0.05 was considered as statistically significant. No statistical methods were used to predetermine sample sizes.

REFERENCES

The references are:

[1] Carstens, E., Follansbee, T. & Iodi Carstens, M. The Challenge of Basic Itch Research. Acta Derm. Venereol. 100, adv00023 (2020).
[2] Yosipovitch, G., Rosen, J.D. & Hashimoto, T. Itch: From mechanism to (novel) therapeutic approaches. J. Allergy Clin. Immunol. 142, 1375-1390 (2018).
[3] Chen, X.J. & Sun, Y.G. Central circuit mechanisms of itch. Nat. Commun. 11, 3052 (2020).
[4] Usoskin, D., et al. Unbiased classification of sensory neuron types by large-scale single-cell RNA sequencing. Nat. Neurosci. 18, 145-153 (2015).
[5] Meixiong, J. & Dong, X. Mas-Related G Protein-Coupled Receptors and the Biology of Itch Sensation. Annu. Rev. Genet. 51, 103-121 (2017).
[6] Tsantoulas, C. & McMahon, S.B. Opening paths to novel analgesics: the role of potassium channels in chronic pain. Trends Neurosci. 37, 146-158 (2014).
[7] Yuan, A., et al. The sodium-activated potassium channel is encoded by a member of the Slo gene family. Neuron 37, 765-773 (2003).
[8] Budelli, G., et al. SLO2 Channels Are Inhibited by All Divalent Cations That Activate SLO1 K+ Channels. J. Biol. Chem. 291, 7347-7356 (2016).
[9] Sharma, N., et al. The emergence of transcriptional identity in somatosensory neurons. Nature 577, 392-398 (2020).
[10] Martinez-Espinosa, P.L., et al. Knockout of Slo2.2 enhances itch, abolishes KNa current, and increases action potential firing frequency in DRG neurons. Elife 4 (2015).
[11] Kupari, J., et al. Single cell transcriptomics of primate sensory neurons identifies cell types associated with chronic pain. Nat. Commun. 12, 1510 (2021).
[12] Tavares-Ferreira, D., et al. Spatial transcriptomics of dorsal root ganglia identifies molecular signatures of human nociceptors. Sci. Transl. Med. 14, eabj8186 (2022).
[13] Biton, B., et al. The antipsychotic drug Loxapine is an opener of the Na+-activated potassium channel Slack (slo2.2). J. Pharmacol. Exp. Ther. 340, 706-715 (2011).
[14] Chakrabarti, A., et al. Loxapine for schizophrenia. Cochrane Database Syst. Rev., CD001943 (2007).
[15] Schmiedl, S., et al. Loxapine for Treatment of Patients With Refractory, Chemotherapy-Induced Neuropathic Pain: A Prematurely Terminated Pilot Study Showing Efficacy But Limited Tolerability. Front Pharmacol 10, 838 (2019).
[16] Tavares-Ferreira, D., et al. Spatial transcriptomics of dorsal root ganglia identifies molecular signatures of human nociceptors. Sci. Transl. Med. 14, eabj8186 (2022).
[17] Kupari, J., et al. Single cell transcriptomics of primate sensory neurons identifies cell types associated with chronic pain. Nat. Commun. 12, 1510 (2021).
[18] Griffin, A.M., et al. Discovery of the First Orally Available, Selective KNa1.1 Inhibitor: In Vitro and In Vivo Activity of an Oxadiazole Series. ACS Med Chem Lett 12, 593-602 (2021).
[19] Lu, R., et al. Slack channels expressed in sensory neurons control neuropathic pain in mice. J. Neurosci. 35, 1125-1135 (2015).
[20] Avdeef, A. The rise of PAMPA. Expert Opin. Drug Metab. Toxicol. 1, 325-342 (2005).
[21] Muller, J., Esso, K., Dargo, G., Konczol, A. & Balogh, G.T. Tuning the predictive capacity of the PAMPA-BBB model. Eur. J. Pharm. Sci. 79, 53-60 (2015).
[22] Avdeef, A. Permeability - PAMPA. in Absorption and Drug Development: Solubility, Permeability, and Charge State, Second Edition 319-498 (Wiley, 2012).
[23] Pagliara, A., et al. Molecular properties and pharmacokinetic behavior of cetirizine, a zwitterionic H1-receptor antagonist. J. Med. Chem. 41, 853-863 (1998).
[24] Popovic, D., Nuss, P. & Vieta, E. Revisiting Loxapine: a systematic review. Ann Gen Psychiatry 14, 15 (2015).

25 Sjostedt, E., et al. An atlas of the protein-coding genes in the human, pig, and mouse brain. Science 367 (2020).
26 Karlsson, M., et al. A single-cell type transcriptomics map of human tissues. Sci Adv 7 (2021).
27 Lu, R., et al. Slack channels expressed in sensory neurons control neuropathic pain in mice. J. Neurosci. 35, 1125-1135 (2015).
28 Popovic, D., Nuss, P. & Vieta, E. Revisiting Loxapine: a systematic review. Ann Gen Psychiatry 14, 15 (2015).
29 Liu, Q., et al. Sensory neuron-specific GPCR Mrgprs are itch receptors mediating chloroquine-induced pruritus. Cell 139, 1353-1365 (2009).
30 Liu, Q., et al. The distinct roles of two GPCRs, MrgprC11 and PAR2, in itch and hyperalgesia. Sci. Signal. 4, ra45 (2011).
31 Kitamura, A., Takata, R., Aizawa, S., Watanabe, H. & Wada, T. A murine model of atopic dermatitis can be generated by painting the dorsal skin with hapten twice 14 days apart. Sci Rep 8, 5988 (2018).
32 Li, M., et al. Topical vitamin D3 and low-calcemic analogs induce thymic stromal lymphopoietin in mouse keratinocytes and trigger an atopic dermatitis. Proc. Natl. Acad. Sci. U. S. A. 103, 11736-11741 (2006).
33 Grundy, L., et al. Chronic linaclotide treatment reduces colitis-induced neuroplasticity and reverses persistent bladder dysfunction. JCI Insight 3, e121841 (2018).

## Claims

1. **A compound,** wherein the compound has the formula I:

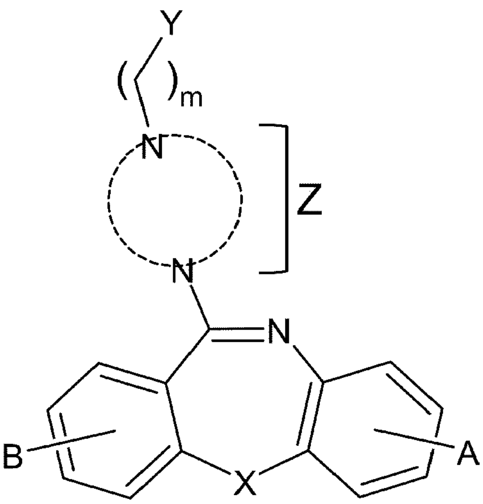

(I),

or a salt, complex, diastereomer, enantiomer and/or tautomer of thereof,
wherein

**A** is one or two substituents independently selected from -H, -F, -Cl, -Br, -I, $-CF_3$, $-OCF_3$, $-CF_2H$, and $-OCF_2H$;
**B** is one or two substituents independently selected from -F, -Cl, -Br, -I, $-CF_3$, $-OCF_3$, $-CF_2H$, and $-OCF_2H$;
**X** is selected from -S- and -O-;
**m** is 0 to 3;
**Y** is selected from

-H;
a nitrile group,
a 5- to 6-membered unsaturated heterocycle comprising 3 hetero atoms that are selected from O and N, preferably wherein 2 or 3 heteroatoms are nitrogen, wherein the heterocycle is mono or di-substituted with =S or =O, preferably with =O; or
a linear $-C_{1-3}$alkyl, wherein a, preferably one, $-(CH_2)-$ of the linear $-C_{1-3}$alkyl is optionally monosubstituted with -OH, and wherein the terminal $-CH_3$ of the linear - $C_{1-3}$alkyl is monosubstituted with -OR' or $-SR^1$, preferably $-OR^1$, wherein $R^1$ is H, $-(CH_2)-COOH$ or $-(CH_2)-(CH_2)-OH$; or
$-(CH_2)_i-CX^1R^2$, wherein

$X^1$ is $=NR^3$ or $=O$, wherein $R^3$ is -H or -OH.
i is 0 or 1, and
$R^2$ is $-OR^4$ or $-NR^5R^6$, wherein $R^4$ is -H or $-C_{1-2}$alkyl; wherein $R^5$ and $R^6$ are independently selected from -H or -methyl;

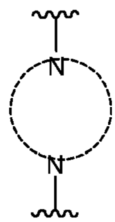
,

herein denoted as "Z", is an aliphatic group comprising 1 or 2 heterocycles which comprise together at least two nitrogen atoms,
wherein the first of the two nitrogen atoms connects Z to

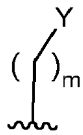

of formula I and the
second of the two nitrogen atom connects Z to

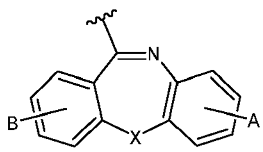

of formula I,
preferably to

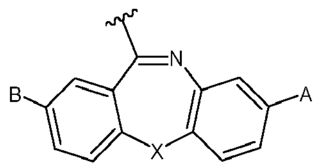
,

and wherein

- the two nitrogen atoms are each a heteroatom in one of two connected 4-membered heterocycles (each heterocycle having no more than one heteroatom), wherein the two interconnected 4-membered heterocycles form a spirocylce, preferably wherein the 4-membered heterocycle with the first nitrogen atom is connected via a shared carbon atom to the 4-membered heterocycle with the second nitrogen atom, thus forming a spirocycle with two heteroatoms that are the two nitrogen atoms; or
- the first of the two nitrogen atoms is connected to a 4-membered heterocycle with one heteroatom, wherein the second of the two nitrogen atoms is the heteroatom in the 4-membered heterocycle that the first of the two nitrogen atoms is connected to; wherein the first nitrogen atom is further substituted with methyl; or the two nitrogen atoms are heteroatoms in a 6- to 7-membered heterocycle with two heteroatoms, wherein the heterocycle is optionally substituted, preferably monosubstituted, with methyl;

tituted, preferably monosubstituted, w

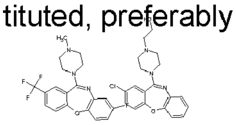

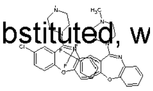

and wherein the compound is not any of,

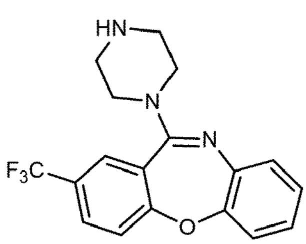

, and

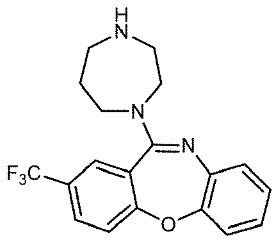

.

**2.** The compound according to claim 1, wherein B is a substituent at the carbon positions 2 and 3, preferably B is the substituent -Cl at the carbon positions 2 and 3, when referring to the following numbering

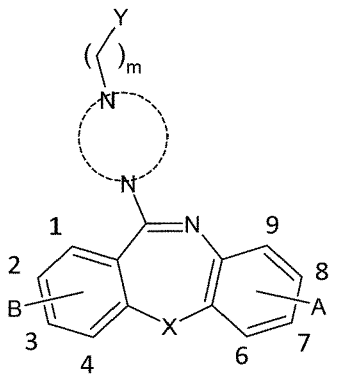

**3.** The compound according to claim 1, wherein A and B are each one substituent, preferably wherein the compound has the formula II

(II).

**4.** The compound of claim 1 to 3, wherein

(i) A is selected from -H or -F.
(ii) B is selected from -Cl or $-CF_3$.
(iii) m is 0 to 2, more preferably m is 0 or 1, most preferably m is 1.
(iv) Y is selected from the group consisting of

| | | | |
|---|---|---|---|
| , | , | , | , |
| -H, | , | , | , |
| , | or | ; | |

Z is selected from the group consisting of

| | | |
|---|---|---|
| 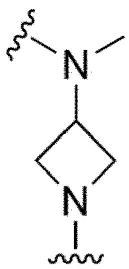 , | 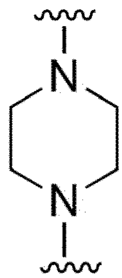 , | , |

| | | |
|---|---|---|
| , | 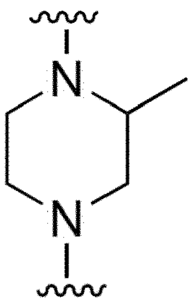 , or | 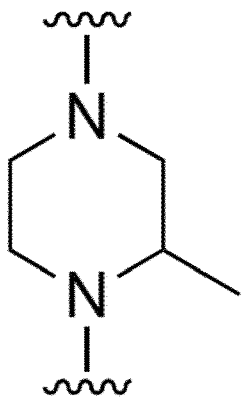 , |

5. The compound according to any one of claims 1 to 4, wherein the compound is selected from the group consisting of compounds 2 to 14,

| Compound No. | Structure |
|---|---|
| 6 | , |
| 8 | , |
| 9 | 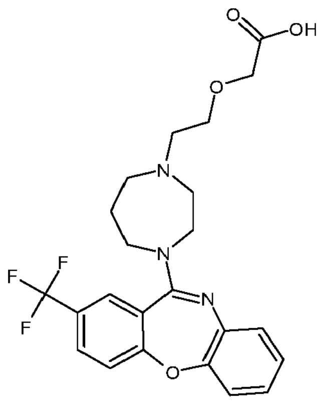 , |
| 10 | 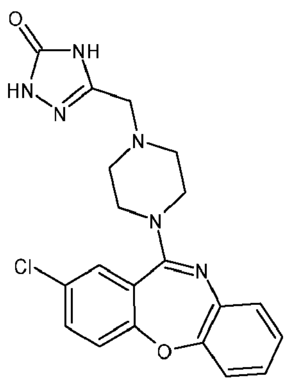 , |

(continued)

| Compound No. | Structure |
|---|---|
| 11 | 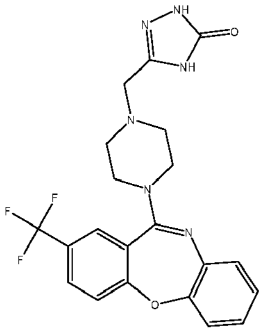 , |
| 12 | 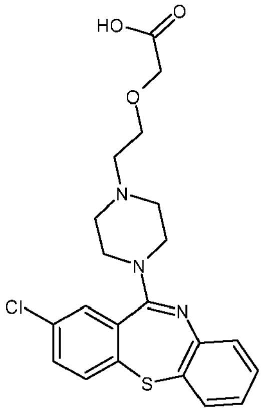 |
| 15 | 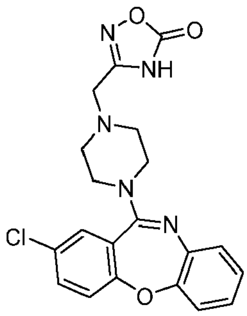 |
| 16 | 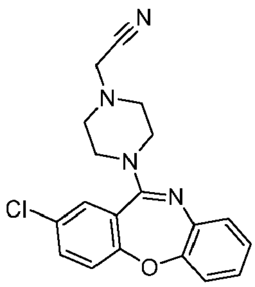 |

(continued)

| Compound No. | Structure |
|---|---|
| 17 | 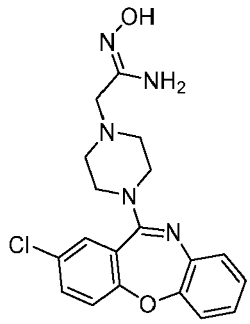 |
| 18 | 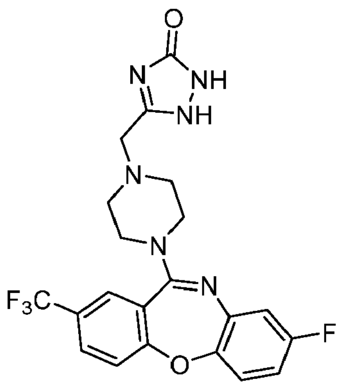 |
| 19 | 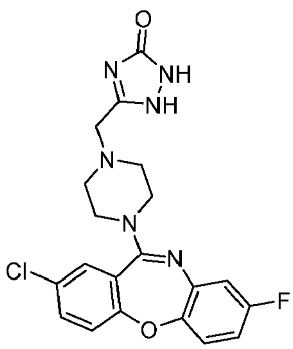 |
| 20 | 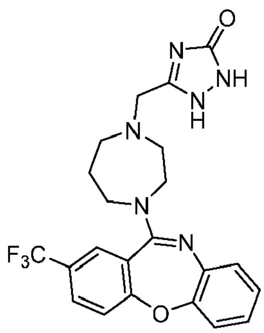 |

(continued)

| Compound No. | Structure |
|---|---|
| 21 | 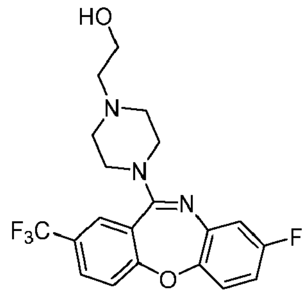 |

preferably wherein compound is selected from the group consisting of compounds 6, 8, 9, 10, 11, 12, 18 ,19, 20, 21, more preferably the compound is selected from the group consisting of the compounds 6, 8, 9, and 10.

6. The compound of claim 1 to 5, wherein the compound has an activity as an activator of a potassium channel in a cell, preferably of a potassium channel Slack ($K_{Na}1.1$), preferably wherein the compound activates the potassium channel with an EC50 of less than 100μM, preferable of between 0.5 and 80μM.

7. The compound of any one of claims 1 to 6, wherein the compound when administered to an animal does not penetrate the blood brain barrier (BBB), or wherein the compound penetrates the BBB less than a reference compound, such as Loxapine.
does not bind the human dopamine receptor, more preferably wherein the compound binds the human dopamine receptor less compared to a reference compound, such as Loxapine.

8. The compound of any one of claims 1 to 7, wherein the compound is isolated and/or wherein the compound has a purity of at least 75%, optionally at least 90%, optionally at least 95%.

9. **A pharmaceutical composition** comprising the compound of any one of claims 1 to 8, or a salt, solvate, or ester thereof, and a pharmaceutically acceptable carrier or excipient.

10. The pharmaceutical composition of claim 9, wherein the compound, or a salt, solvate, or ester thereof, is present in an amount of 0.1% w/w to 10% w/w, optionally in an amount of 1% w/w to 10% w/w.

11. A **compound or composition for use in the treatment of a condition** in a subject, the compound or composition comprising an effective amount of the compound of any one of claims 1 to 8, wherein the compound has the formula I:

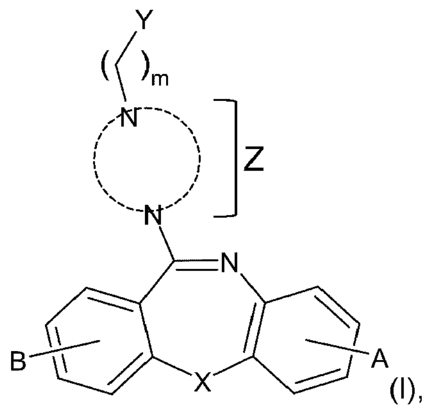

(I),

or a salt, complex, diastereomer, enantiomer and/or tautomer of thereof, wherein

A is one or two substituents independently selected from -H, -F, -Cl, -Br, -I, $-CF_3$, $-OCF_3$, $-CF_2H$, and $-OCF_2H$;

**B** is one or two substituents independently selected from -F, -Cl, -Br, -I, $-CF_3$, $-OCF_3$, $-CF_2H$, and $-OCF_2H$;
**X** is selected from -S- and -O-;
m is 0 to 3;
**Y** is selected from

-H;
a nitrile group,
a 5- to 6-membered unsaturated heterocycle comprising 3 hetero atoms that are selected from O and N, preferably wherein 2 or 3 heteroatoms are nitrogen, wherein the heterocycle is mono or di-substituted with =S or =O, preferably with =O; or
a linear $-C_{1-3}$alkyl, wherein a, preferably one, $-(CH_2)-$ of the linear $-C_{1-3}$alkyl is optionally monosubstituted with -OH, and wherein the terminal $-CH_3$ of the linear - $C_{1-3}$alkyl is monosubstituted with -OR’ or $-SR^1$, preferably $-OR^1$, wherein $R^1$ is H, $-(CH_2)-COOH$ or $-(CH_2)-(CH_2)-OH$; or
$-(CH_2)_i-CX^1R^2$, wherein

$X^1$ is $=NR^3$ or =O, wherein $R^3$ is -H or -OH.
i is 0 or 1, and
$R^2$ is $-OR^4$ or $-NR^5R^6$, wherein $R^4$ is -H or $-C_{1-2}$alkyl; wherein $R^5$ and $R^6$ are independently selected from -H or -methyl;

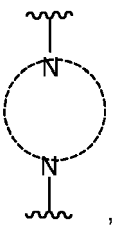
,

herein denoted as "Z", is an aliphatic group comprising 1 or 2 heterocycles which comprise together at least two nitrogen atoms,
wherein the first of the two nitrogen atoms connects Z to

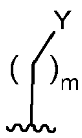

of formula I and the second of the two nitrogen atom connects Z to

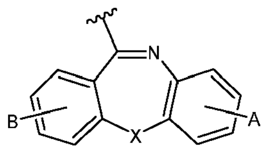

of formula I, preferably to

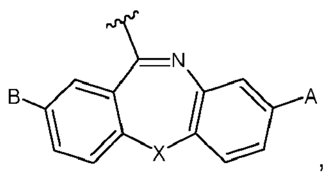
,

and wherein

the two nitrogen atoms are each a heteroatom in one of two connected 4-membered heterocycles (each heterocycle having no more than one heteroatom), wherein the two interconnected 4-membered heterocycles form a spirocylce, preferably wherein the 4-membered heterocycle with the first nitrogen atom is connected via a shared carbon atom to the 4-membered heterocycle with the second nitrogen atom, thus forming a spirocycle with two heteroatoms that are the two nitrogen atoms; or
the first of the two nitrogen atoms is connected to a 4-membered heterocycle with one heteroatom, wherein the second of the two nitrogen atoms is the heteroatom in the 4-membered heterocycle that the first of the two nitrogen atoms is connected to; wherein the first nitrogen atom is further substituted with methyl; or the two nitrogen atoms are heteroatoms in a 6- to 7-membered heterocycle with two heteroatoms, wherein the heterocycle is optionally substituted, preferably monosubstituted, with methyl;

preferably the compound of any one of claims 1 to 8, or a salt, solvate, or ester thereof, preferably, preferably wherein the composition is the pharmaceutical composition of claims 9 or 10, wherein the condition is treatable by activating a potassium channel in a cell associated with the pathology of the condition, the treatment comprising administering the compound or composition to the subject in need thereof.

**12.** The compound or composition for use of claims 11, wherein the condition is a pruritic condition, such as an acute or chronic pruritus, optionally wherein the pruritic condition is associated with a second pathology, such as one selected from:

- a dermatological disorder such as xerosis or xeroderma (dry skin), dermatitis or eczema (e.g., atopic dermatitis), psoriasis (e.g., plaque psoriasis), prurigo (e.g., prurigo nodularis), urticaria (e.g., chronic idiopathic urticaria), a connective tissue disorder (e.g., dermatomyositis), post-burn pruritus;
- a kidney disorder (e.g., chronic kidney disease, chronic kidney failure or end-stage renal disease), dialysis (e.g., hemodialysis), uremic pruritus;
- a hepato-biliary disorder (e.g., cholestasis, primary biliary cholangitis, primary sclerosing cholangitis, secondary sclerosing cholangitis, hepatitis, toxic liver disease, chronic liver disease or cirrhosis), cholestatic pruritus;
- an endocrine disorder (e.g., hyperthyroidism or diabetes mellitus);
- a metabolic disorder (e.g., iron deficiency or iron overload);
- a benign or malignant neoplasm (e.g., a solid tumor, a carcinoma or a hematological neoplasm [e.g., Hodgkin's lymphoma, non-Hodgkin's lymphoma, myeloproliferative disease, or polycythemia vera]);
- an infectious disease (e.g., a viral infection such as an infection with herpes simplex, herpes zoster, varicella, human immunodeficiency virus (HIV), hepatitis; a bacterial infection or a parasitosis);
- a neurological disorder (e.g., a degenerative neurological disease, multiple sclerosis, a brain tumor, postherpetic neuralgia, small-fiber neuropathies, brachioradial pruritus or notalgia paresthetica), neuropathic itch, neurogenic itch;
- a psychiatric disease (e.g., depression, obsessive compulsive disorder, delusional disorder, eating disorder, or anxiety);
- drug-induced pruritus (e.g., by opioids, antibiotics, antimalarial agents, ACE inhibitors, angiotensin receptor antagonists, anti-arrhythmic agents, antidepressants, antidiabetic drugs, antihypertensive drugs, anticonvulsants, anti-inflammatory drugs, betablockers, bronchodilators, calcium antagonists, diuretics, hormones, immunosuppressive drugs, antilipids, neuroleptics, plasma expanders, tranquillizers, or uricostatics);
- pruritus in the elderly;
- pruritus in pregnancy; and/or
- chronic idiopathic pruritus.

**13.** The compound or composition for use of claim 11 or 12, wherein the condition is pain or a pain related noxious sensation in the subject, preferably wherein the pain is selected from

- neuropathic pain induced by traumatic nerve injury, cancer and cancer treatments (e.g., chemotherapy), neurological conditions (e.g., multiple sclerosis), neurodegenerative conditions (e.g., Parkinson's disease), trigeminal neuralgia, diabetic peripheral neuropathy, stroke, shingles, HIV, Hansen's disease (leprosy), Guillain-Barre syndrome, blood vessel disease, vascular malformations, autoimmune conditions
- acute post-operative pain,
- inflammatory pain, rheumatoid arthritis, osteoarthritis, or/and
- nociplastic pain.

**14.** The compound or composition for use of claim 11 to 13, wherein administering comprises intravenous, intraperitoneal,

subcutaneous, intramuscular, intrathecal, peridural, topical, oral, gastric and/or rectal administration.

15. **A method of synthesizing** a compound according to any one of claims 1 to 8, preferably, wherein the method comprises a synthesis step of synthesizing a lactam core, preferably wherein said lactam core comprises the structure:

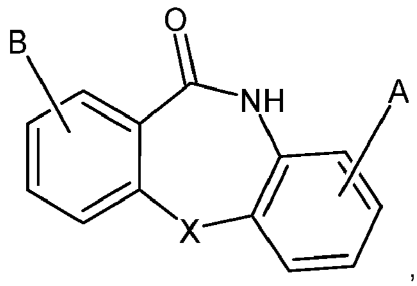

,

wherein A, X and B are defined as in any one of claims 1 to 8.

FIGURE 1

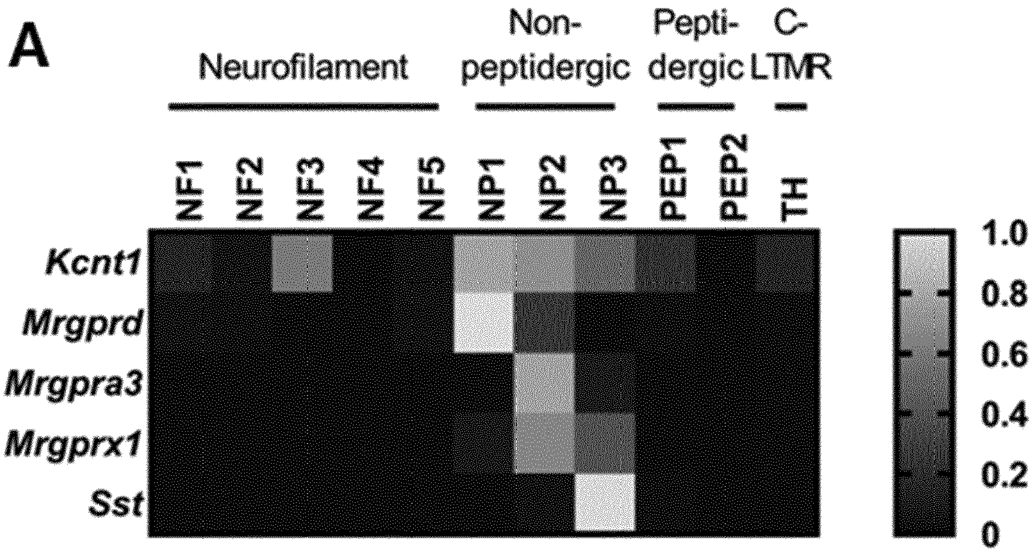
A
Neurofilament
Non-peptidergic
Pepti-dergic
C-LTMR
NF1
NF2
NF3
NF4
NF5
NP1
NP2
NP3
PEP1
PEP2
TH
Kcnt1
Mrgprd
Mrgpra3
Mrgprx1
Sst
1.0
0.8
0.6
0.4
0.2
0

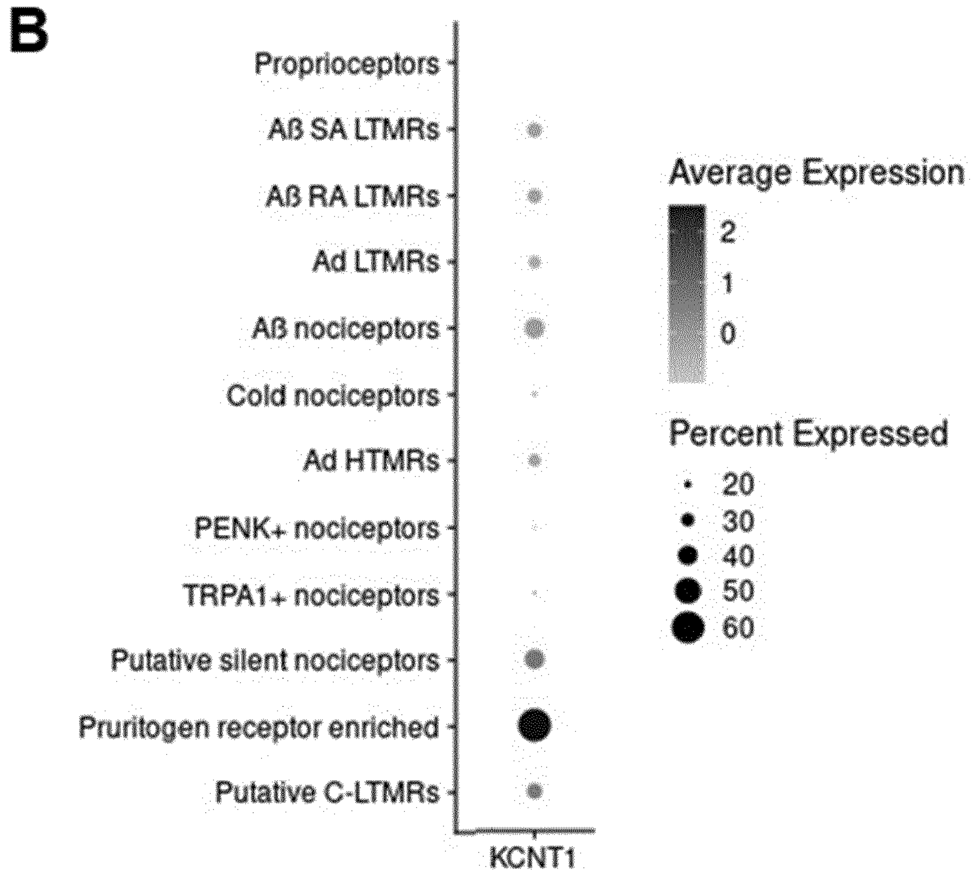
B
Proprioceptors
Aß SA LTMRs
Aß RA LTMRs
Ad LTMRs
Aß nociceptors
Cold nociceptors
Ad HTMRs
PENK+ nociceptors
TRPA1+ nociceptors
Putative silent nociceptors
Pruritogen receptor enriched
Putative C-LTMRs
KCNT1
Average Expression
2
1
0
Percent Expressed
20
30
40
50
60

FIGURE 1 cont.

C

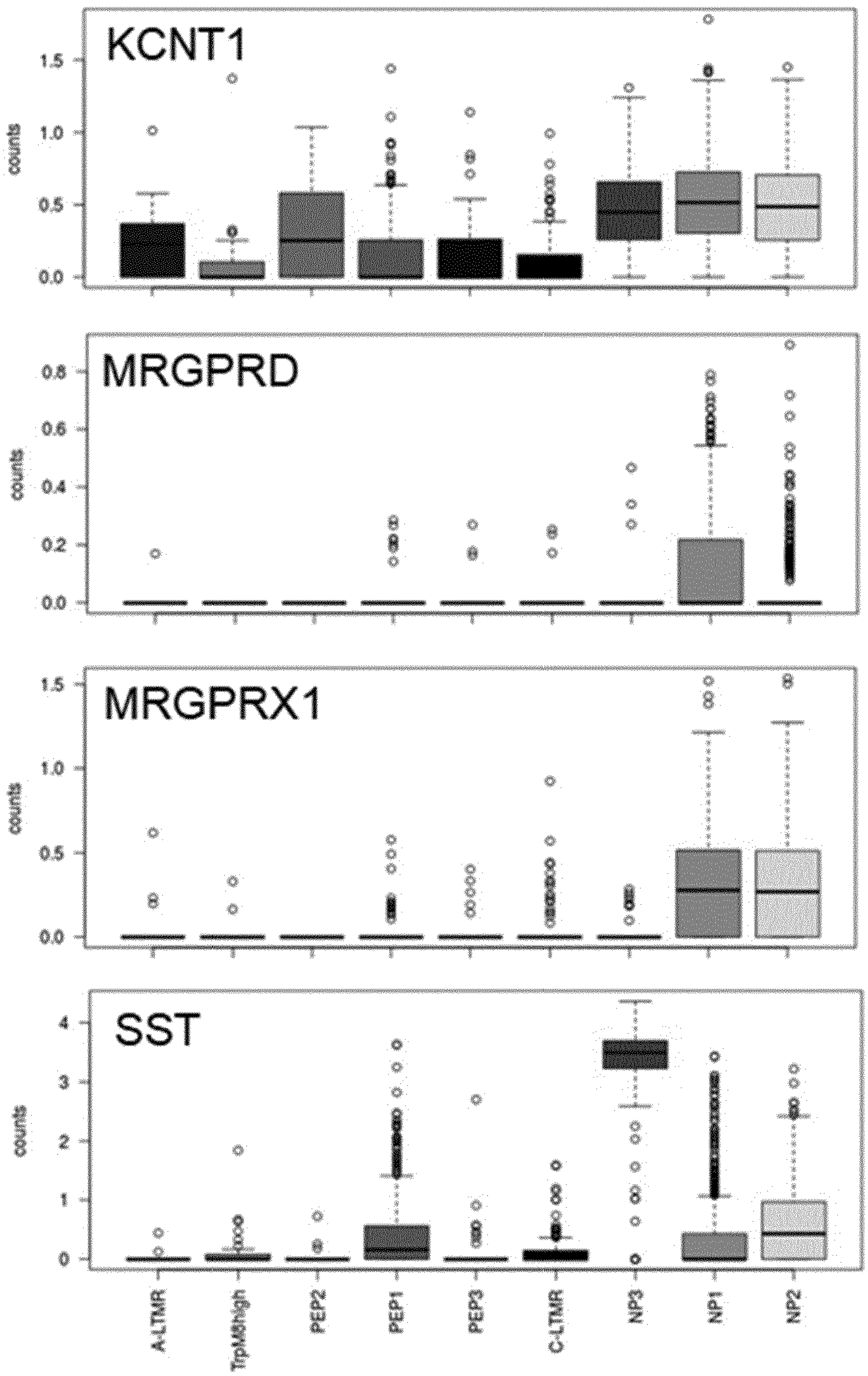

FIGURE 2

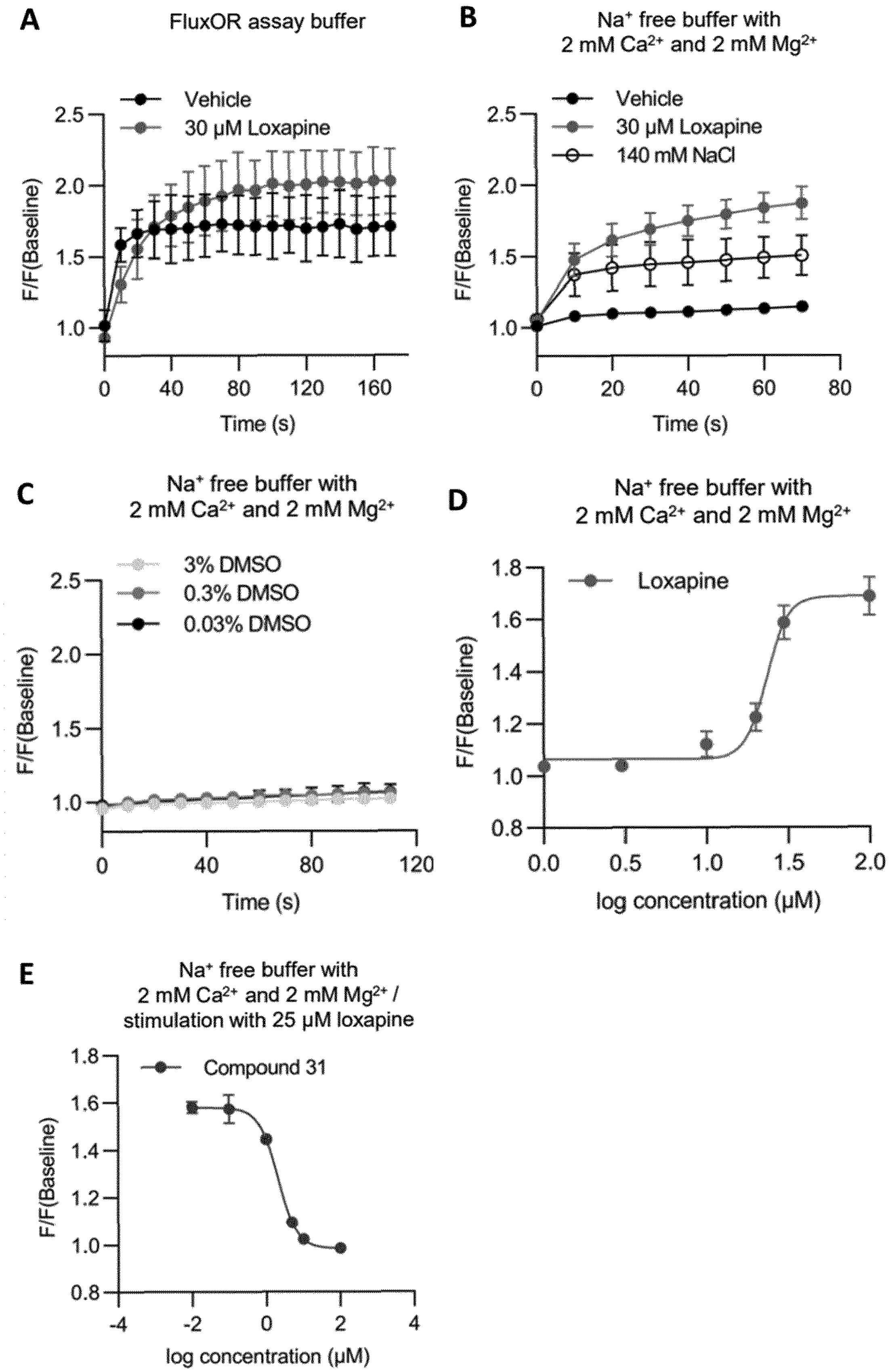
A
FluxOR assay buffer
Vehicle
30 μM Loxapine
F/F(Baseline)
Time (s)
B
Na+ free buffer with
2 mM Ca2+ and 2 mM Mg2+
Vehicle
30 μM Loxapine
140 mM NaCl
F/F(Baseline)
Time (s)
C
Na+ free buffer with
2 mM Ca2+ and 2 mM Mg2+
3% DMSO
0.3% DMSO
0.03% DMSO
F/F(Baseline)
Time (s)
D
Na+ free buffer with
2 mM Ca2+ and 2 mM Mg2+
Loxapine
F/F(Baseline)
log concentration (μM)
E
Na+ free buffer with
2 mM Ca2+ and 2 mM Mg2+ /
stimulation with 25 μM loxapine
Compound 31
F/F(Baseline)
log concentration (μM)

FIGURE 3

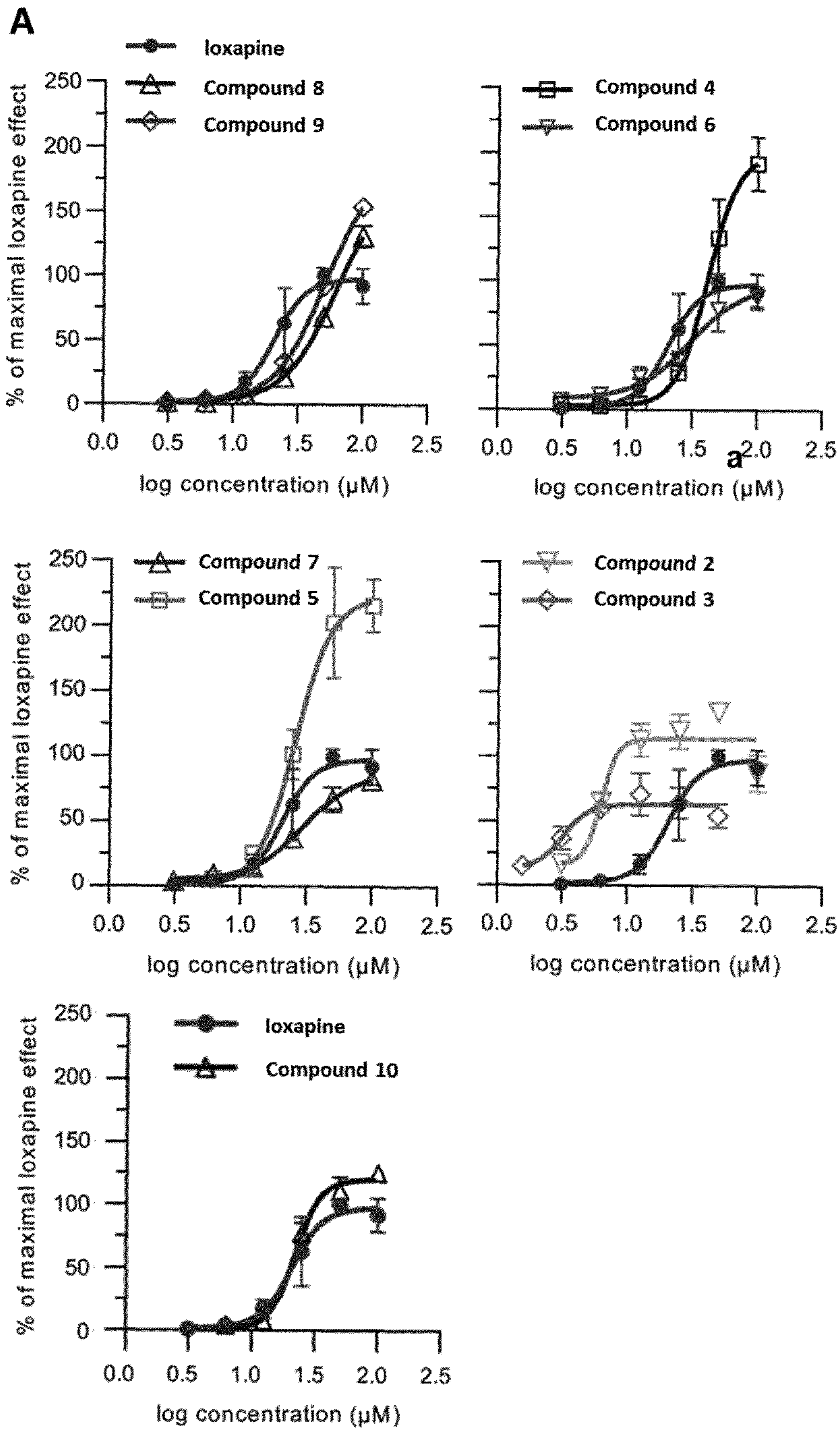
A
loxapine
Compound 8
Compound 9
Compound 4
Compound 6
Compound 7
Compound 5
Compound 2
Compound 3
loxapine
Compound 10
% of maximal loxapine effect
log concentration (µM)

FIGURE 3 cont.

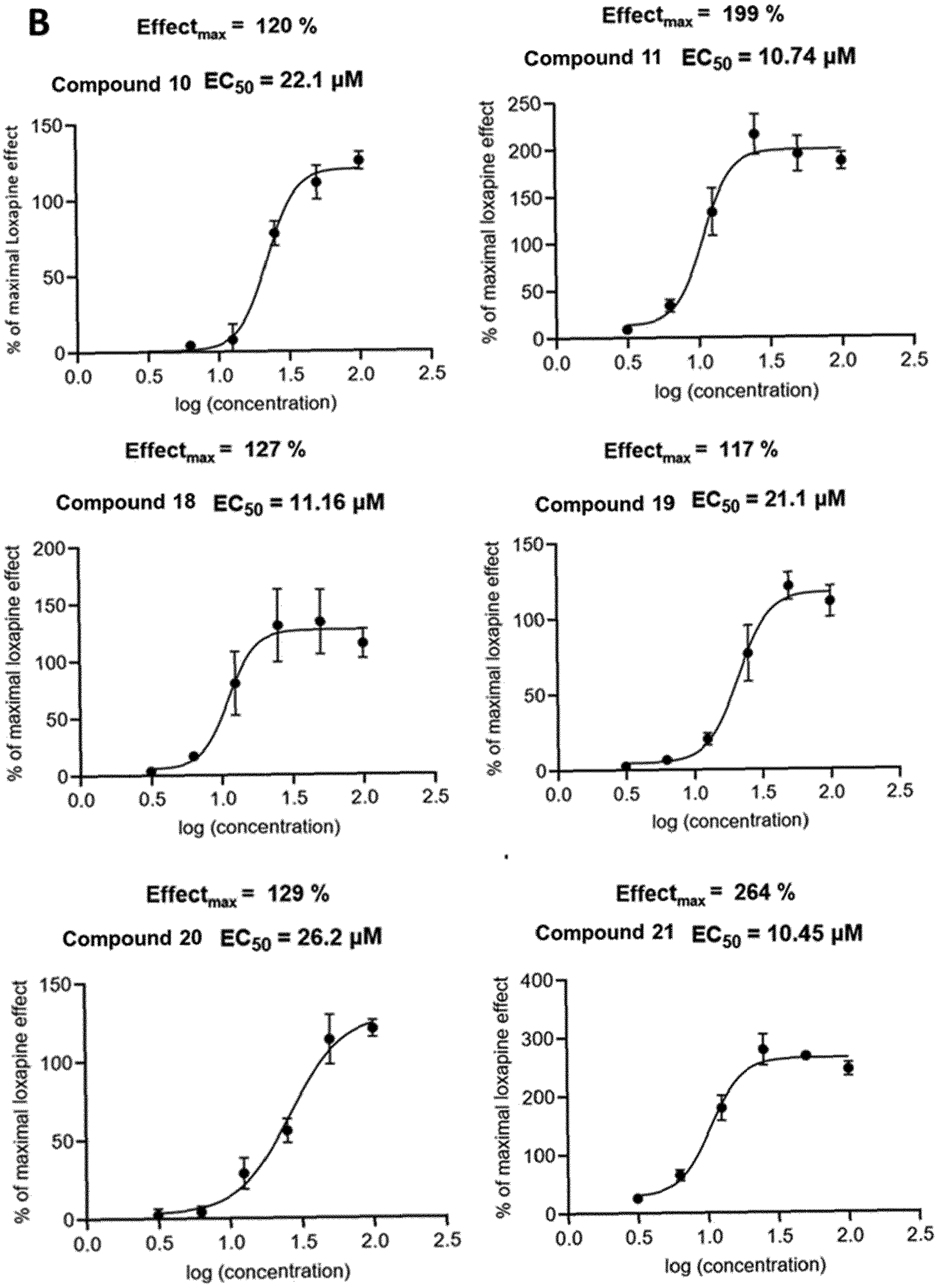

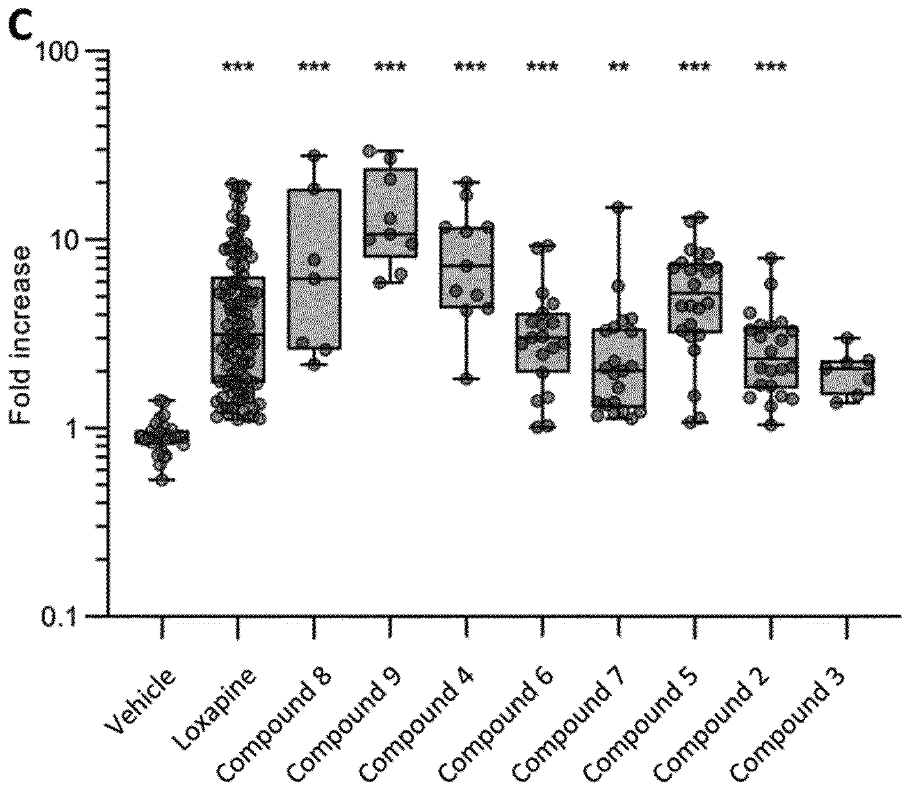
C
Fold increase
100
10
1
0.1
*** *** *** *** *** ** *** ***
Vehicle
Loxapine
Compound 8
Compound 9
Compound 4
Compound 6
Compound 7
Compound 5
Compound 2
Compound 3

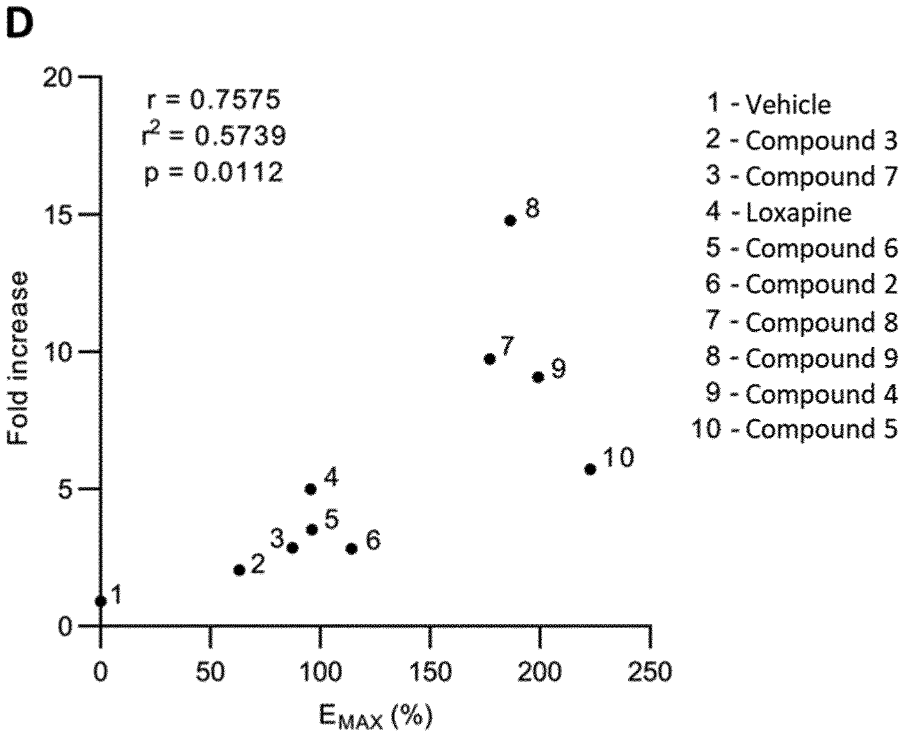
D
r = 0.7575
r2 = 0.5739
p = 0.0112
Fold increase
EMAX (%)
1 - Vehicle
2 - Compound 3
3 - Compound 7
4 - Loxapine
5 - Compound 6
6 - Compound 2
7 - Compound 8
8 - Compound 9
9 - Compound 4
10 - Compound 5

E

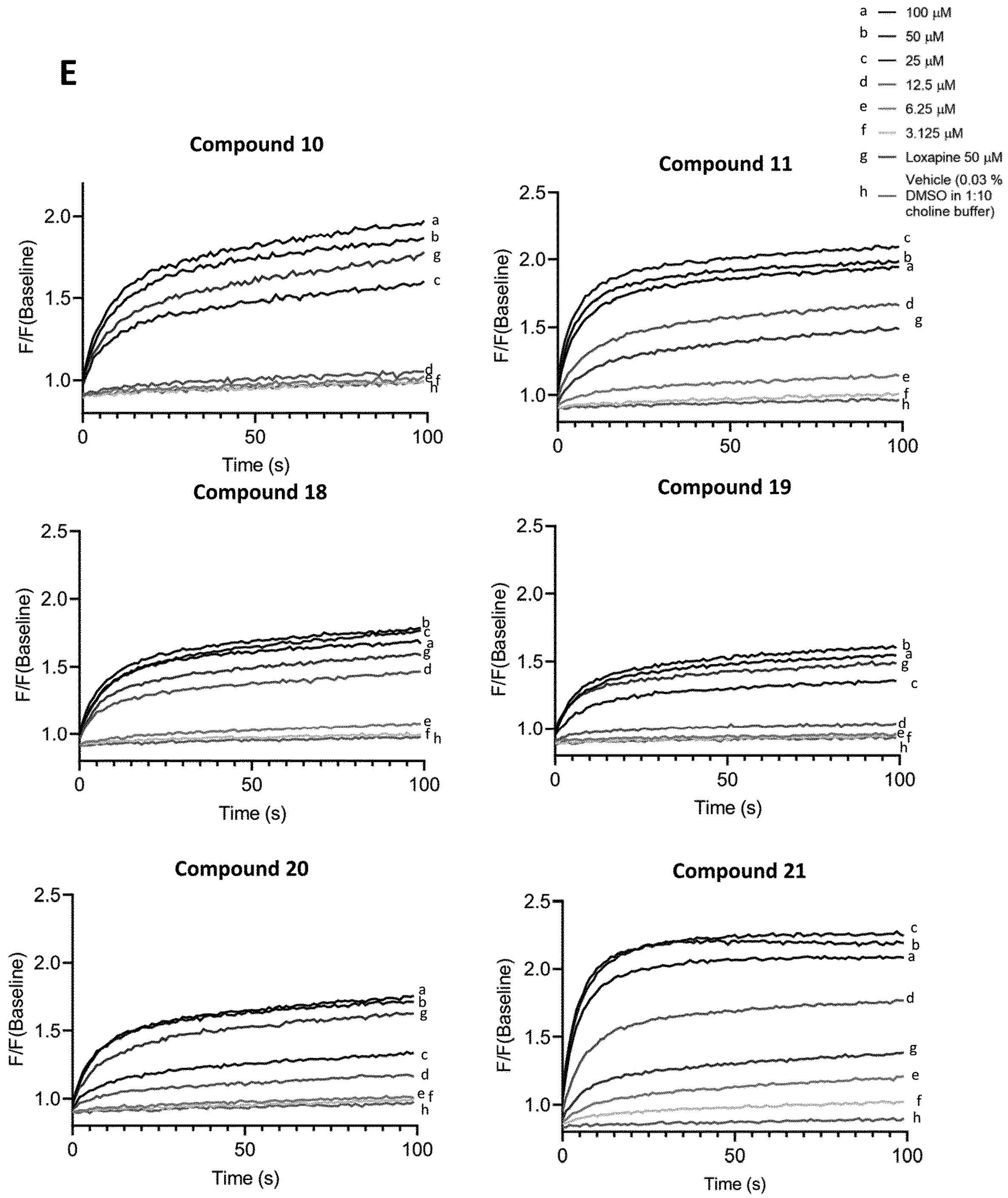
a 100 µM
b 50 µM
c 25 µM
d 12.5 µM
e 6.25 µM
f 3.125 µM
g Loxapine 50 µM
h Vehicle (0.03 % DMSO in 1:10 choline buffer)
Compound 10
Compound 11
Compound 18
Compound 19
Compound 20
Compound 21
F/F(Baseline)
Time (s)

FIGURE 4

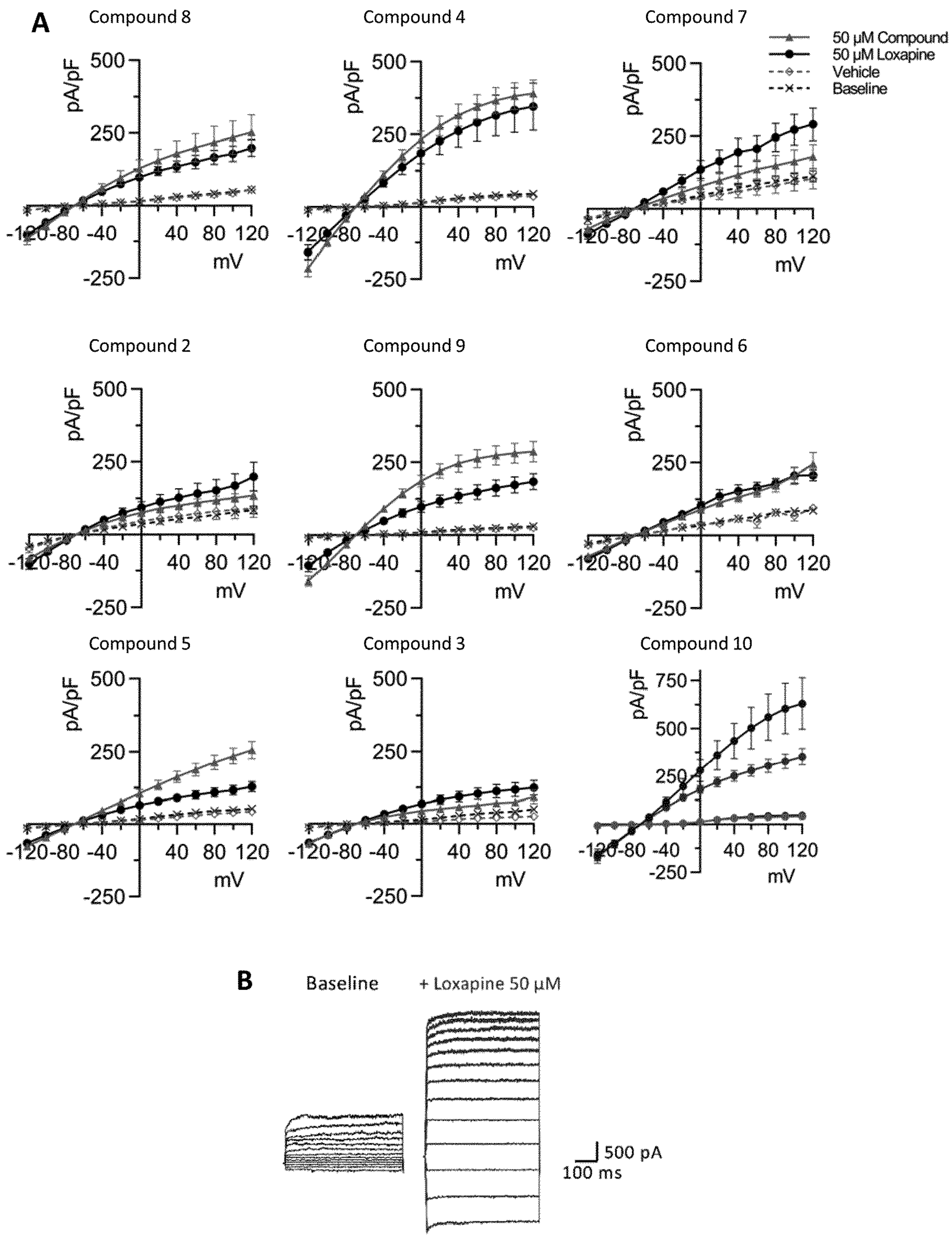
A
Compound 8
Compound 4
Compound 7
50 µM Compound
50 µM Loxapine
Vehicle
Baseline
Compound 2
Compound 9
Compound 6
Compound 5
Compound 3
Compound 10
pA/pF
mV
500
250
-250
750
-120 -80 -40 40 80 120
B
Baseline
+ Loxapine 50 µM
500 pA
100 ms

FIGURE 5

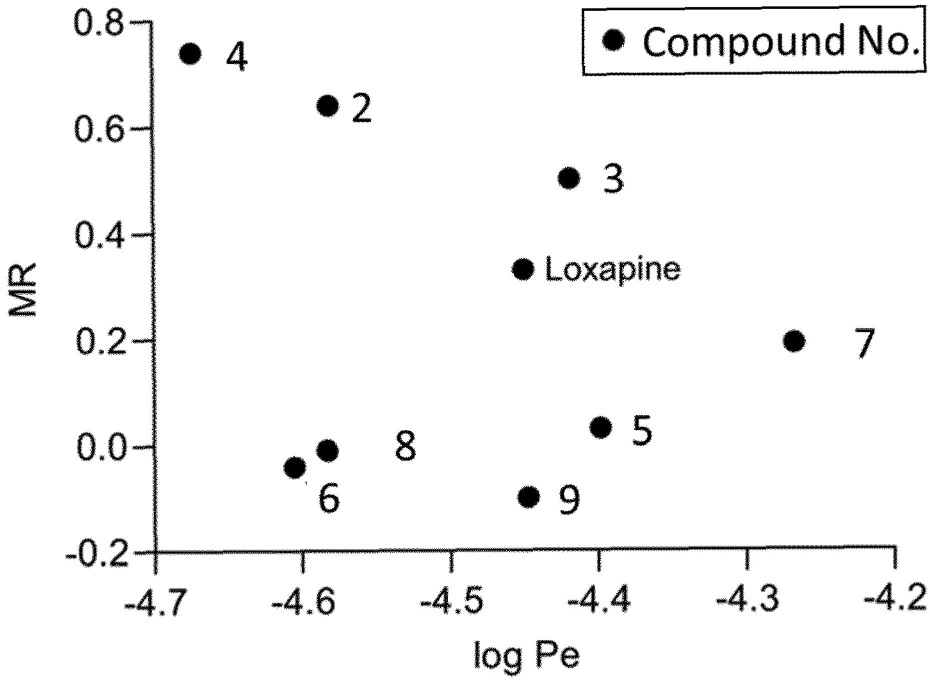
Compound No.
4
2
3
Loxapine
7
5
8
6
9
0.8
0.6
0.4
0.2
0.0
-0.2
MR
-4.7
-4.6
-4.5
-4.4
-4.3
-4.2
log Pe

FIGURE 6

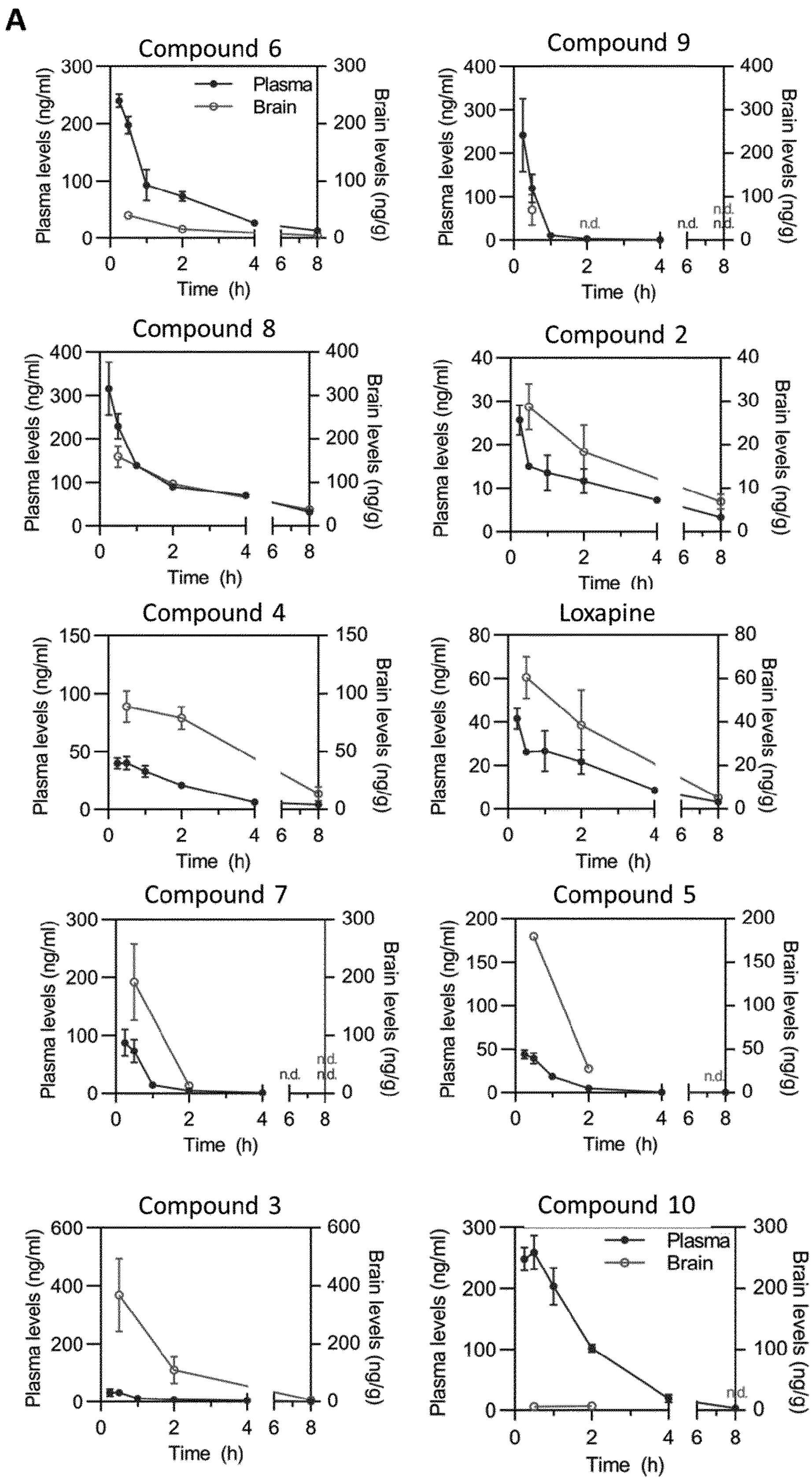
A
Compound 6
Compound 9
Compound 8
Compound 2
Compound 4
Loxapine
Compound 7
Compound 5
Compound 3
Compound 10
Plasma
Brain
Plasma levels (ng/ml)
Brain levels (ng/g)
Time (h)
n.d.

FIGURE 6 cont.

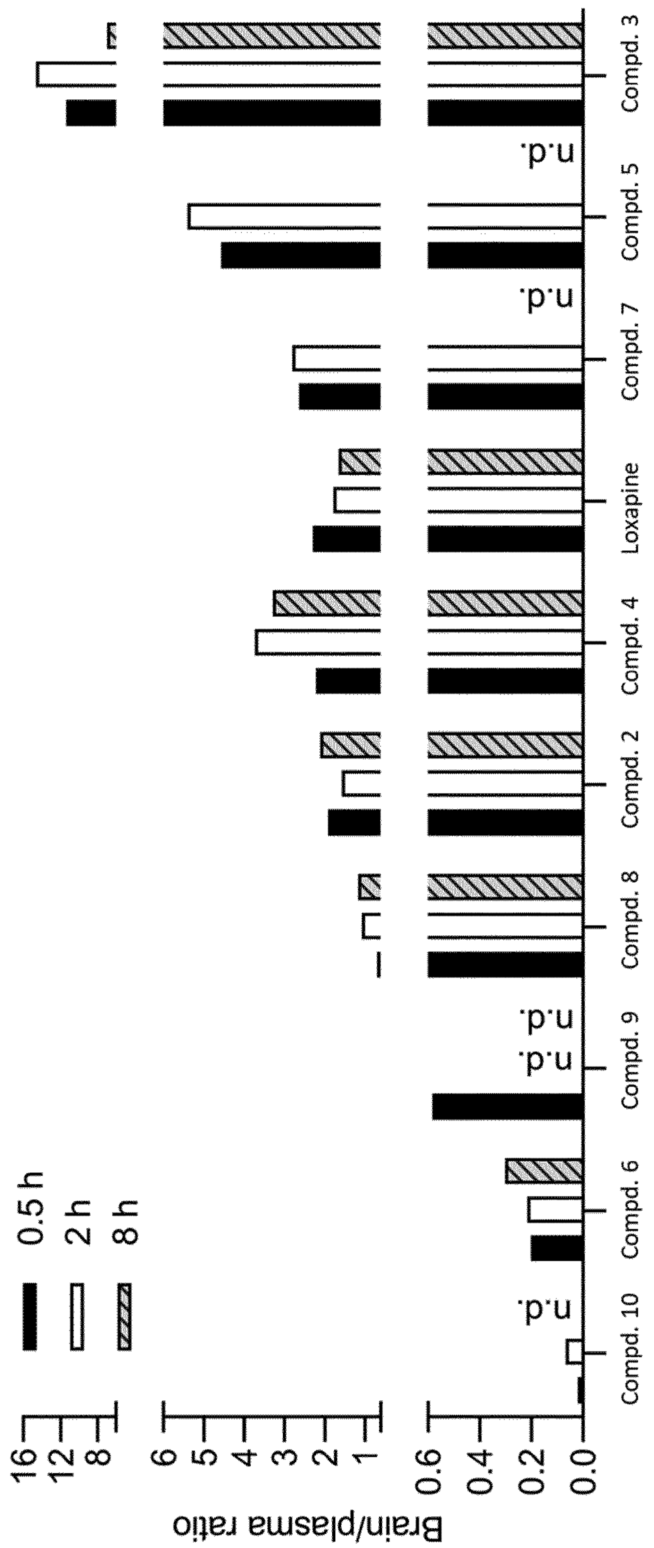

FIGURE 7

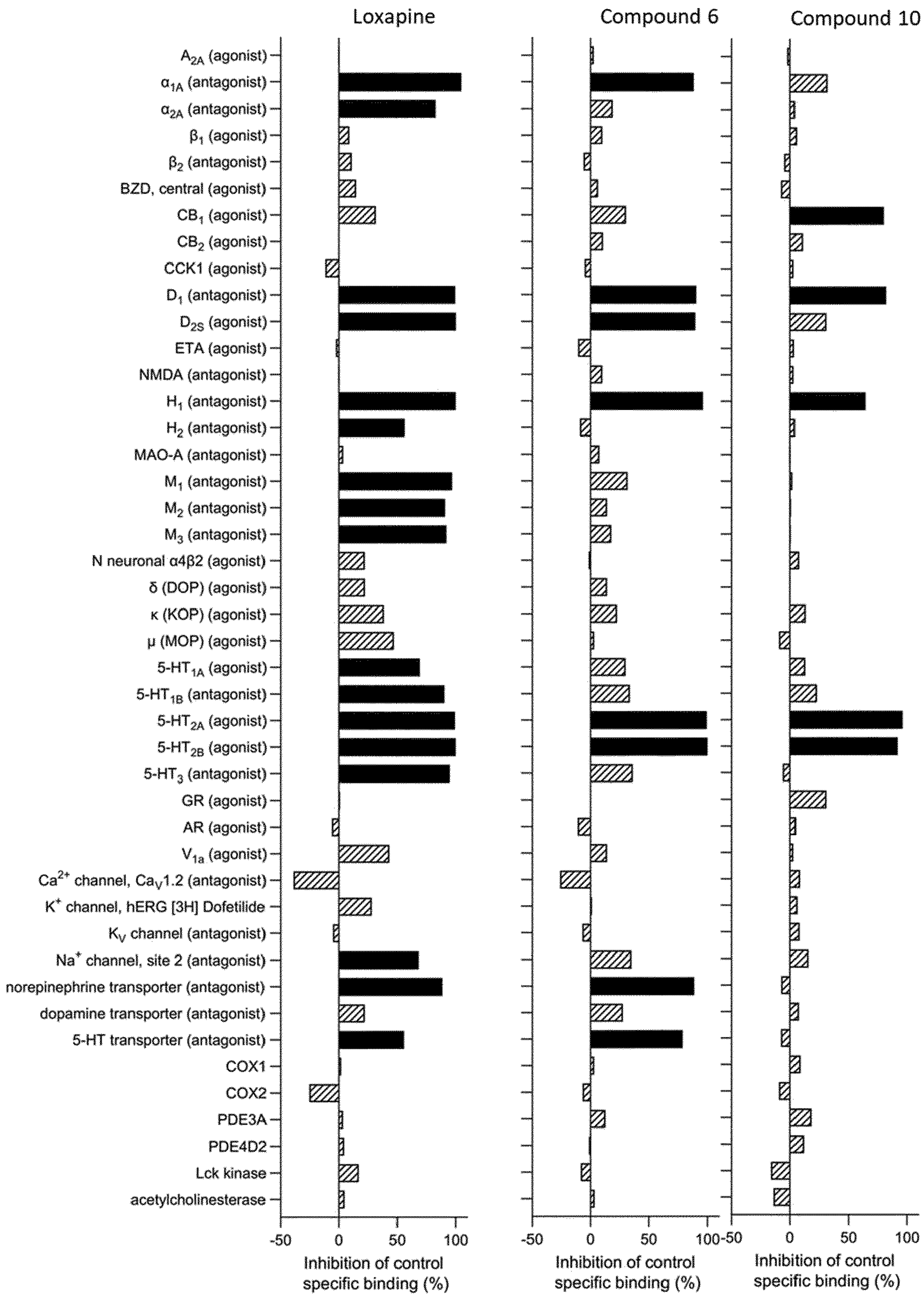
Loxapine
Compound 6
Compound 10
A2A (agonist)
α1A (antagonist)
α2A (antagonist)
β1 (agonist)
β2 (antagonist)
BZD, central (agonist)
CB1 (agonist)
CB2 (agonist)
CCK1 (agonist)
D1 (antagonist)
D2S (agonist)
ETA (agonist)
NMDA (antagonist)
H1 (antagonist)
H2 (antagonist)
MAO-A (antagonist)
M1 (antagonist)
M2 (antagonist)
M3 (antagonist)
N neuronal α4β2 (agonist)
δ (DOP) (agonist)
κ (KOP) (agonist)
μ (MOP) (agonist)
5-HT1A (agonist)
5-HT1B (antagonist)
5-HT2A (agonist)
5-HT2B (agonist)
5-HT3 (antagonist)
GR (agonist)
AR (agonist)
V1a (agonist)
Ca2+ channel, CaV1.2 (antagonist)
K+ channel, hERG [3H] Dofetilide
KV channel (antagonist)
Na+ channel, site 2 (antagonist)
norepinephrine transporter (antagonist)
dopamine transporter (antagonist)
5-HT transporter (antagonist)
COX1
COX2
PDE3A
PDE4D2
Lck kinase
acetylcholinesterase
-50 0 50 100
-50 0 50 100
-50 0 50 100
Inhibition of control specific binding (%)
Inhibition of control specific binding (%)
Inhibition of control specific binding (%)

FIGURE 8

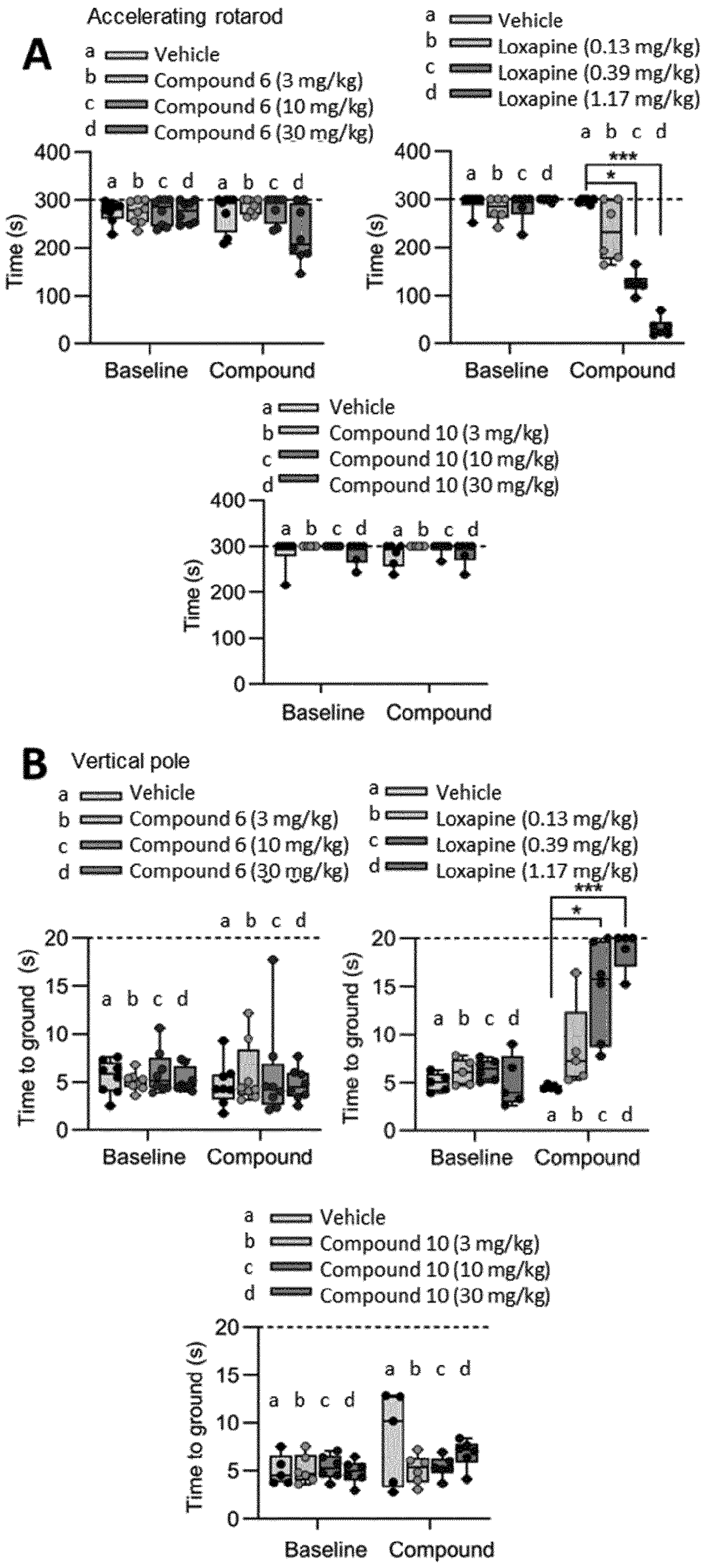
Accelerating rotarod
A
a Vehicle
b Compound 6 (3 mg/kg)
c Compound 6 (10 mg/kg)
d Compound 6 (30 mg/kg)
Time (s)
Baseline
Compound
a Vehicle
b Loxapine (0.13 mg/kg)
c Loxapine (0.39 mg/kg)
d Loxapine (1.17 mg/kg)
a Vehicle
b Compound 10 (3 mg/kg)
c Compound 10 (10 mg/kg)
d Compound 10 (30 mg/kg)
B
Vertical pole
Time to ground (s)

FIGURE 8 cont.

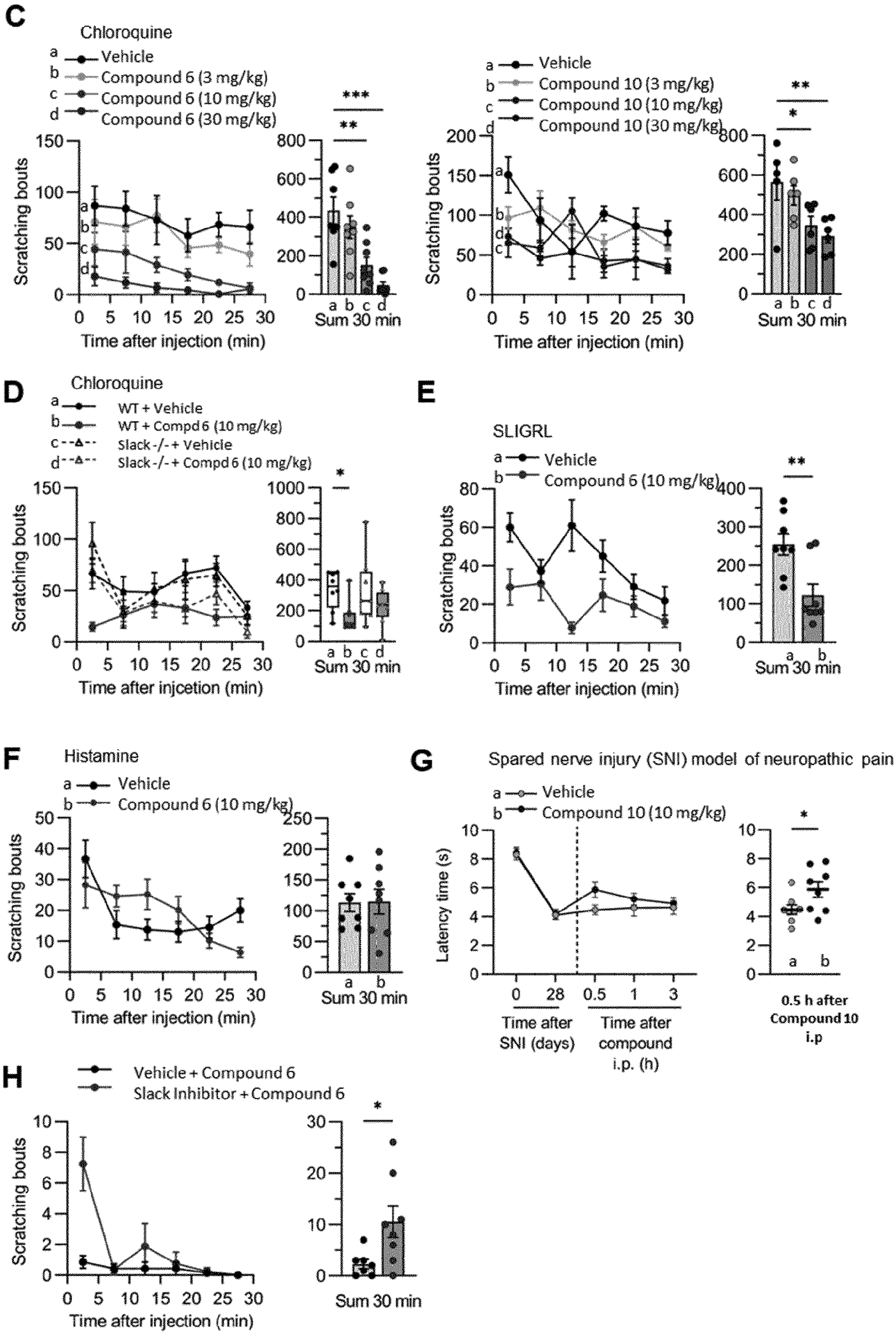

FIGURE 9

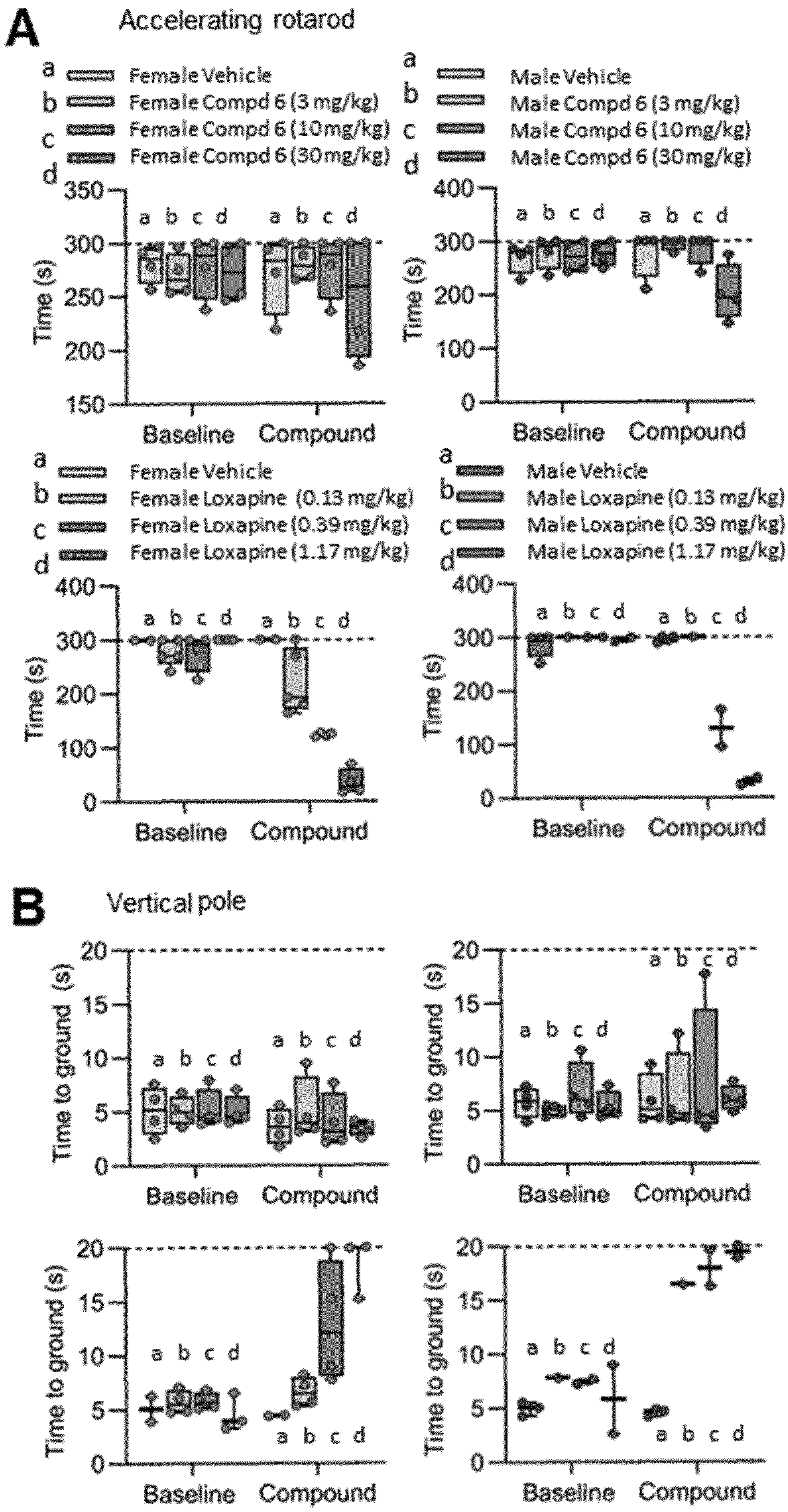
A
Accelerating rotarod
a Female Vehicle
b Female Compd 6 (3 mg/kg)
c Female Compd 6 (10mg/kg)
d Female Compd 6 (30mg/kg)
a Male Vehicle
b Male Compd 6 (3 mg/kg)
c Male Compd 6 (10mg/kg)
d Male Compd 6 (30mg/kg)
Time (s)
Baseline
Compound
a Female Vehicle
b Female Loxapine (0.13 mg/kg)
c Female Loxapine (0.39 mg/kg)
d Female Loxapine (1.17 mg/kg)
a Male Vehicle
b Male Loxapine (0.13 mg/kg)
c Male Loxapine (0.39 mg/kg)
d Male Loxapine (1.17 mg/kg)
B
Vertical pole
Time to ground (s)

FIGURE 9 cont.

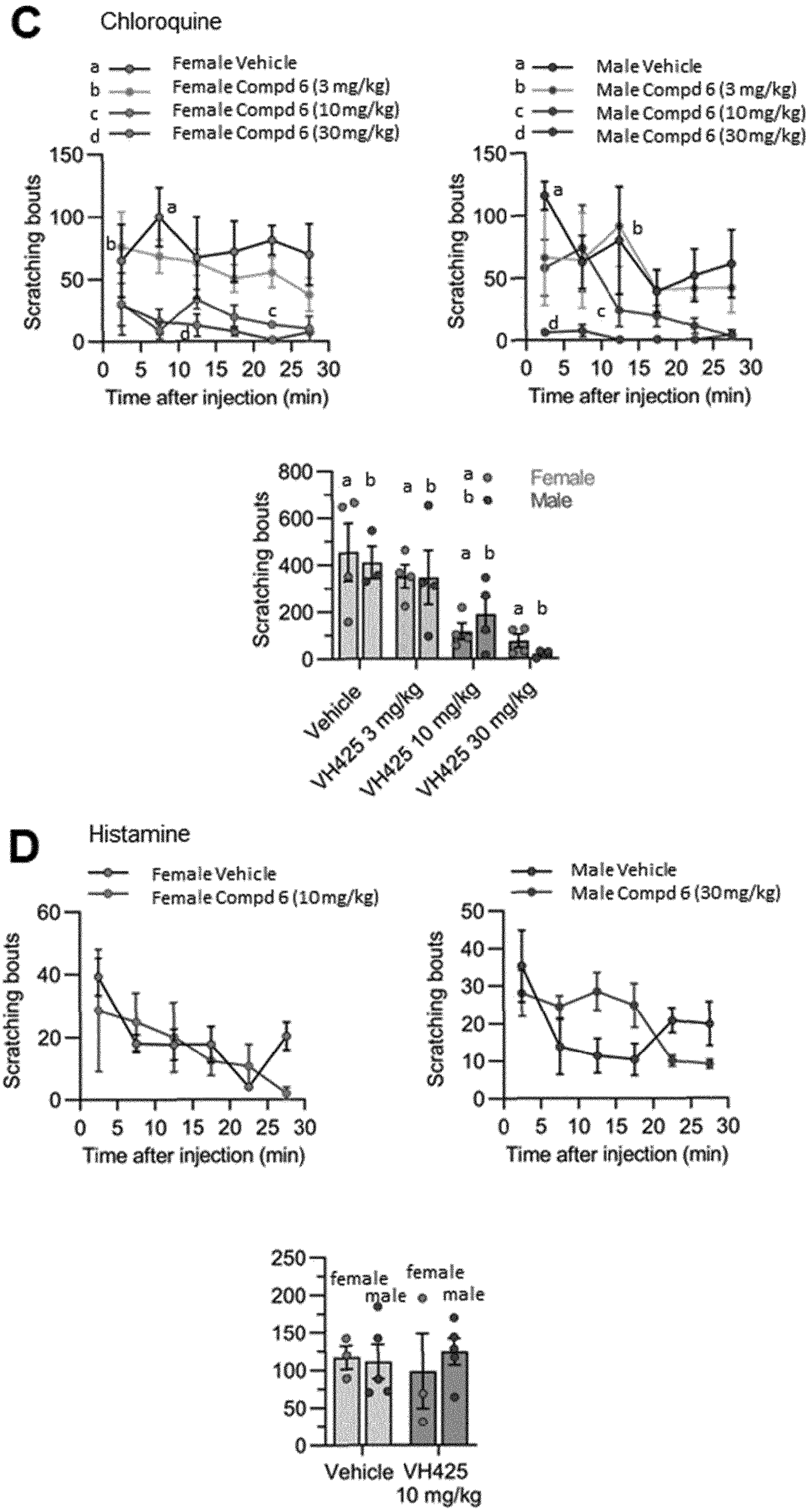

FIGURE 10

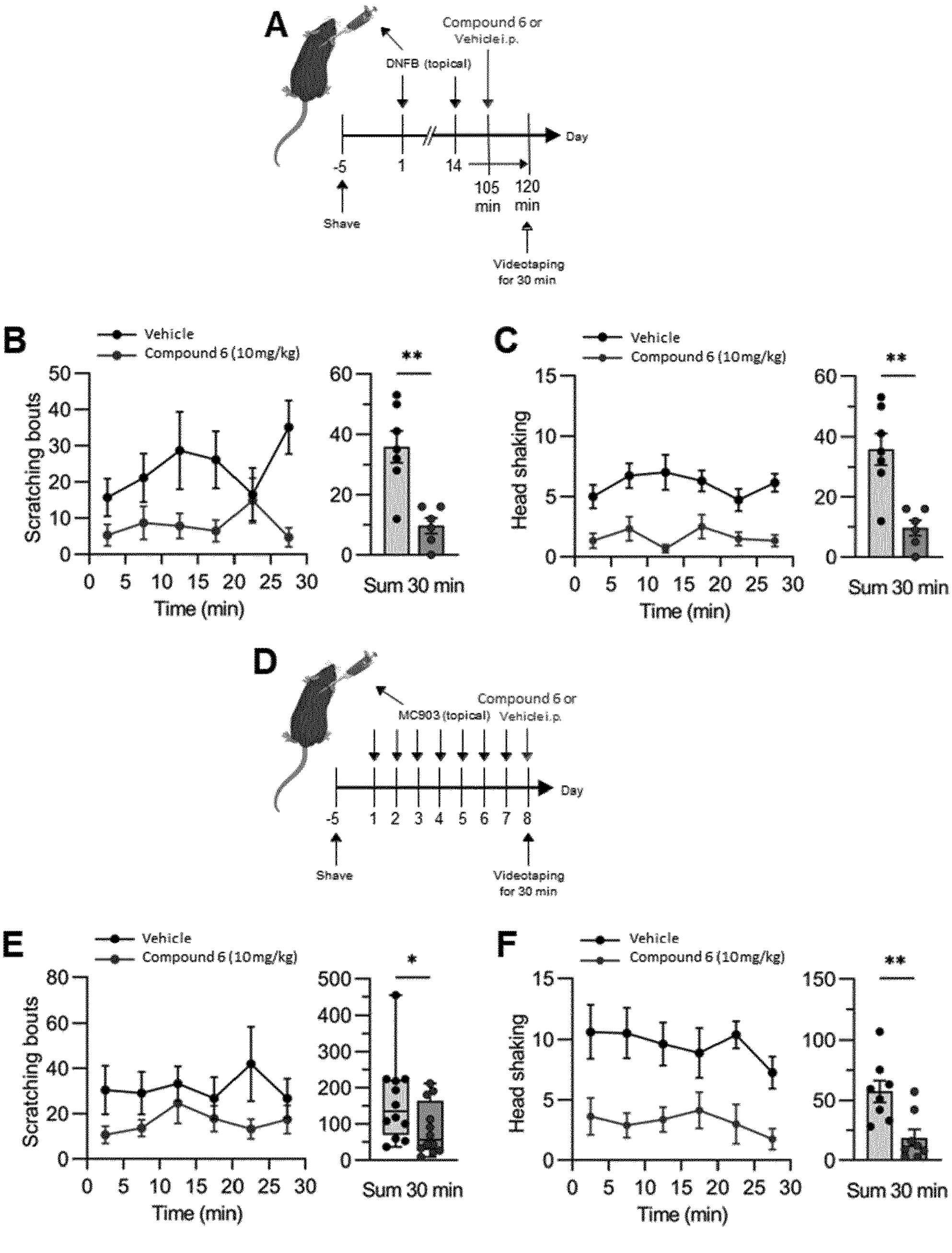
A
Compound 6 or Vehicle i.p.
DNFB (topical)
Day
-5
1
14
105 min
120 min
Shave
Videotaping for 30 min
B
Vehicle
Compound 6 (10mg/kg)
Scratching bouts
Time (min)
Sum 30 min
**
C
Vehicle
Compound 6 (10mg/kg)
Head shaking
Time (min)
Sum 30 min
**
D
MC903 (topical)
Compound 6 or Vehicle i.p.
Day
-5 1 2 3 4 5 6 7 8
Shave
Videotaping for 30 min
E
Vehicle
Compound 6 (10mg/kg)
Scratching bouts
Time (min)
Sum 30 min
*
F
Vehicle
Compound 6 (10mg/kg)
Head shaking
Time (min)
Sum 30 min
**

FIGURE 11

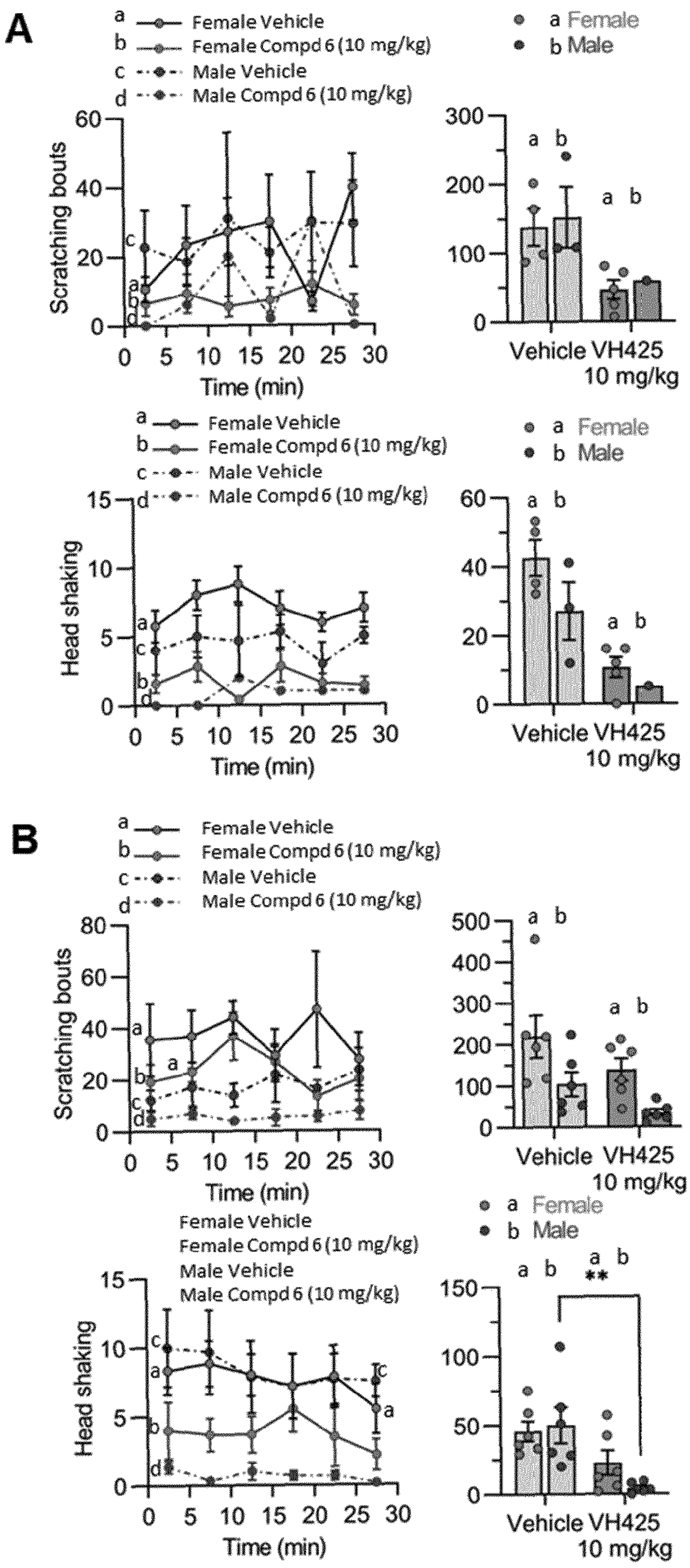
A
a Female Vehicle
b Female Compd 6 (10 mg/kg)
c Male Vehicle
d Male Compd 6 (10 mg/kg)
Scratching bouts
Time (min)
a Female
b Male
Vehicle
VH425 10 mg/kg
Head shaking
B
**

FIGURE 11 cont.

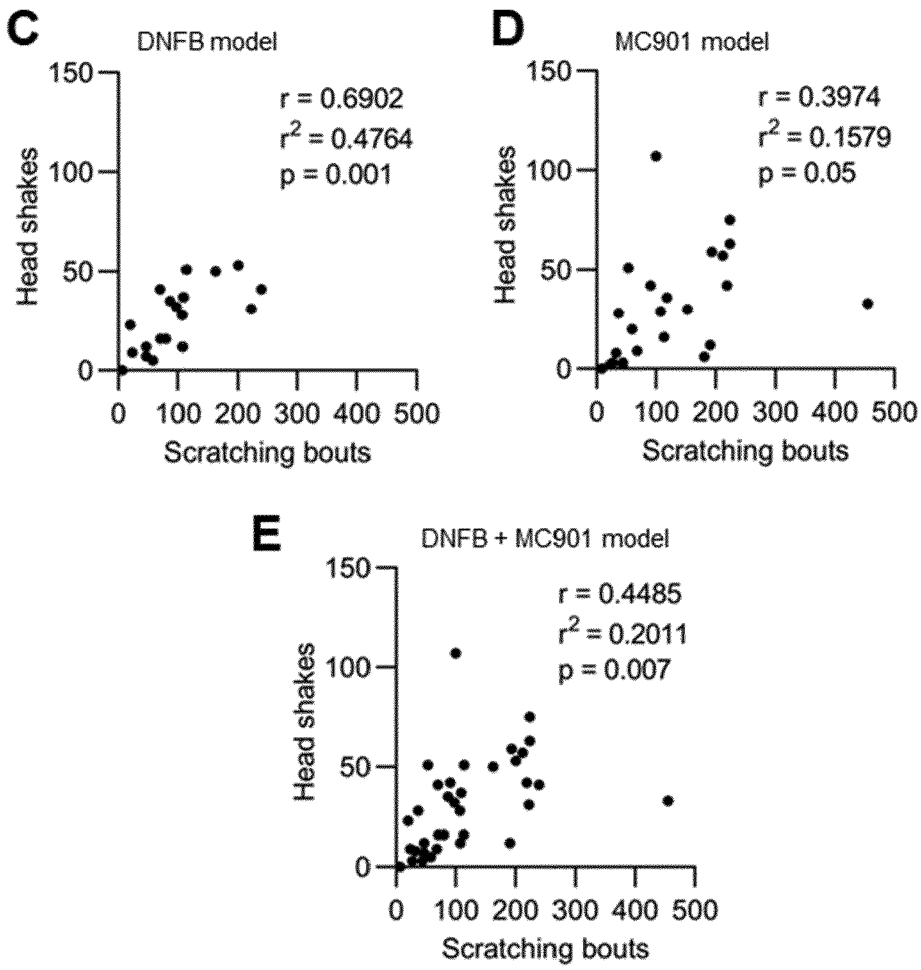

FIGURE 12

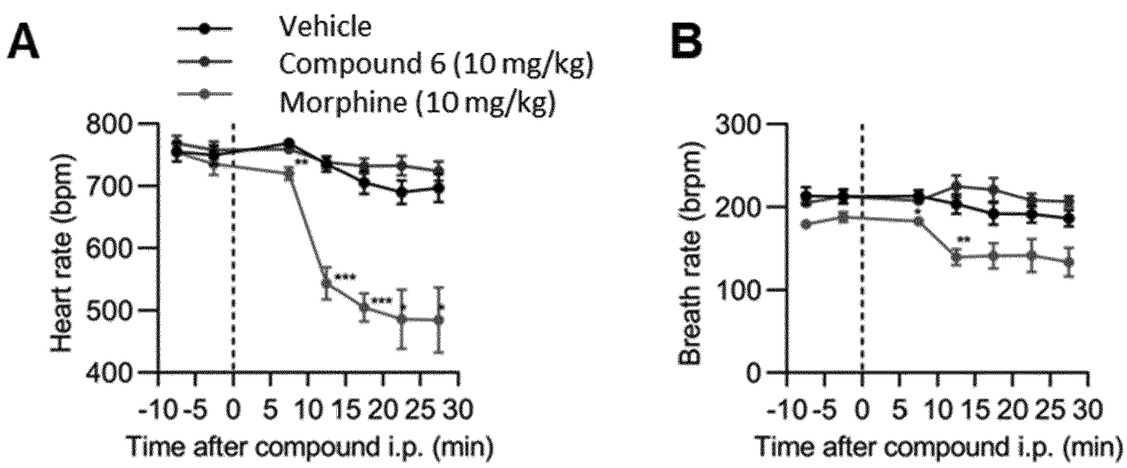
A
Vehicle
Compound 6 (10 mg/kg)
Morphine (10 mg/kg)
Heart rate (bpm)
800
700
600
500
400
-10 -5 0 5 10 15 20 25 30
Time after compound i.p. (min)
B
Breath rate (brpm)
300
200
100
0
-10 -5 0 5 10 15 20 25 30
Time after compound i.p. (min)

FIGURE 13

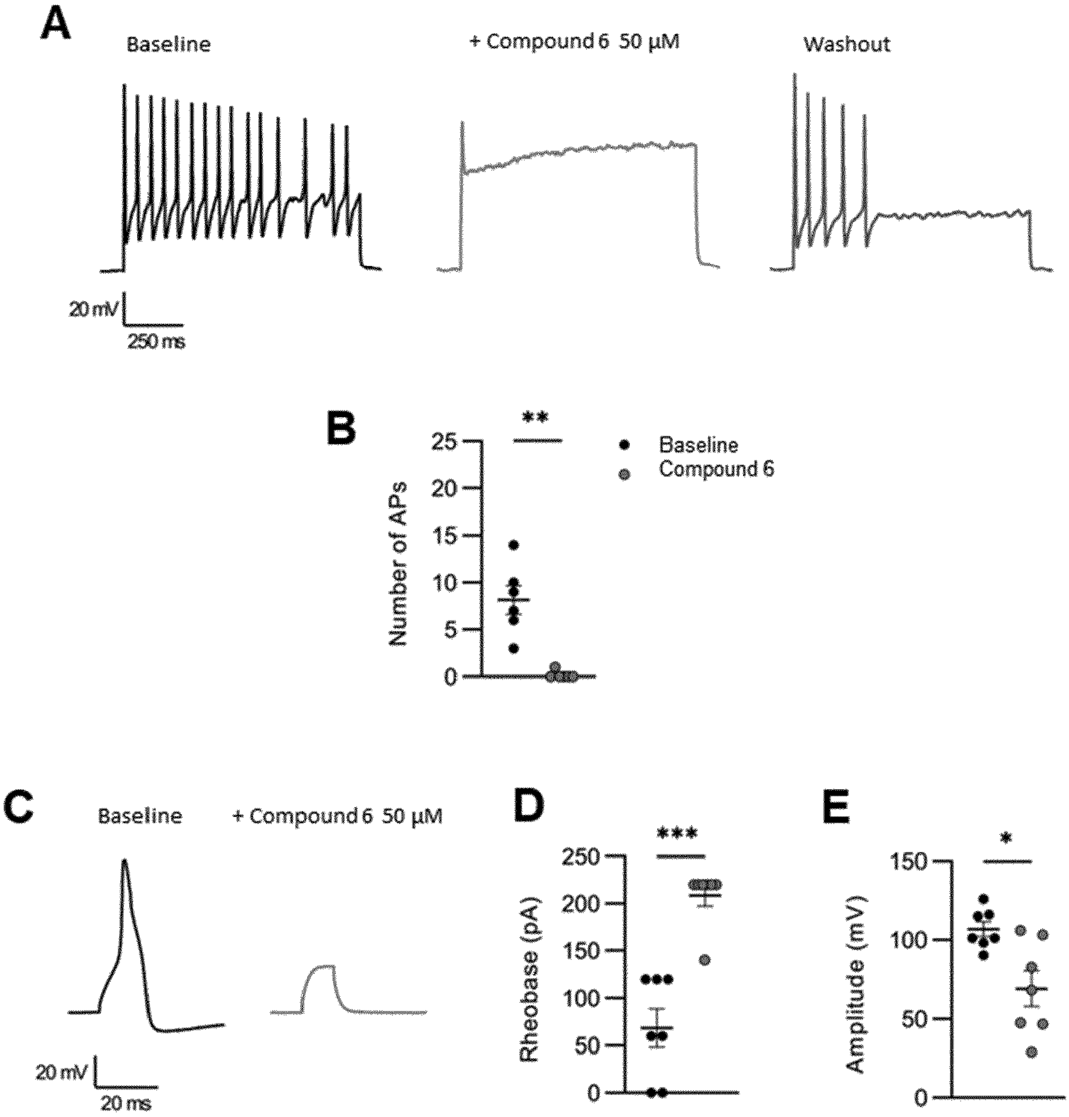
A
Baseline
+ Compound 6 50 µM
Washout
20 mV
250 ms
B
**
Number of APs
25
20
15
10
5
0
Baseline
Compound 6
C
Baseline
+ Compound 6 50 µM
20 mV
20 ms
D
***
Rheobase (pA)
250
200
150
100
50
0
E
*
Amplitude (mV)
150
100
50
0

Europäisches Patentamt
European Patent Office
Office européen des brevets

# PARTIAL EUROPEAN SEARCH REPORT

under Rule 62a and/or 63 of the European Patent Convention. This report shall be considered, for the purposes of subsequent proceedings, as the European search report

Application Number
EP 23 16 0797

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 107 469 085 A (HARVARD COLLEGE; GEN HOSPITAL CORP) 15 December 2017 (2017-12-15) * page 22; claims * | 1-15 | INV. C07D413/04 C07D413/14 C07D417/04 A61K31/553 A61K31/554 A61P17/00 |
| X | WO 2006/088786 A2 (COMBINATORX INC [US]; FOLEY MICHAEL A [US] ET AL.) 24 August 2006 (2006-08-24) * page 32, fifth and sixth compounds; claims * | 1-4,6-15 | |
| X | WO 2017/024037 A1 (HARVARD COLLEGE [US]; CHILDREN'S MEDICAL CENTER CORP [US]) 9 February 2017 (2017-02-09) * page 18, scheme 2; claims * | 1-4,6-15 | |
| X | CN 101 060 847 A (HYPNION INC [US]) 24 October 2007 (2007-10-24) * page 123 - page 131; claims * | 1-3,6-15 | |
| | -/-- | | |

TECHNICAL FIELDS SEARCHED (IPC)

C07D
A61K

## INCOMPLETE SEARCH

The Search Division considers that the present application, or one or more of its claims, does/do not comply with the EPC so that only a partial search (R.62a, 63) has been carried out.

Claims searched completely :

Claims searched incompletely :

Claims not searched :

Reason for the limitation of the search:

see sheet C

2

| Place of search | Date of completion of the search | Examiner |
|---|---|---|
| Berlin | 27 November 2023 | Beyss-Kahana, Ellen |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04E07)

Europäisches Patentamt
European Patent Office
Office européen des brevets

# PARTIAL EUROPEAN SEARCH REPORT

Application Number
EP 23 16 0797

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2008/063603 A2 (GEN HOSPITAL CORP) 29 May 2008 (2008-05-29) * page 38, scheme 3; claims * ----- | 1-3,6-15 | |
| X | WO 2010/081036 A2 (HARVARD COLLEGE [US]; RITTER TOBIAS [US] ET AL.) 15 July 2010 (2010-07-15) * page 104; claims * ----- | 1-3,6-15 | |
| X | DE 18 02 728 A1 (AMERICAN CYANAMID CO) 26 June 1969 (1969-06-26) * claims; examples * ----- | 1-3,6-15 | |
| | | | TECHNICAL FIELDS SEARCHED (IPC) |
| X | CH 422 793 A (WANDER AG DR A [CH]) 31 October 1966 (1966-10-31) * table II, compounds 23, 27 * ----- | 1-4,6-15 | |
| X | US 3 660 406 A (HOWELL CHARLES FREDERICK ET AL) 2 May 1972 (1972-05-02) * claims; examples * ----- | 1-4,6-15 | |
| X | CH 436 297 A (WANDER AG DR A [CH]) 31 May 1967 (1967-05-31) * table II, compounds 16, 22 * ----- | 1-4,6-15 | |
| X | WO 2009/154563 A1 (ASTRAZENECA AB [SE]; BROWN DEAN [US] ET AL.) 23 December 2009 (2009-12-23) * schemes 1, 2; claims; examples * ----- | 1-3,6-15 | |

-/--

2

EPO FORM 1503 03.82 (P04C10)

Europäisches Patentamt
European Patent Office
Office européen des brevets

# PARTIAL EUROPEAN SEARCH REPORT

Application Number
EP 23 16 0797

| DOCUMENTS CONSIDERED TO BE RELEVANT | | | CLASSIFICATION OF THE APPLICATION (IPC) |
|---|---|---|---|
| Category | Citation of document with indication, where appropriate, of relevant passages | Relevant to claim | |
| X | MCEVOY F J ET AL: "2-TRIFLUOROMETHOXYDIBENZ B,E3?4 1,43?4DIAZEPINE AND 2-TRIFLUOROMETHOXYDIBENZ B,F3?4 1,43?4-OXAZEPINE DERIVATIVES", JOURNAL OF MEDICINAL CHEMISTRY, AMERICAN CHEMICAL SOCIETY, US, vol. 13, 1 March 1970 (1970-03-01), pages 295-297, XP000999698, ISSN: 0022-2623, DOI: 10.1021/JM00296A032 * table 1, compounds 20d, 3j * ----- | 1-4,6-15 | |
| | | | TECHNICAL FIELDS SEARCHED (IPC) |

2

EPO FORM 1503 03.82 (P04C10)

Europäisches Patentamt
European Patent Office
Office européen des brevets

# INCOMPLETE SEARCH SHEET C

Application Number
EP 23 16 0797

Claim(s) completely searchable:
5

Claim(s) searched incompletely:
1-4, 6-15

Reason for the limitation of the search:

The present claims 1-4, 6-15 relate to an extremely large number of possible compounds, pharmaceutical compositions and uses thereof, due to the fact that formulas (I), (II) of claims 1, 3 accordingly contain so many options, variables, possible permutations and provisos. Support and disclosure within the meaning of Articles 84 and 83 EPC are to be found, however, for only a very small proportion of the compounds claimed, see especially the working examples.
Non-compliance with the substantive provisions is such that a meaningful search of the whole claimed subject-matter can not be carried out (Rule 63 EPC and Guidelines B-VIII, 3).
It is furthermore stressed that, the initial phase of the search revealed a very large number of documents relevant to the issue of novelty. So many documents were retrieved that it is impossible to determine which parts of claims 1-4, 6-15 may be said to define subject-matter for which protection might legitimately be sought (Article 84 EPC). For these reasons, a meaningful search of the whole claimed subject-matter of claims 1-4, 6-15 can not be carried out (Rule 63 EPC).
The Search Division considers the subject-matter of claim 1, wherein the nitrogen containing ring means a piperazine or a diazepam moiety, B is always in position 2, Y never means a hydrogen atom, to represent a subject-matter on which a meaningful search can be performed since it represents a legitimated scope of protection in view of the examples and biological results put forward.
Pursuant to Rule 63(2) EPC), the search of claims 1-4, 6-15 was consequently restricted to compounds of formula (I) wherein the nitrogen containing ring means a piperazine or a diazepam moiety, B is always in position 2, Y never means a hydrogen atom.

## ANNEX TO THE EUROPEAN SEARCH REPORT ON EUROPEAN PATENT APPLICATION NO.

EP 23 16 0797

This annex lists the patent family members relating to the patent documents cited in the above-mentioned European search report. The members are as contained in the European Patent Office EDP file on
The European Patent Office is in no way liable for these particulars which are merely given for the purpose of information.

27-11-2023

| Patent document cited in search report | Publication date | Patent family member(s) | Publication date |
|---|---|---|---|
| CN 107469085 A | 15-12-2017 | CN 101610789 A | 23-12-2009 |
| | | CN 107469085 A | 15-12-2017 |
| WO 2006088786 A2 | 24-08-2006 | AU 2006214517 A1 | 24-08-2006 |
| | | BR PI0607851 A2 | 13-06-2009 |
| | | CA 2597306 A1 | 24-08-2006 |
| | | CN 101160280 A | 09-04-2008 |
| | | EP 1850855 A2 | 07-11-2007 |
| | | JP 2008530129 A | 07-08-2008 |
| | | KR 20070103499 A | 23-10-2007 |
| | | TW 200640467 A | 01-12-2006 |
| | | US 2006281722 A1 | 14-12-2006 |
| | | WO 2006088786 A2 | 24-08-2006 |
| WO 2017024037 A1 | 09-02-2017 | AU 2016301282 A1 | 29-03-2018 |
| | | CA 2994545 A1 | 09-02-2017 |
| | | CN 108348774 A | 31-07-2018 |
| | | EP 3331609 A1 | 13-06-2018 |
| | | IL 257326 A | 29-03-2018 |
| | | JP 6833811 B2 | 24-02-2021 |
| | | JP 2018530518 A | 18-10-2018 |
| | | JP 2021075546 A | 20-05-2021 |
| | | KR 20180069782 A | 25-06-2018 |
| | | RU 2018107695 A | 05-09-2019 |
| | | US 2018237392 A1 | 23-08-2018 |
| | | US 2020377459 A1 | 03-12-2020 |
| | | US 2022041552 A1 | 10-02-2022 |
| | | WO 2017024037 A1 | 09-02-2017 |
| CN 101060847 A | 24-10-2007 | CN 101060847 A | 24-10-2007 |
| | | SI 1804804 T1 | 30-04-2012 |
| | | UA 86265 C2 | 10-04-2009 |
| | | ZA 200702334 B | 25-09-2008 |
| WO 2008063603 A2 | 29-05-2008 | AU 2007322033 A1 | 29-05-2008 |
| | | CA 2668652 A1 | 29-05-2008 |
| | | CA 3002443 A1 | 29-05-2008 |
| | | CN 103933572 A | 23-07-2014 |
| | | DK 2101819 T3 | 29-04-2013 |
| | | EP 2101819 A2 | 23-09-2009 |
| | | EP 2425858 A2 | 07-03-2012 |
| | | EP 2446903 A2 | 02-05-2012 |
| | | ES 2402789 T3 | 08-05-2013 |
| | | JP 5926301 B2 | 25-05-2016 |
| | | JP 2010510227 A | 02-04-2010 |
| | | JP 2014111626 A | 19-06-2014 |

EPO FORM P0459

For more details about this annex : see Official Journal of the European Patent Office, No. 12/82

# ANNEX TO THE EUROPEAN SEARCH REPORT ON EUROPEAN PATENT APPLICATION NO. EP 23 16 0797

This annex lists the patent family members relating to the patent documents cited in the above-mentioned European search report.
The members are as contained in the European Patent Office EDP file on
The European Patent Office is in no way liable for these particulars which are merely given for the purpose of information.

27-11-2023

| Patent document cited in search report | Publication date | Patent family member(s) | Publication date |
|---|---|---|---|
| | | JP 2016185955 A | 27-10-2016 |
| | | JP 2018039788 A | 15-03-2018 |
| | | KR 20090082507 A | 30-07-2009 |
| | | KR 20140097446 A | 06-08-2014 |
| | | US 2010099772 A1 | 22-04-2010 |
| | | US 2015087714 A1 | 26-03-2015 |
| | | US 2017319517 A1 | 09-11-2017 |
| | | US 2021186906 A1 | 24-06-2021 |
| | | WO 2008063603 A2 | 29-05-2008 |
| WO 2010081036 A2 | 15-07-2010 | AU 2010203461 A1 | 28-07-2011 |
| | | CA 2749317 A1 | 15-07-2010 |
| | | EP 2385944 A2 | 16-11-2011 |
| | | JP 2012514655 A | 28-06-2012 |
| | | KR 20110110297 A | 06-10-2011 |
| | | US 2012095217 A1 | 19-04-2012 |
| | | WO 2010081036 A2 | 15-07-2010 |
| DE 1802728 A1 | 26-06-1969 | NONE | |
| CH 422793 A | 31-10-1966 | NONE | |
| US 3660406 A | 02-05-1972 | AR 192587 A1 | 28-02-1973 |
| | | AR 192798 A1 | 14-03-1973 |
| | | AT 311979 B | 10-12-1973 |
| | | AT 311983 B | 10-12-1973 |
| | | AU 3400171 A | 05-04-1973 |
| | | BE 774398 A | 25-04-1972 |
| | | CA 971169 A | 15-07-1975 |
| | | CH 576470 A5 | 15-06-1976 |
| | | CS 172359 B2 | 29-12-1976 |
| | | DD 97210 A5 | 20-04-1973 |
| | | DE 2153349 A1 | 27-04-1972 |
| | | DK 134066 B | 06-09-1976 |
| | | ES 396390 A1 | 01-01-1975 |
| | | ES 399188 A1 | 01-06-1975 |
| | | FR 2111840 A1 | 09-06-1972 |
| | | GB 1340898 A | 19-12-1973 |
| | | HK 63276 A | 15-10-1976 |
| | | IL 37953 A | 28-07-1975 |
| | | NL 7114713 A | 28-04-1972 |
| | | PL 81422 B1 | 30-08-1975 |
| | | SE 380528 B | 10-11-1975 |
| | | SU 437301 A3 | 25-07-1974 |
| | | US 3660406 A | 02-05-1972 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO.

EP 23 16 0797

This annex lists the patent family members relating to the patent documents cited in the above-mentioned European search report. The members are as contained in the European Patent Office EDP file on
The European Patent Office is in no way liable for these particulars which are merely given for the purpose of information.

27-11-2023

| Patent document cited in search report | Publication date | Patent family member(s) | Publication date |
|---|---|---|---|
| CH 436297 A | 31-05-1967 | AT 250975 B | 12-12-1966 |
| | | AT 252925 B | 10-03-1967 |
| | | AT 258912 B | 27-12-1967 |
| | | CH 436297 A | 31-05-1967 |
| WO 2009154563 A1 | 23-12-2009 | AR 072200 A1 | 11-08-2010 |
| | | AU 2009260905 A1 | 23-12-2009 |
| | | BR PI0914231 A2 | 03-11-2015 |
| | | CA 2728675 A1 | 23-12-2009 |
| | | CL 2010001471 A1 | 06-05-2011 |
| | | CN 102131791 A | 20-07-2011 |
| | | CR 11860 A | 25-02-2011 |
| | | CU 20100252 A7 | 15-11-2011 |
| | | CY 1113845 T1 | 27-07-2016 |
| | | DK 2307389 T3 | 02-04-2013 |
| | | DO P2010000393 A | 31-01-2011 |
| | | EA 201001889 A1 | 30-08-2011 |
| | | EC SP10010698 A | 31-01-2011 |
| | | EP 2307389 A1 | 13-04-2011 |
| | | ES 2402084 T3 | 26-04-2013 |
| | | HK 1156308 A1 | 08-06-2012 |
| | | HN 2010002683 A | 15-01-2013 |
| | | HR P20130234 T1 | 30-04-2013 |
| | | IL 210081 A | 31-12-2013 |
| | | IL 229560 A | 30-07-2015 |
| | | JP 5468068 B2 | 09-04-2014 |
| | | JP 2011524898 A | 08-09-2011 |
| | | JP 2014098006 A | 29-05-2014 |
| | | KR 20110019781 A | 28-02-2011 |
| | | ME 01516 B | 20-04-2014 |
| | | MY 157518 A | 15-06-2016 |
| | | NI 201000224 A | 19-03-2012 |
| | | NZ 590616 A | 28-09-2012 |
| | | PE 20110029 A1 | 11-02-2011 |
| | | PL 2307389 T3 | 31-05-2013 |
| | | PT 2307389 E | 28-03-2013 |
| | | RS 52703 B | 30-08-2013 |
| | | SI 2307389 T1 | 30-04-2013 |
| | | SV 2010003769 A | 20-05-2011 |
| | | TW 201002702 A | 16-01-2010 |
| | | UA 105903 C2 | 10-07-2014 |
| | | US 2009318415 A1 | 24-12-2009 |
| | | US 2011160184 A1 | 30-06-2011 |
| | | US 2012202991 A1 | 09-08-2012 |
| | | UY 31919 A | 29-01-2010 |
| | | WO 2009154563 A1 | 23-12-2009 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO.

EP 23 16 0797

This annex lists the patent family members relating to the patent documents cited in the above-mentioned European search report.
The members are as contained in the European Patent Office EDP file on
The European Patent Office is in no way liable for these particulars which are merely given for the purpose of information.

27-11-2023

| Patent document cited in search report | Publication date | Patent family member(s) | Publication date |
|---|---|---|---|
| | | ZA 201100488 B | 26-10-2011 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **CARSTENS, E. ; FOLLANSBEE, T. ; LODI CARSTENS, M.** The Challenge of Basic Itch Research. *Acta Derm. Venereol.,* 2020, vol. 100, adv00023
- **YOSIPOVITCH, G. ; ROSEN, J.D ; HASHIMOTO, T.** Itch: From mechanism to (novel) therapeutic approaches. *J. Allergy Clin. Immunol.,* 2018, vol. 142, 1375-1390
- **CHEN, X.J. ; SUN, Y.G.** Central circuit mechanisms of itch. *Nat. Commun.,* 2020, vol. 11, 3052
- **USOSKIN, D. et al.** Unbiased classification of sensory neuron types by large-scale single-cell RNA sequencing. *Nat. Neurosci.,* 2015, vol. 18, 145-153
- **MEIXIONG, J. ; DONG, X.** Mas-Related G Protein-Coupled Receptors and the Biology of Itch Sensation. *Annu. Rev. Genet.,* 2017, vol. 51, 103-121
- **TSANTOULAS, C. ; MCMAHON, S.B.** Opening paths to novel analgesics: the role of potassium channels in chronic pain. *Trends Neurosci.,* 2014, vol. 37, 146-158
- **YUAN, A. et al.** The sodium-activated potassium channel is encoded by a member of the Slo gene family. *Neuron,* 2003, vol. 37, 765-773
- **BUDELLI, G. et al.** SLO2 Channels Are Inhibited by All Divalent Cations That Activate SLO1 K+ Channels. *J. Biol. Chem.,* 2016, vol. 291, 7347-7356
- **SHARMA, N. et al.** The emergence of transcriptional identity in somatosensory neurons. *Nature,* 2020, vol. 577, 392-398
- **MARTINEZ-ESPINOSA, P.L. et al.** Knockout of Slo2.2 enhances itch, abolishes KNa current, and increases action potential firing frequency in DRG neurons. *Elife,* 2015, vol. 4
- **KUPARI, J. et al.** Single cell transcriptomics of primate sensory neurons identifies cell types associated with chronic pain. *Nat. Commun.,* 2021, vol. 12, 1510
- **TAVARES-FERREIRA, D. et al.** Spatial transcriptomics of dorsal root ganglia identifies molecular signatures of human nociceptors. *Sci. Transl. Med,* 2022, vol. 14, eabj8186
- **BITON, B. et al.** The antipsychotic drug Loxapine is an opener of the Na+-activated potassium channel Slack (slo2.2). *J. Pharmacol. Exp. Ther.,* 2011, vol. 340, 706-715
- **CHAKRABARTI, A. et al.** Loxapine for schizophrenia. *Cochrane Database Syst. Rev.,* 2007, CD001943
- **SCHMIEDL, S. et al.** Loxapine for Treatment of Patients With Refractory, Chemotherapy-Induced Neuropathic Pain: A Prematurely Terminated Pilot Study Showing Efficacy But Limited Tolerability. *Front Pharmacol,* 2019, vol. 10, 838
- **TAVARES-FERREIRA, D. et al.** Spatial transcriptomics of dorsal root ganglia identifies molecular signatures of human nociceptors. *Sci. Transl. Med.,* 2022, vol. 14, eabj8186
- **GRIFFIN, A.M. et al.** Discovery of the First Orally Available, Selective KNa1.1 Inhibitor: In Vitro and In Vivo Activity of an Oxadiazole Series. *ACS Med Chem Lett,* 2021, vol. 12, 593-602
- **LU, R. et al.** Slack channels expressed in sensory neurons control neuropathic pain in mice. *J. Neurosci.,* 2015, vol. 35, 1125-1135
- **AVDEEF, A.** The rise of PAMPA. *Expert Opin. Drug Metab. Toxicol.,* 2005, vol. 1, 325-342
- **MULLER, J. ; ESSO, K. ; DARGO, G. ; KONCZOL, A. ; BALOGH, G.T.** Tuning the predictive capacity of the PAMPA-BBB model. *Eur. J. Pharm. Sci.,* 2015, vol. 79, 53-60
- Permeability - PAMPA. **AVDEEF, A.** Absorption and Drug Development: Solubility, Permeability, and Charge State. Wiley, 2012, 319-498
- **PAGLIARA, A. et al.** Molecular properties and pharmacokinetic behavior of cetirizine, a zwitterionic H1-receptor antagonist. *J. Med. Chem.,* 1998, vol. 41, 853-863
- **POPOVIC, D. ; NUSS, P. ; VIETA, E.** Revisiting Loxapine: a systematic review. *Ann Gen Psychiatry,* 2015, vol. 14, 15
- **SJOSTEDT, E. et al.** An atlas of the protein-coding genes in the human, pig, and mouse brain. *Science,* 2020, vol. 367
- **KARLSSON, M. et al.** A single-cell type transcriptomics map of human tissues. *Sci Adv,* 2021, vol. 7
- **LIU, Q. et al.** Sensory neuron-specific GPCR Mrgprs are itch receptors mediating chloroquine-induced pruritus. *Cell,* 2009, vol. 139, 1353-1365
- **LIU, Q. et al.** The distinct roles of two GPCRs, MrgprC11 and PAR2, in itch and hyperalgesia. *Sci. Signal.,* 2011, vol. 4, 45

- **KITAMURA, A. ; TAKATA, R. ; AIZAWA, S. ; WATANABE, H. ; WADA, T.** A murine model of atopic dermatitis can be generated by painting the dorsal skin with hapten twice 14 days apart. *Sci Rep,* 2018, vol. 8, 5988
- **LI, M. et al.** Topical vitamin D3 and low-calcemic analogs induce thymic stromal lymphopoietin in mouse keratinocytes and trigger an atopic dermatitis. *Proc. Natl. Acad. Sci. U. S. A.,* 2006, vol. 103, 11736-11741
- **GRUNDY, L. et al.** Chronic linaclotide treatment reduces colitis-induced neuroplasticity and reverses persistent bladder dysfunction. *JCI Insight,* 2018, vol. 3, e121841